# EUROPEAN PATENT APPLICATION

(11) **EP 4 574 167 A1**
(43) Date of publication of application: **25.06.2025**
(21) Application number: 23383323.5
(22) Date of filing: 19.12.2023
(51) Int. Cl.: A61K 39/012, A61P 33/02

(54) **TOXOPLASMA GONDII TACHYZOITE AND BRADYZOITE NATIVE PROTEINS INACTIVATED VACCINE**

(71) Applicant: Saluvet-Innova S.L., 28040 Madrid (ES)
(72) Inventor: REGIDOR CERRILLO, Javier, 28040 Madrid (ES); FERRE PÉREZ, Ignacio, 28040 Madrid (ES); ORTEGA MORA, Luis Miguel, 28040 Madrid (ES)
(74) Representative: Hoffmann Eitle

(57) **Abstract**

The present invention relates to new protein compositions, methods for producing said protein compositions, pharmaceutical compositions comprising said protein compositions and methods for treating infections caused by *Toxoplasma gondii.* In particular, the present invention provides a protein composition comprising native *T. gondii* tachyzoite-specific proteins and native *T. gondii* bradyzoite and/or cyst specific proteins.

## Description

### FIELD OF THE TECHNOLOGY

The present invention relates to the field of protein compositions, in particular to the field of vaccines. The present invention relates to new protein compositions, methods for producing said protein compositions, pharmaceutical compositions comprising said protein compositions and methods for preventing, controlling, and treating infections caused by *Toxoplasma gondii.*

### BACKGROUND OF THE INVENTION

*Toxoplasma gondii* (*T. gondii*) is a parasite belonging to the *Subphylum* Apicomplexa (*Phylum* Alveolata) in which the genera *Plasmodium, Babesia, Cryptosporidium, Eimeria* and *Toxoplasma* are grouped, which include some of the most important disease-causing organisms known to man and animals. *T. gondii* was discovered in 1908 by scientists working in Tunisia and Brazil (Nicolle and Manceaux, 1908, Comtes Rendues. Academy of Sciences 147, 736; Splendore, 1908, Rev Soc of Scient Sao Paulo 3, 109-112). It can chronically infect all warm-blooded animals, including human. More than 33% of the worldwide population is at risk of infection.

It is a facultative heteroxenous parasite, having many potential routes of transmission within and between different host species. It has a complex life cycle, in which three infective stages have been identified: tachyzoites, which is the fast-replicating stage involved in the acute phase of infection, bradyzoites, a slow-replication or a quiescent stage contained within tissue cysts that is involved in the chronic phase of infection and sporulated oocysts that bear sporozoites, the environmental resistant stage (Tenter et al., 2000, Int J Parasitol 30(12-13): 1217-58; Dubey, 2022, Toxoplasmosis of animals and humans, 3rd ed. Boca Raton: CRC Press; p. 1-542).

The transmission of this parasite can be horizontal by ingestion of food or drink contaminated with infectious oocysts shed in the feces of feline definitive hosts such as domestic cat or tissue cysts present in raw or undercooked meat from intermediate hosts, or vertical, consisting of the transmission through the animal placenta of tachyzoites from mother to the foetus (Tenter et al., 2000, Int J Parasitol 30(12-13): 1217-58). After being consumed tissue cysts (carnivores and omnivores) or oocysts (all warm-blooded animals), bradyzoites and sporozoites, respectively, are released and infect intestinal epithelium, that after several rounds of intestinal replication converts to tachyzoites that are disseminated to other tissues throughout the body for acute phase of infection and replicate intracellularly until the cell lysis, causing tissue necrosis. During tachyzoite dissemination immunocompetent individuals mount a powerful, cell-mediated immune response to the tachyzoites that can control infection and tachyzoites driving into bradyzoites inside tissue cysts in brain and other organs such as heart, tongue, diaphragm, and skeletal muscle. Immune responses do not clear chronic infection and *T. gondii* can remain viable as bradyzoite inside cysts in the tissues of infection, for many years, possibly for the life of the host (Dubey, 2022, Toxoplasmosis of animals and humans, 3rd ed. Boca Raton: CRC Press; p. 1-542). Spontaneous tachyzoite - bradyzoite conversion and formation of cysts can occur *in vitro* in recently obtained *T. gondii* isolates or under stress conditions for the growth (Colos-Arango et al., 2023, Int J Parasitol. 53(9): 491-504; Ferreira da Silva et al., 2008, Mol. BioSyst. 4: 824-834). There are many components at play when the parasite converts from tachyzoites to bradyzoites and *vice versa.* Biology of tachyzoite to bradyzoite conversion involves transcriptome and translational gene regulation, parasite metabolism changes and cell cycle regulation, change of protein markers and generation of tissue cyst wall (Tu et al., 2018, Microbes Infect. 20(9-10):466-476; Tu et al., 2019, mBio. 10(2): e00469-19). *T. gondii* expresses different stage specific isoforms of metabolic enzymes, TgLHD1 and TgENO2 in bradyzoite stage and TgLHD2 and TgENO1 in tachyzoite stage, according to energy requirements for each stage. Bradyzoite antigen 1/Heat shock protein 30 (TgBAG1) was one of the first and it is one of the most abundant proteins within the bradyzoite. Tachyzoites have specific plasma membrane proteins with antigenic properties such as surface antigen 1 (TgSAG1/p30), 2A (TgSAG2A/p22), and TgSAG1-related sequence proteins 1 to 3 (TgSRS1-3), which are involved in tachyzoite host cell attachment and invasion and regulation of the host immune response to tachyzoites. Bradyzoites modify surface antigens by expression of specific TgSAG4A, TgSAG2C, TgSAG2D, TgSAG2X, TgSAG2Y, TgSRS9 and TgBSR4. Bradyzoite rhoptry protein 1 (TgBRP1) is also only secreted from the rhoptries after parasite differentiation and it localizes to the matrix of the tissue cyst. In addition to TgBRP1, other specific proteins have been identified as components of cyst wall and cyst matrix, such as TgCST1 to 7, TgMCP4 and TgMAG1, some of them with high relevance in virulence (Tu et al., 2018, Microbes Infect. 20(9-10):466-476; Tu et al., 2019, mBio. 10(2): e00469-19). Bradyzoite markers have been identified as highly expressed during chronic phase of infection, whereas tachyzoite markers have been identified as highly expressed during acute phase of infection (Buchholz et al., 2011, Eukaryot Cell. 10: 1637-47; Garfoot et al., 2019, BMC Genomics 20: 859; Pittman et al., 2014, BMC Genomics 15: 806).

Most infected immunocompetent humans are asymptomatic or have mild clinical signs (mild flu-like, myalgia or other nonspecific symptoms). However, when primary infection occurs during pregnancy, tachyzoites can reach the placenta and foetus, and *T. gondii* can cause abortion or severe congenital defects such as mental retardation, blindness and hydrocephaly. In immunocompromised patients, reactivation of *T. gondii,* when bradyzoites egress from cysts and transition back to rapidly proliferating tachyzoites, or primary infection can occur and can lead to chorioretinitis, encephalitis or pneumonitis (Dubey, 2022, Toxoplasmosis of animals and humans, 3rd ed. Boca Raton: CRC Press; p. 1-542). The treatment of human toxoplasmosis is complex, and the gold standard therapy in humans, for instance, involves the combination of pyrimethamine and sulfadiazine. The efficacy of these drugs varies if the treatment begins in an advanced stage of the disease, the reactivation of parasites as well as drug resistance can occur (Montazeri et al., 2018, Front. Microbiol. 9: 2587; Dunay et al., 2018, Clin. Microbiol. Rev. 31(4): e00057-17). Although pharmacological treatments effectively target tachyzoites, they were inefficient against bradyzoites and latent stages of *T. gondii* (Dunay et al., 2018, Clin. Microbiol. Rev. 31(4): e00057-17). In that sense, vaccines are one of the most interesting therapeutic options for controlling the long-term disease and decrease the risk of infection by this parasite.

Pigs are intermediate hosts of *T. gondii* and frequently bear tissue cysts in their bodies. They are one of the primary sources of parasite transmission to humans. Studies show that 30% of domestic pigs have been exposed to this parasite globally, having a seroprevalence of 19% (Dubey et al., 2020, Vet. Parasitol. 288: 109185). Like the infection in humans, pigs infected with *T. gondii* do not normally display clinical signs. Some infected animals can develop mild signs, like fever or apathy, but they usually recover. Outbreaks of toxoplasmosis in pigs have been recorded in Brazil and China, although clinical signs are rare. Toxoplasmosis in pigs can cause abortions, although the losses are rather modest in comparison with small ruminants (Dubey et al., 2020, Vet. Parasitol. 288: 109185).

Regarding small ruminants, goats and sheep, *T. gondii* is transmitted vertically from mother to foetus (Dubey et al., 2020, Vet Parasitol. 286: 109195; Dubey et al., 2020, Res. Vet. Sci. 132: 292-307). *T. gondii* is one of the main parasites responsible for abortions, which can add up to 1.5 million lambs lost in Europe every year being a significant loss to producers and national economies (Innes et al., 2009, Parasitology. 136(14): 1887-94). *T. gondii* can remain also in sheep and congenitally infected lambs in the brain and muscle tissues as a source of infection for humans.

*T. gondii* can also cause acute and fatal toxoplasmosis in wild animals as marsupials, monkeys from new-world and meerkats, species highly susceptible to infection (de Barros et al., 2022, Acta Tropica 231: 106432).

There is only one commercially available vaccine for the treatment of toxoplasmosis in sheep (Toxovax^{®}, MSD, New Zealand; NZ220023 (A)). Toxovax^{®} is a live attenuated vaccine of tachyzoites of the modified *T. gondii* strain S48 approved in some European countries and New Zealand and used to reduce abortions in sheep. The "incomplete" S48 strain is not able to form tissue cysts or oocysts, the main infective parasite stages (Buxton et al., 1991, Vet. Rec. 129: 89-93). Nevertheless, Toxovax^{®} fails to fully eliminate this parasite, its shelf life is relatively short, and it is expensive. Toxovax^{®}, similarly as other live attenuated vaccines cannot be used on pregnant sheep due to risk of transmission to the foetus and foetal lost. Toxovax^{®} cannot be used for at least three months before sheep pregnancy. In addition, there is the concern of the possibility of reversion to a pathogenic form, since the molecular bases of attenuation of the S48 strain are yet to be completely studied and understood (Chu & Quan, 2021, Vaccines 9: 413). Apart from Toxovax^{®}, other live vaccines have been generated through chemical-induced mutation of *T. gondii* or genetically modified mutants, deficient for producing oocysts, bradyzoites or attenuated in virulence ((EP0687471 (A1); Ramakrishnan et al., 2019, Sci Rep. 9(1): 1474; Freyre et al., 1993, J Parasitol. 79(5): 716-9; Ismael et al., 2006. J Infect Dis. 194(8): 1176-83; US2022235101 (A1)). Immunization with the attenuated mutant Mic1-3KO previously to pregnancy demonstrated 62-91% of protection against abortion in sheep after oral infection with 400 sporulated oocysts of PRU strain at mid-gestation (Mévélec et al., 2010. Vet. Res. 41: 49). Nevertheless, live vaccines preserve some safety and production limitations of Toxovax^{®}.

In the last years, the most investigated strategy has been the development of subunit or DNA vaccines against *T. gondii* infection and vertical transmission in pregnant animals. A wide array of recombinant antigens has been explored such as single- or few potential vaccine candidates against *T. gondii* infection in mice usually with partial results (for review see Chu & Quan, 2021, Vaccines 2021, 9: 413; Barros et al., 2021, Front Immunol. 11: 621997). DNA and nano-particle platforms as alternative to recombinant protein vaccine also has been evaluated to improve immune protection with selected vaccine candidates. Evaluation of specific bradyzoite antigens has been limited to studies from a DNA platform in mice with reduction by 62% in vaccinated with the mixture of pcDNA-BAG1 and pcDNA-MAG1 (Nielsen et al., 2006, Exp. Parasitol. 112: 274-279). SAG2C, -2D, and -2X are specific surface antigens expressed on the bradyzoites of *T. gondii* are also proposed as vaccine candidates against chronic infection with significant reduction of tissue cysts in infected and challenged mice (CN103251960A).

Inactivated or killed vaccines and subunit vaccines involving native antigens have been also explored as vaccine candidates.

Killed whole tachyzoite preparations as vaccines were evaluated against foetal lost in sheep, but with demonstrated absence of protection against subsequent challenge with *T. gondii* (Beverley et al., 1971. Br. Vet. J. I27: 529). A killed vaccine of 3×10⁷ disintegrated *Toxoplasma* tachyzoites of the S48 strain adjuvanted with Freund's incomplete adjuvant was also evaluated in sheep. After two immunizations previous to mating, the ewes were challenged intravenously with 1×10⁴ live *T. gondii* tachyzoites of S48 at mid-pregnancy, but vaccination significantly reduced fertility and fecundity with no observation of protection against foetal lost in the vaccinated ewes (Wilkins et al., 1987. New Zealand Veterinary Journal, 35:3, 31-34). Killed or inactivated vaccines based on bradyzoites have not been previously evaluated probably due to difficulties to produce *in vitro* under stress conditions (EP0687471 (A1)).

Vaccines based on complex tachyzoite protein extracts or protein fractions derived from *T*. *gondii* tachyzoites have been also assessed against toxoplasmosis.

Partial protection against tissue cyst formation was obtained with a vaccine containing crude *T. gondii* rhoptry proteins -200 µg per dose- incorporated in the immunostimulant complexes (ISCOM) adjuvant when it was tested in pigs after immunization by nasal route three times at 21 day-interval and challenged with 1000 oocysts of *T. gondii* VEG strain. Parasite was detected in the 75% of brains from pigs of the vaccinated group *versus* 100% of brains from pigs of the control group (Cunha et al., 2012. Vet. Parasitol. 186: 216- 221).

In addition, pigs immunized subcutaneously with 2000 µg of excretory/secretory antigens (ESA) emulsified with Freund's adjuvant and homologous challenge with 10⁷ tachyzoites of GJS isolate, showed reduction on parasite infection determined by mice bioassay and microscopically examination of the mouse brain for *T. gondii* tissue cysts. *T. gondii* infection was detected in 1 out of 5 of immunized pigs in comparison to 5 out of 5 of non-immunized pigs with a reduction on frequency of parasite detection of the 89% (Wang et al., 2013. Parasitol. Res. 112:3835-3842).

Total lysate antigens (TLA) obtained by sonication of RH tachyzoites - 500 µg- combined with Quil-A^{®} adjuvant were also evaluated in terms of parasite DNA load reduction in pigs after oral challenge of with 3500 *T. gondii* tissue cysts of the IPB-LR isolate and boost 7 days after challenge. Protection against *T. gondii* infection in pig tissues was partial with only a 40% of reduction in frequency of parasite detection in TLA vaccinated group (Rahman et al., 2019. Front. Immunol. 10: 2223).

*T. gondii* membrane antigens obtained from RH tachyzoites with MEGA-10 detergent were assessed formulated into immune stimulating complexes (ISCOMS) for their ability to induce protective immune responses against foetal lost during pregnancy in sheep. Ewes were immunized subcutaneously with 40-50 µg of protein three times, 28 days before mating, at 26 days of pregnancy and at 70 days of pregnancy and challenged orally at day 91 of pregnancy with 2000 *T. gondii* sporulated oocysts of the M1 isolate. Protection against foetal lost was partial. Lamb mortality in the vaccinated group was 36.4% after a mean gestation of 141 days while in the unvaccinated control group it was 64.7% after a mean gestation of 131.5 days, but these results were not found to be significant (Buxton et al., 1989. Br. Vet. J. 145, 451).

Hence, there is still an urgent need for a *T. gondii* vaccine capable of inducing a strong immune response in the host as well as of rendering an efficient protection against *T. gondii* infection.

### SUMMARY OF THE INVENTION

The inventors have addressed the above need and have unexpectedly found that a protein composition comprising native *T. gondii* tachyzoite-specific and native *T. gondii* bradyzoite and/or cyst specific proteins can confer heterologous protection against *T. gondii* infection, particularly in mice, pigs and sheep. The new antigenic extract of *T. gondii* constitutes an efficient vaccine against *T. gondii,* as shown in the examples.

Hence, in one aspect, the present invention relates to a protein composition comprising native *T. gondii* tachyzoite-specific proteins and native *T. gondii* bradyzoite and/or cyst specific proteins, preferably native *T. gondii* tachyzoite-specific proteins and native *T. gondii* bradyzoite and/or cyst specific proteins selected from Tables 1 and 2, respectively. The present invention also relates to protein compositions comprising, in addition to tachyzoite and bradyzoite and/or cyst specific proteins, at least one native *T. gondii* acute phase infection-specific protein and at least one native *T. gondii* chronic phase infection-specific protein, as described herein.

The invention further provides a method for producing a protein composition, preferably the protein composition of the present invention, comprising the following steps:
a. Providing *Toxoplasma gondii* cells in a hypertonic solution;
b. Centrifuging said solution obtained in step (a) under conditions suitable for separating the soluble fraction (supernatant) and insoluble fraction (precipitate);
c. Recovering the precipitate from step (b); and
d. Mixing said precipitate with a non-ionic surfactant.

Further, the invention provides a pharmaceutical composition comprising the protein composition of the invention, and/or the protein composition produced by the method of the present invention.

In a further embodiment, the invention provides a vaccine comprising the protein composition of the invention, and/or the protein composition produced by the method of the present invention.

The invention also provides the use of the protein composition, pharmaceutical composition and/or vaccine of the present invention in medicine, preferably for use in a method of therapeutic treatment (after infection or after the clinical manifestation of the disease caused by the infection) and/or prophylactic treatment (before infection or before the clinical manifestation of the disease caused by the infection) of infections caused by

*Toxoplasma gondii.*

### BRIEF DESCRIPTION OF THE DRAWINGS

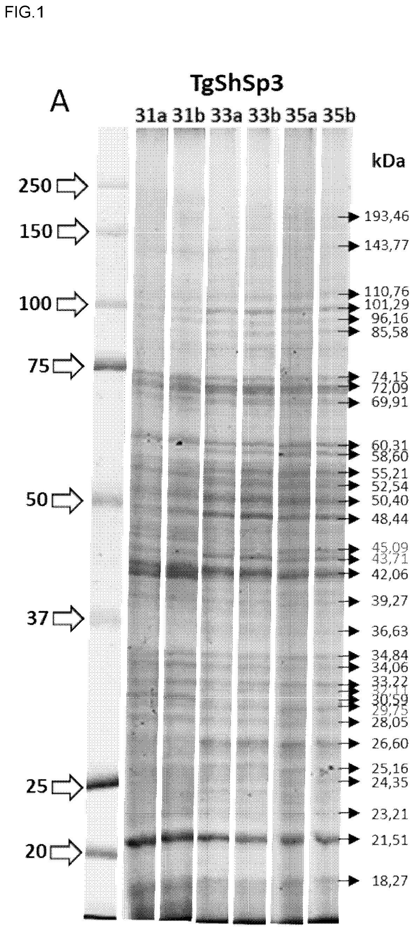

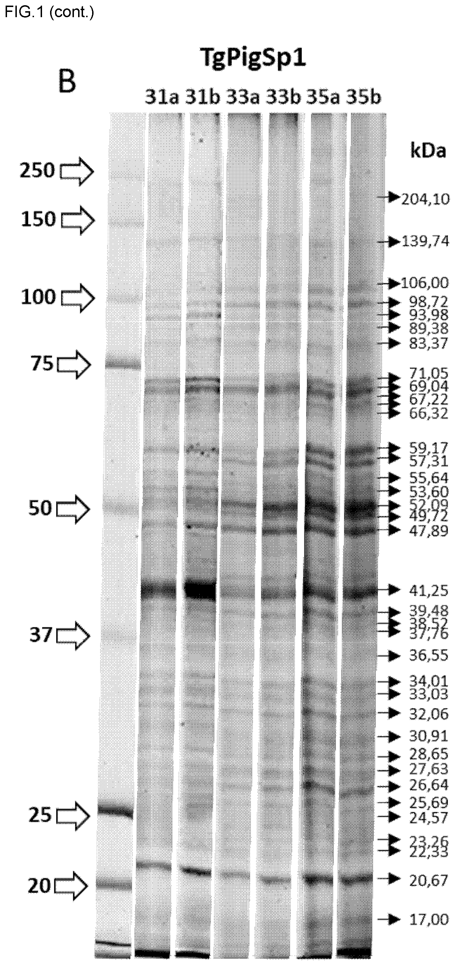

**Figure 1****:** Protein pattern in Coomassie stained SDS-PAGE gels of 50 µg of the *Toxoplasma gondii* antigen extract (TgAE) obtained from the TgShSp3 isolate (A) and the TgPigSp1 isolate (B). Each gel includes two replicates -a and b- of passages p31, p33 and p35 as technical and biological replicates, respectively. White arrows on the left highlight bands and relative molecular weight in kDa of Kaleidoscope^{™} marker. Black arrows on the right highlight protein bands and the relative molecular weight in kDa recognized on TgAE of the TgShSp3 isolate (A) and the TgPigSp1 isolate (B). Relative molecular weight in red indicates specific protein bands found in the TgShSp3 TgAE (A) and TgPigSp1 TgAE (B).

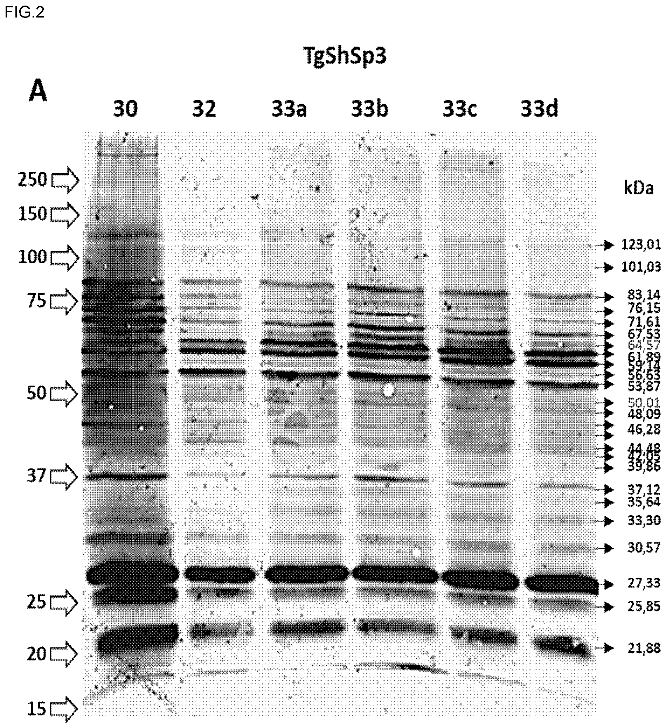

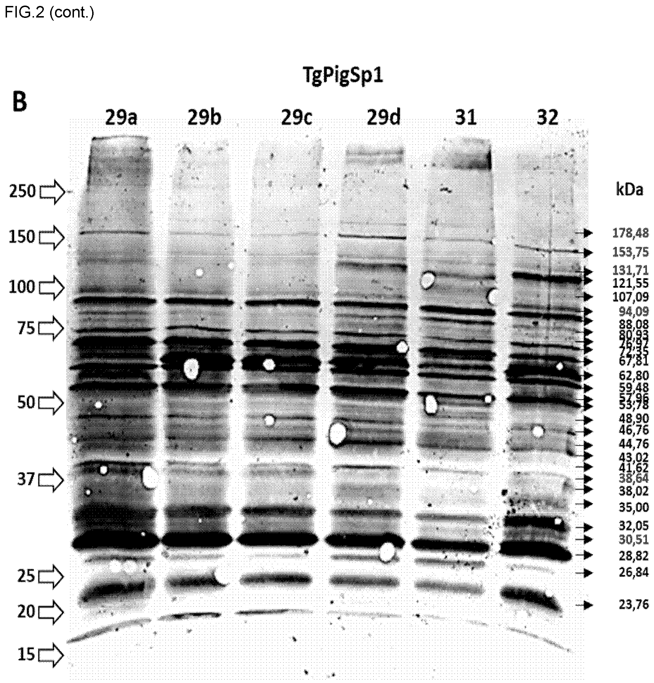

**Figure 2****:** Pattern of protein antigens by immunoblotting of the *Toxoplasma gondii* antigen extract (TgAE) of the TgShSp3 isolate (A) and TgAE of the TgPigSp1 isolate (B) with sera from orally infected mice with 100 oocysts of the *T. gondii* TgShSp1 isolate at 42 days post-infection. Each immunoblotting includes different biological replicates (TgSpSh3- passages p30, p32, and four technical replicates of p33 a-d; TgPigSp1 -passages p29 with four technical replicates a-d, p31 and p32). White arrows on the left represent bands and relative molecular weight in kDa of Kaleidoscope^{™} marker. Black arrows on the right highlight immunoreactive bands and their relative molecular weight in kDa recognized on each TgAE. Relative molecular weight in red indicates specific proteins found in the TgShSp3 TgAE (A) and TgPigSp1 TgAE (B).

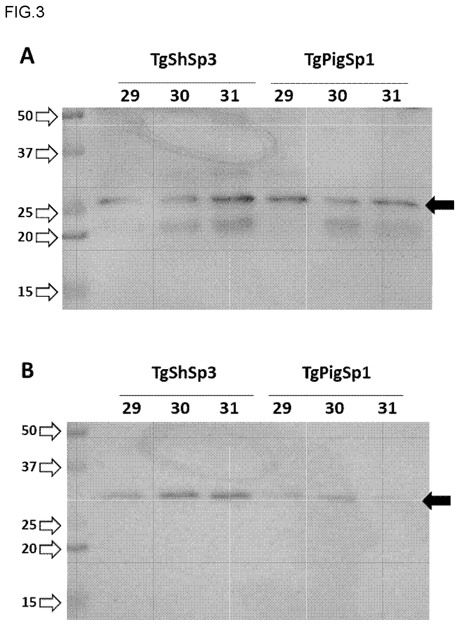

**Figure 3****:** Immunoblotting of the *Toxoplasma gondii* antigen extracts (TgAEs) of the TgShSp3 and TgPigSp1 isolates for identifying specific bradyzoite protein TgBAG1 of 25 kDa (A) and specific tachyzoite protein TgSAG1 of 30 kDa (B). Each immunoblotting includes different biological replicates of passages p29, p31 and p32 of the TgSpSh3 and TgPigSp1 isolates. White arrows on the left represent bands and relative molecular weight in kDa of Kaleidoscope^{™} marker. Black arrows on the right highlight TgBAG1 in (A) and TgSAG1 in (B).

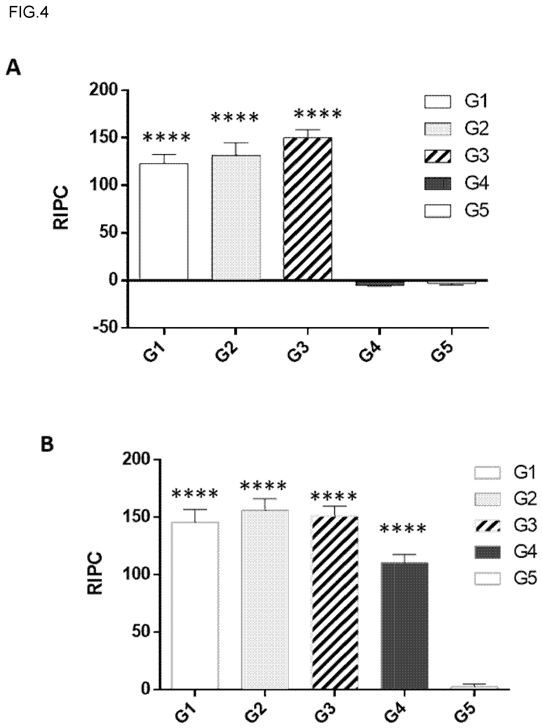

**Figure 4****:** IgG levels against *T. gondii* measured by ELISA in sera of mice groups at day 16 after booster - at five days prior challenge- of the groups immunized with the *Toxoplasma gondii* antigen extract (TgAE) vaccine of the TgShSp3 isolate (G1), TgAE vaccine of the TgShSp1 isolate (G2), a mix of TgAEs of the TgShSp3 and TgPigSp1 (G3), saponin (G4) and PBS (G5) (A) and at day 42 after challenge of groups G1, G2, G3 and G4 (B). The column represents the mean relative index per cent (RIPC), and the error bars, the standard error of the mean. **** indicates significant differences p<0.0001 with the sentinel group G4.

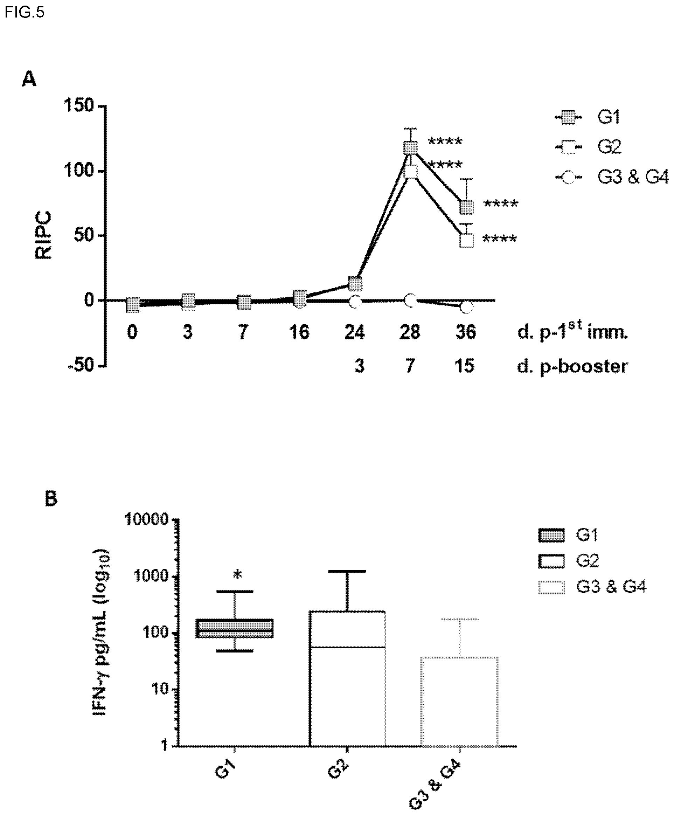

**Figure 5****:** Immunogenicity of *Toxoplasma gondii* antigen extract (TgAE) vaccine in piglets. (A) Kinetics of IgG levels against *T. gondii* measured by ELISA in sera from piglets of the groups immunized with 40 µg of the TgAE (G1), 20 µg of the TgAE (G2) or PBS (G3 & G4). Each point represents the mean IgG levels measured throughout the experiment (X-axis; days after first immunization: -d.p-1^{st} imm.; days after booster- d. p-booster) as the relative index per cent (RIPC) value (Y-axis). The error bars represent the standard error of the mean. **** indicates significant differences p<0.0001 with the control groups G4 and G5. (B) Interferon-gamma (IFN-y) levels measured in supernatants of *T*. *gondii*-stimulated blood cell cultures from piglets of the groups G1, G2, G3 and G4 at day 28 after first immunization prior to challenge. * indicates significant differences p<0.05 with the control groups G4 and G5 inoculated with PBS.

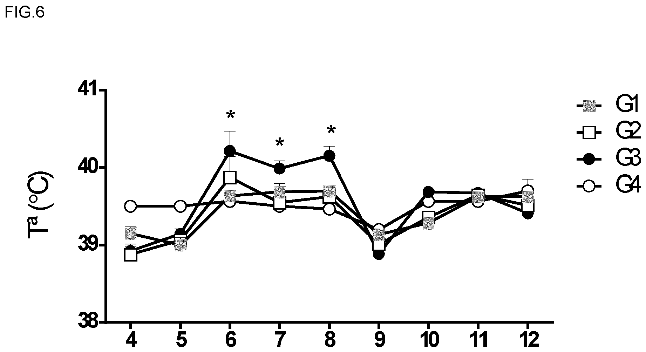

**Figure 6****:** Rectal temperatures of piglets from day 4 to 12 after challenge with 1000 TgShSp1 oocysts in the groups immunized with 40 µg of the *Toxoplasma gondii* antigen extract (TgAE) (G1), 20 µg of the TgAE (G2) or PBS (G3) and (G4). Each point represents the mean rectal temperature in °C (Y-axis) recorded at day of challenge -day 0- until the end of the experiment (X-axis; days after challenge) and error bars the standard statistical error of the mean. * indicates significant differences p<0.05 with the sentinel group G4.

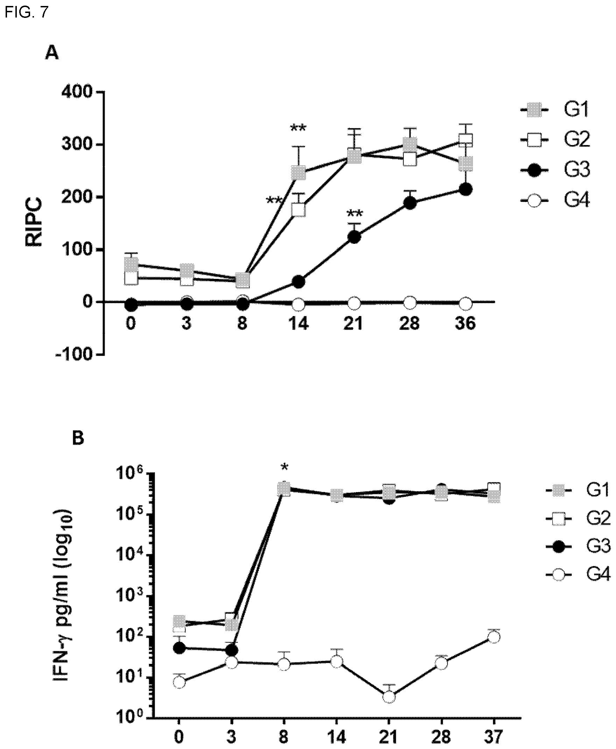

**Figure 7****:** Kinetics of IgG levels against *T. gondii* measured by ELISA (A) and kinetics of production of IFN-y levels determined in blood stimulation assays (B) after challenge in the groups immunized with 40 µg of the *Toxoplasma gondii* antigen extract (TgAE) (G1), 20 µg of the TgAE (G2) and PBS (G3) and sentinel (G4). Each point represents the mean IgG levels as the relative index per cent (RIPC) value in (A) and the mean IFN-y levels assessed in picograms/mL in supernatants of *T*. *gondii*-stimulated blood cell cultures as log₁₀ in (B) from the challenge -day 0- to the end of experiment (X-axis- days after challenge). The error bars represent the standard error of the mean. * and ** indicate significant differences p < 0.05 and p< 0.01, respectively, at day 8 after challenge, when humoral and cellular responses were substantially increase in challenged groups (G1, G2 and G3) and maintained until the end of experiment versus the sentinel group G4.

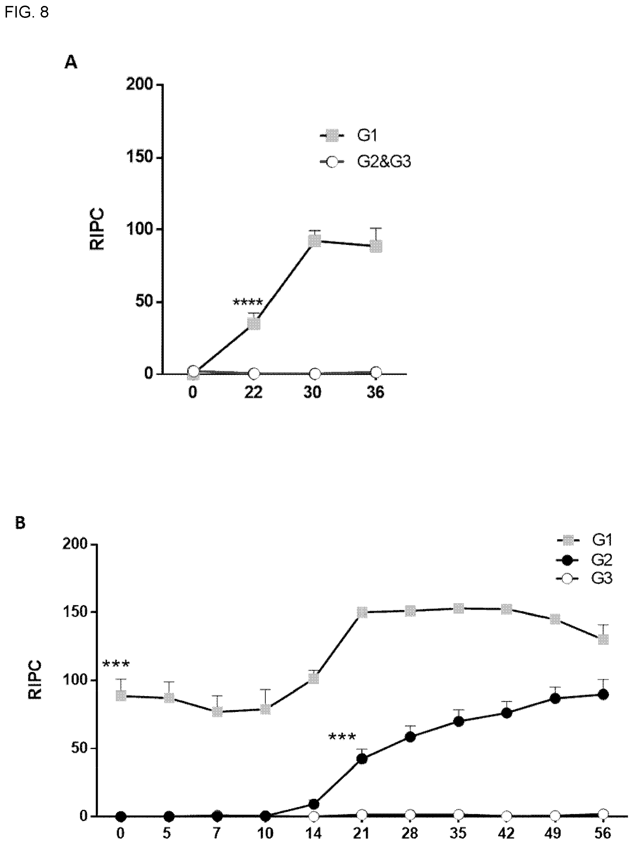

**Figure 8****:** Kinetics of IgG levels against *T. gondii* measured by ELISA in sera of ewes after immunization (A) and challenge (B) of the groups immunized with 40 µg of the *Toxoplasma gondii* antigen extract (TgAE) vaccine (G1), immunized with PBS and challenged (G2) and the sentinel group (G3). Each point in (A) and (B) represents the mean IgG levels as the relative index per cent (RIPC) value (Y axis) measured throughout the experiment (X-axis in A; days after first immunization; X-axis in B; days after challenge). The error bars represent the standard error of the mean. *** and **** indicate significant differences p < 0.001 and p< 0.0001, respectively, at day 22 after first immunization in (A) and at days 0 (group G1) and 21 (group G3) after challenge, when humoral responses were substantially increase in challenge groups and maintained until the end of experiment *versus* the sentinel group G3.

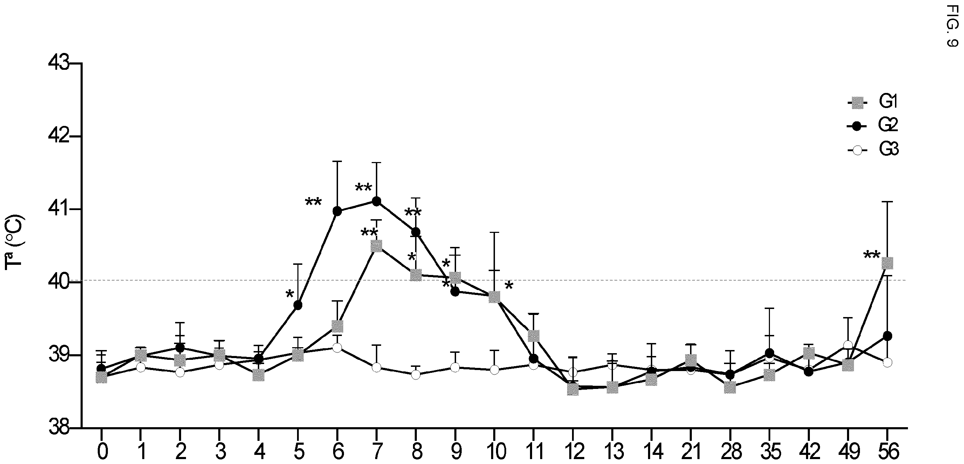

**Figure 9****:** Rectal temperatures of ewes throughout the experiment after challenge with 10 TgShSp1 oocysts in the groups immunized with 40 µg of the *Toxoplasma gondii* antigen extract (TgAE) (G1), PBS (G2) and the sentinel group (G3). Each point represents the mean rectal temperature in °C (Y-axis) recorded throughout of experiment (X-axis, days after challenge). The error bars represent the standard statistical error of the mean. * and ** indicate significant differences p<0.05 and p<0.01, respectively, with the sentinel group G3.

### DETAILED DESCRIPTION OF THE INVENTION

### The protein composition of the present invention

A first aspect of the present invention relates to a protein composition ("**the protein composition of the present invention**") comprising native *T. gondii* tachyzoite-specific proteins and native *T. gondii* bradyzoite and/or cyst specific proteins. Hence, in a first aspect, the present invention provides a protein composition comprising at least one native *T. gondii* tachyzoite-specific protein and at least one native *T. gondii* bradyzoite and/or cyst specific protein. Preferably, the composition of the invention comprises more than one, such as 2, 3, 4, 5, 6 or 7, or more, native *T. gondii* tachyzoite-specific proteins and more than one, such as 2, 3, 4, 5, 6, 7, 8 or 9, or more, native *T. gondii* bradyzoite and/or cyst specific proteins. The native *T. gondii* tachyzoite-specific protein(s) is(are) preferably selected from the proteins depicted in Table 1. The native *T. gondii* bradyzoite and/or cyst specific protein(s) is(are) preferably selected from the proteins depicted in Table 2. In a further embodiment, the protein composition of the present invention comprises two native tachyzoite-specific proteins of Table 1 and two native specific bradyzoite and/or cyst proteins of Table 2. In a further embodiment, the protein composition of the present invention comprises three native tachyzoite-specific proteins of Table 1 and three native specific bradyzoite and/or cyst proteins of Table 2. In a further embodiment, the protein composition of the present invention comprises four native tachyzoite-specific proteins of Table 1 and four native specific bradyzoite and/or cyst proteins of Table 2. In a further embodiment, the protein composition of the present invention comprises five native tachyzoite-specific proteins of Table 1 and five native specific bradyzoite and/or cyst proteins of Table 2. In a further embodiment, the protein composition of the present invention comprises six native tachyzoite-specific proteins of Table 1 and six native specific bradyzoite and/or cyst proteins of Table 2. In a further embodiment, the protein composition of the present invention comprises seven native tachyzoite-specific proteins of Table 1 and seven native specific bradyzoite and/or cyst proteins of Table 2. In a further embodiment, the protein composition of the present invention comprises seven native tachyzoite-specific proteins of Table 1 and eight native specific bradyzoite and/or cyst proteins of Table 2. In a preferred embodiment, the composition comprises all proteins depicted in Tables 1 and 2.

In the context of the present invention, a **"native protein"** refers to a protein which is obtained from a proper organism, i.e., from *T. gondii* (i.e., a protein which is not recombinantly produced by an organism different from *T. gondii*), and which is in a folded and assembled form with operative structure and function, including the original post-translational modifications (PTMs), such as glycosylation, phosphorylation, nitrosylation, disulfide bridge formation, or proteolytic processing. Recombinantly produced proteins are manipulated versions of native proteins produced by genetically modified organisms such as genetically modified bacteria, yeast or other eucaryotic cells. The recombinant DNA is cloned into a vector introduced in a specific expression system (e.g., mammalian, bacteria, yeast, or insect cells) to support the expression of the gene of interest and the production of the recombinant protein. For instance, proteins produced in prokaryotes do not undergo PTMs. Mammalian cells can perform some mammalian-specific post-translational modifications, but the currently available recombinant expression systems do not guarantee producing proteins with epitope conservation; with the same folding, assembly and which comprise the same PTMs as a native protein. Hence, oftentimes, native proteins show higher potential as efficient antigens and vaccine candidates as compared with recombinantly produced proteins.

*T. gondii* has the ability to develop a chronic infection in the brain and other tissues such as muscles of its host by transitioning from the fast-growing tachyzoite stage (**acute** phase of the infection) to a latent bradyzoite stage (**chronic** phase of the invention). A hallmark of the bradyzoite is the development of tissue cysts in the tissues of the host. The bradyzoite parasites within the cyst with a carbohydrate and protein-rich wall have a slow replication cycle, or quiescent, allowing them to remain hidden from the host (chronic phase of the invention). *T. gondii* undergoes a major transformational change during the switch from a tachyzoite to a bradyzoite, and *vice versa.* This transformational change is accompanied by a change in protein repertoire (i.e., by a change in the proteins expressed specifically by each of the parasite stages, tachyzoite, bradyzoite and/or cyst stages). The skilled person is able to identify specific tachyzoite and specific bradyzoite and/or cyst proteins, e.g., as described in previous studies by Tu *et al.* (Tu et al., 2018, Microbes Infect. 20(9-10):466-476; Tu et al., 2019, mBio. 10(2): e00469-19). These studies further provide specific reference to *T. gondii* native bradyzoite and/or cyst specific antigens (proteins) and to *T. gondii* native tachyzoite-specific antigens (proteins). Tu *et al.,* 2019 provide a data set of potential specific *T. gondii* cyst wall proteins, identifying known cyst wall proteins and validating several novel cyst wall proteins, see, e.g., Table 1 on p. 5 of this document. Finally, Tu *et al.,* 2018 highlight research on bradyzoites conducted within the past few years that have provided insights into bradyzoite differentiation. For instance, Figure 1 in this document provides the localization of various tachyzoite and bradyzoite and/or cyst markers or specific proteins.

Tables 1 and 2 below list common identified *T. gondii* specific tachyzoite proteins (Table 1) and specific bradyzoite and/or cyst proteins (Table 2).

The skilled person is also aware of the changes in the repertoire of parasite proteins associated with acute and chronic phases of infection. Hence, there are proteins from *T*. *gondii* highly expressed and more abundant during acute phase of infection (with the predominance of fast-growing tachyzoite stage) that differ from proteins highly expressed and more abundant during chronic phase of infection (with predominance of the latent bradyzoite inside tissue cysts). The skilled person is also able to identify which proteins are highly expressed and more abundant during acute phase of infection ("***T. gondii* acute phase infection-specific proteins**") and which ones are highly expressed and more abundant with chronic phase of infection ("***T. gondii* chronic phase infection-specific proteins**")**.** For instance, Buchholz et al., 2011 (Eukaryot Cell. 10: 1637-47), Garfoot et al., 2019 (BMC Genomics 20: 859), Pittman et al., 2014 (BMC Genomics 15: 806), together with Tu et al., 2019 (mBio 10: e00469-19) and Tu et al., 2018 (Microbes Infect. 20: 466-476) describe the differences between acute phase infection- and chronic phase infection-specific proteins and provide specific reference to *T. gondii* acute phase infection-specific proteins and *T. gondii* chronic phase infection-specific proteins. For example, Buchholz *et al.* report an analysis of the transcriptomes of *in vitro* and *in vivo* bradyzoites and analyse the changes in transcript profiles between tachyzoite-to-bradyzoite developmental forms and between in *vitro* and *in vivo* bradyzoites, see, e.g., Buchholz et al., 2011 (Eukaryot Cell. 10: 1637-47) starting on p. 1640. Garfoot et al., 2019 (BMC Genomics 20: 859) provide an *in vivo* proteomic profile of *T. gondii* bradyzoites. Pittman et al., 2014 (BMC Genomics 15: 806) aimed at providing a comprehensive analysis of *T. gondii* and the host during both acute and chronic infection. For this, they collected RNA-seq data from three experimental groups of mice: uninfected, 10 (acute infection) and 28 (chronic infection) days post-infection. Tables 3 and 4 list common *T. gondii* differentially expressed genes which were more abundant >5-fold in acute vs chronic infection (Table 3) and chronic vs acute infection (Table 4). The skilled person can thus clearly identify whether a *T. gondii* protein is an acute phase infection-specific protein or a chronic phase infection-specific protein.

In one embodiment, the protein composition of the present invention comprises at least one, preferably more than one, and preferably all of the native specific tachyzoite proteins listed in Table 1. In another embodiment, the protein composition of the present invention comprises at least one, preferably more than one, and preferably all of the native proteins specific of bradyzoite and/or cyst stages listed in Table 2.

In a preferred embodiment, the protein composition of the present invention comprises the native specific tachyzoite proteins listed in Table 1 and the native specific bradyzoite and/or cyst proteins listed in Table 2. Hence, in a preferred embodiment, the protein composition of the present invention comprises all native protein specific of tachyzoite and bradyzoite and/or cyst stages listed in Table 1 and Table 2.

**Table 1: List of specific tachyzoite proteins (native specific tachyzoite proteins).**

| **Accession** | **Description (transcript product)** | **SEQ ID NO.²** | **Amino acid sequence** |
|---|---|---|---|
| TGME49_213280-t26_1-p1 | SAG-related sequence SRS25 | 6 | MRTSRLLCAFGAVLLCWVPSWTAHAEEHFAVFSTCR TSFGRKRILVHSGDSVTIQCPGAIASNPQDVSKYVCP GEEPNCTDATKATYQTMFPIAPPDFQFWSGGDSKSN YGILKIPEVKDPVTISYSWQAAHSTFTGHAANSHLILD VNPKEEEVTRTVTSTVVVVSGCETQSLFTFLLVVASGI AMQQVL |
| TGME49_233450-t26_1-p1 | SAG-related sequence SRS29A | 94 | MVRTSLIEHRTGRRVVAAPDRGSLQVCIVFNIALSATI |
| | | | AAMMTSPLSVASMTSPLLTWDGNKVTCHPEKGDVD DWIGLVPQQNEVTFACEGKGVTPVPKNLVNSKSREV CAQGMSANACEKDPRPLSEILKGAQNDWLNGEDLS RGITFRVPKNNFPGLPRIFRIGCRTRSNICMISLHIYAK SAYTSGQVTECAYESNVSLPPVRITPATNHATIVCGPY GGISPLDYKKEFCTGEPHTSCGRQKYADVLPRYTDAW WNGDPSTAEGATLTVPNGHFPKETQTLKFMCLERLD TQPRRCVVTVEIAAGVSGSKPSGGESIPPQAPGTEGS RKEAPNTNNSGGTPTGGGSDDDTTAPGTGSSGTDS RGDLGDGRDQDAKGGMSRGNSESGSNGGATQPSR LLGLPSAVIGTLAAAMFGAV |
| TGME49_233460-t26_1-p1 | SAG-related sequence SRS29B | 17 | MSVSLHHFIISSGFLASMFPKAVRRAVTAGVFAAPTL MSFLRCGAMASDPPLVANQVVTCPDKKSTAAVILTP TENHFTLKCPKTALTEPPTLAYSPNRQICPAGTTSSCTS KAVTLSSLIPEAEDSWWTGDSASLDTAGIKLTVPIEKF PVTTQTFVVGCIKGDDAQSCMVTVTVQARASSVVN NVARCSYGANSTLGPVKLSAEGPTTMTLVCGKDGVK VPQDNNQYCSGTTLTGCNEKSFKDILPKLSENPWQG NASSDNGATLTINKEAFPAESKSVIIGCTGGSPEKHHC TVQLEFAGAAGSAKSSAGTASHVSIFAMVTGLIGSIA ACVA |
| TGME49_233480-t26_1-p1 | SAG-related sequence SRS29C | 18 | MATRASLARQKTGAACLAVLTASPSVVAAKPWKRW VGVSVILASAFAVGVSAGPPYRYEPEKFTCRPKKGILS QWVSLLYQVQHNITFACEEATPVPTTLISEEHGLMVC AESMTPEECEANPAPLSAFLPGATKEWVTGDSVLTG LKISVPESQYPANAKSFRVGCRHNTKTGNTCMLTIHV EPRDPAVERQEARCSYTENSTLPKIFVTKDSNTMTLA CGPHGAPMPESYTENYCSTPDTCDEKPFTSVIPGYLS KWFFGDPKSPLGAMLRIPPEQIPSSPQIIYLGCTGPTD GEGPKYNCTVPVQLGGEDPSEGSRPGGGSGGGKRG GGQGGGGGLAGSDSRQGSARHSLPSGMALSASALL AMTALAY |
| TGME49_271050-t26_1-p1 | SAG-related sequence SRS34A | 31 | MSFSKTTSLASLALTGLFVVFKFALASTTETPAPIECTA GATKTVEAPSSGSVVFQCGDKLTISPSGEGDVFYGKE CTDSRKLTTVLPGAVLKAKVEQPPKGPATYTLSYDGT PEKPQVLCYKCVAEAGAPAGRNNDGGSSAPTPKDCK LIVRVPGADGRVTSGFDPVSLTGKVLAPGLAGLLITFV |
| TGME49_285870-t26_1-p1 | SAG-related sequence SRS20A | 35 | MVSCKRLAASRRQCLFFNACLFCLFICTVQIRAIAGAD PEKCDQDGSLLNLRVTGNSSVEFKCGGAVPNLHPNP GKTPPIVCENSSCTNEVPLNIPNATLVKDSSGKATFTV SDASVGATTVYLKCSSLANTAARVGAGQPGQEAEKE KTCIVQTSVWGPPIKGSKEYPTPDACKNGTLSLEVNSS KKSVTFACGDNETLSPELFDMVFSTKECTTSSTLAEHL SGASLVQHARTNAADDKKPAYTFSVTSLPTEEKTLCY QCTKKNAQMKGEAQNTCKVLIKVAKQETDTGSTTV APPNESRAEHGQWNTALMISLITIPVVLMKML |
| TGME49_308840-t26_1-p1 | SAG-related sequence SRS51 | 46 | MELNLATQNASLGVTQATRAKSVSLNVMQTTTGSV MVGQEGWLEDGAAMTDNKHDCQLSVVQFVPLGSI SVKQWCYFKVGYVYACLEYNGGLAKRQQGAEPPSLS VTALFITMAVPREISWTQSQREMRLQIPYGRLLTRQS TLFFVALLAVVLHDSFVAFHGHLCHFAEAAAHQGSCT |
| | | | SQGTSSPMQVCTCNVPEQGETSNHAVVVSAEKNQL MAKCAIGTVTLPVDLMEKQVVCPAGMTDPQSCVSP SGTIPFASLLSGDGASAVAWVGDEVNYAAVLQVPKQ NLPPVDKEFIVGCTQEGKGHCLLHVTLSARASSKDSQ TVTCAYGSASNADVHRVTVSPKDPTFTLDCSSDGIM QPASFTTSYCPATGPLQENCDEKYASILQGYSSSWW QVSQAGTSNKAVLTIPHDNFPEAQKKVLVGCKASTAI PNSTLCTVELTITSSSPSSAWFLSLSVTMTLAVAGAVS SLV |

| | | | |
|---|---|---|---|
| ¹Accession number and protein description for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format); ² Example of predicted sequence for the corresponding protein on the ToxoDB database (ToxoDB-51 TgondiiME49 AnnotatedProteins.fasta, Last modified: 2021-03-06 09:21; Size: 9.2M | | | |

**Table 2: List of specific bradyzoite proteins and tissue cyst proteins (native bradyzoite and/or cyst specific proteins).**

| **Accession** | **Description (transcript product)** | **SEQ ID NO.²** | **Amino acid sequence** |
|---|---|---|---|
| TGME49_207160-t26_1-p1 | SAG-related sequence SRS49D | 52 | MAAAHSAAACRYSTFWPCLLRERQSGTSSVSFVYPS QFLSLSLLVILTGSFAQQSAGNQANSQSVTCESNASPL VLRITSKTNEVKFKCGTDLQLRENPAGSNKFWGNAA CTKEVDASSVTFTSSPSPAKVAGNKGTEYSLALKNSSL PLSPFTVYFSCDPPSTTGVGETGKAKVPASATTCIVQV SVFSQTAVTVPETNKCKNGQVTVAVTSKSKSVTFGCS EGATLKPALLDHVFIEKATEKSGGASTGREEEVVLQDL VPNSSLVENAANTGNDTVGYTLSCPDLPSSPQNIFYK CVSPASAREQVGTQTECKVLINIEEKPEAETPATPEPS RGEQGVVLGSAFMIAFISCFALVAGNMF |
| TGME49_208730-t26_1-p1 | microneme protein, putative | 54 | MVPLSRMAMVSVAYFILGALVYGSANVFGEMQPRQ LAKSSTDDPTIHDEGHGAAAPSTPRAGTELMTPHPLA SRAKAVTSAIIQESKSTLFNTGHVTTTEHAPKNPGLLG KPVKQTEALQLASPEKGVTTMEPKAASQHADRSGNP AVKKSTSGAQQAAAQSGKVLTKLTPIEKNPAPLHRQP MTRATPVGPKPADPAGKAVKKAATGIPKPAAPGGK AVKVTPVARKPVAPKAAAPDGKAVKKATVVVPKPAA PSGKAVKKPVVSVPKPATLGGKAVKKPAAGVPKPAA PDGKAVRKPVVGVPKPAAPDGKAAKKPASGVPKPA DPAGKAVKKAATGIPKPAAPGGKAVKVTPVARKPVA PKAAAPDGKAVKKATVVVPKPAAPSGKAVKKPVVSV PKPATLDGKAVRKPVVGVPKPAAPDGKAVKKPVVGV PKPAAPDDTGINKATLVTRKPEAPDVKVVKKATVVVP KPEAPDIKVMTDQTPTTPPSPPVRARAASATTIVTAP APVPTRPVLTTTSTAATVTTIKTETPRAASRLQQDLNA FCTTQFQKLCSETIDGGRYCGVSGAVARFGPGGHDS DSQWRCYEESIMNTTTKGVQCMDDCGNQMQCVG |
| | | | AVSVKTVATPPLRALSAFISQRRWIYCSSFQRSADALC NFKRTGNVARYDPGRTRWMCFNPVDISLEGQSYCA NNCGGFTPCAGGLSPGEIPTRTVYIIQQSDTAEAFAAL HPCRHDEICIPSTVYPPACVKAHKIIAIQQLSKQQEAM DNYCQTEMDQMCRVGKWTTYCYQLWLSRMVSTEE DEEPKWRCYPFALLNFSDLSTCIDGCSNRVPCHGAPS PTSTLTTEFSKLKTIAENIAFCSPFQKAANDHCNKRYG EGWVARQDVTTDDWACFQTTKLPTYRWTGCVDNC GNTSLCAAGVPEQLHSAAAEAFVHPDPALERVVESV PSPCLTGESCTPTAMNPPYCSETRPNPQPAPLRDHTI RNALDCSKGAPLARRNPTSK |
| TGME49_253330-t26_1-p1 | Rhoptry kinase family protein, truncated (incomplete catalytic triad) | 72 | MANTSVRRRQLLSSVLLLQWLTTVLGVAWGPNPIDG SKHGQFPSLRRTEGVSQSGSGHRGSVYRIPLADMTF WSWMSYLKELPDIDESRNPILKRLLSGSFLRRDGSTTV NVRVPARELVRLLSLTPEQQREGVSAKVRLINLLDPKY SVYEPYLYREILPKRSPLLLPSLGEYRGAFLTYIFHPLSKG LVGDMLETGRSHPDVQVLAANMVAALKSLHNLGLL HRSIELNSFSVLPDGTVVLGGLDTAAPIGTETRLWVGF FGQETAPEIDRNFLADLALTQGRHTVKSDVYSLGVAF RNLVQLLGNGNLGPEDRGAVRQDHLELLDKLSQKMI EEEPGNRPTIEEIMKDPLFEGLNFEDIEEGKARPFRYK QNRL |
| TGME49_258870-t26_1-p1 | hypothetical protein | 95 | MRRYTAFNTRLIELSASRRATSYSVLFPSADRFTMSFT PFFVTLGCDADEYLIIEQLVCCDHSDLTDAPSCCIVAC VPCPPRCTWTIPSDESVLRCCFLATPICIVMRFVLSLF MMECAVGRSGIPTKCRCFLGCPFPATSRSSTASRHYL LLFFLDVLVISVTVDIYVRAYPIERVRAHTGDERPFNVT TGSSERRGNTPHDGITWIAEHLADTDSGKDSHDYRFL NDDQHNHVEEGDGEVGGRTAAADEEEESSRILPTFT VQDIPSGDADVHKAAGDVDTSTTTAVDEDAVGMP TEGDIPKGDKQVYEAFGRQGSRTVVAEVVEDDSKDS RASQETMDNINHTTQRLLRGMDDGAFVRGGGVQE FVKAMTVPRIEEKTKGQLAGVLRLIEALEAMKGSNSK SDQSPRRFGCSVLQRPFRTVVDYDVVITTAAALANAG VNVAAGGGGPVFPVFPDVAKVRSLAEEPLDGQQAA ARESQLRSRGFVVPVADMVAAVKREWSEEPIDCGRC TVGGGYLPVRLLKKEVDEGEELLHFADVEYPTSISRGT VVSVPASWLIVKGLSLRELTPGVTGFILCVENGEKPVF VPRGQGLFSQYAEYRAGAFRERVAVDQFVIVRGSRR NESVLEAKLILDPRAYSVYQSVNRYDPSFLFFGTIEGPP ITQSLFVTSALKTSNECIIERTVDDGPVSLDCKGWRKF ERRMREYEETKAAITKRSRWRLIKYLVLALGVASAGLL YAGHRKERPHGILRVRPSQIMKHKYYDKIMVALRGR RHAARGYWKAFLDRGIQELLVPQPALHLLSYFDAMH PENLRDALRTVRGDTDTVSRWMDWASGRREAAKG VLYKGAGGYGMAATGVIATLYGLLRSGTYAYRRSKLY LRGRKVKQYAANVLSQKNGRRKARRMIVFLVVQ |
| TGME49_259020-t26_1-p1 | bradyzoite antigen BAG1 | 74 | MAPSASHPPGACPPGCTKHPATATAISPSGVCPMRA FHPAGPHSHFSCYDDLRNRLSHDKNVRPVASQQLDY LDEVSPFALAYYPPPFWGGVGLNPIDDMLFETALTAN EMMEDITWRPRVDVEFDSKKKEMIILADLPGLQKDD |
| | | | VTIEVDNGAIVIKGEKTSKEAEKVDDGKTKNILTERVS GYFARRFQLPSNYKPDGISAAMDNGVLRVTIKVEDSG GAKQQISVK |
| TGME49_264660-t26_1-p1 | SAG-related sequence SRS44 | 96 | MTAPFLRVYTDILSERKVRVEGTWHLVSIKATHDQPL CACKQTWSVHFAWPVDTMKLSYFFFALSSSGATLLLF AVTQLATQVSGFDLEEPRDLRHEMKKIEVIHGSEGAR FECPVESVLLPEQAMSGDIRAATVFAVAADGSCNIFD PPSLLGDVVPGAYLQEELSVNADKAAKTYTLVIPGLPA KEVKLCYVCAHPDNPAGSQVTVITVRRSGDSENGNP ASSPVCRPSFSLEAWALGVRQPSVVLDVAAPGQSVR FSCLSTATELPSEAGARPAPAQVYPVNGRGWCDLSK GPSSLEKFLLGAAMTAEVRQRGGGFLTTYTLNVPRLP STNRRFCYVCPRNRDPADGCAVLVKVRGGMVEDPI GKPAAVPTCHPQESPEGIAAGTKSAALILDIVRPYSSV KFACPNNSEVLPHNAVSDDGLKASVFAVRKDGSCIIG EHTLKLQDVVPGATLTGTTLVDKGSVTRVYTFFVGML PTRSQKVCYVCARNSLAPQGCGVVISVKGKENGKPA KVSVCEPQYLQGVAERGLLSPSVELLVDKPEGVAQFV CPGNSVVFPEHATDESPGIATVYQASNANACDVWE NPVKLDNVLQGATLRADTSDEDTMMAKTYTFKVPA LPSTETKLCYACVRDGEIANSCGVMITVKPKGPTTEEI PSALLERGLCLPVYSTDVAKTGLRRPSVVLKVDKRNRV VEFTCPHSSLVFPDQAADSDAGEAVVYTVSGGSCDV WENPVYLTKILPGASLVSETAVDASEITTKYTFTVKQL PKTPKKLCYLCVYNRDVMNSCGVLIDVSSNSMTRLSK TSEEGIHTNPTVPTTLELQCPPDYSARTAATGLRLPEM ELLVDSVNRTARFVCPDDSSVFPEIDGEADPASGKVY RMTGDSCDTREETAFLQDEVPGAELQRRTVVDGGV VQRYYIFKASRLPEEDKKLCFLCVRQRNPADSCAVMIT VKADSAAAVPIHPFRPRPAVKLATYVCKPENSTEVAE TGLKAPALSLTIDSRAHSIQFICPESSIVFPDLAAVPDQ DSATVYLVGEGGSCNVAASPRLLSDVLPGATLGSRTT VDEGVVNRTYTLTAPKLPEEDVKLCYLCVRNRQIAESC GIHITVKGTKAESVAKPAGPICSLGYSPGSEKTGMREA AIELVVEDPSTPVRFICPEGSYVFPEYATSTDKGIAAVY GVKGNECIFAGPRENLQSLVPGATLRMEAADEGGTV MRTYTFTTPEQPAEDRRFCYMCVRERDIYQSCPVIIV VKGSGHLPEPKAQPQEEHELPVCTPHEQAGTALTGLL DPSVKLWVNEPEEQVSFVCPDGSAVLPDQETSEPQV YAVGAGEYCLVTKPLMSLAKAVPSASLSTDTVVENEV TTRKYTLRVPSLPLEETKLCYTCVSEVDPAKKCGVSVT VKGEGKMPGGLTSSLPSPVCAAQFSQCAFLTGVRMP SIMLTVHEPNTKLVFDCPPNTALYPQNAVVPGKATFF PLNEKGACIYKGPVAESKLYDVVPGARLRAKETVDGA VVRSYTLTVSKLPKKETRLCYLCVRGDEPAYSCGVIVK VKAPAPAAPEPKQLPVCVPSSDPSVGKMRRITPKLTL KIEKPGGSASFECPDGSTIFPDHADALDPKCVLAYKVV RDTCDLRGPTEMVASKLQGAYLTKTFVGTGYKYTFTV PHLPAEQQQVCFMCVMDRDTSRSCGVVINVMPHP AASRSSVPECKLKHVPAAATTGLREASLILNVREPSSV VEFGCPRNSVIMPSTATSSEHLAQVYSVRDRHICDM |
| | | | KEFLRPLADLVPGASLKLVHTPEDGDTTRTHMFRVGS LPVKDTALCYLCVDPGDHSHNCGVVINVKGSEVPPPP KDFGCAPSHSEEVERTGKLSPAVRLMVTSTKEPIRFEC RDNAKVYPPAASLIKPAKAVVYPVSAGGRCIVGKPPL SLNRVVPGAKLKTKAKLVTGAITTTGFVFVPVLPSIQK DFCFACVDRGDITNSCGVLVTVPPFWAPPVQETVCN DVKTLTMTVHSPGSVTLRCGLEFPKVDPPIDDPASQG QVYYGKSCGDVGALRTVIPGASVVKEPFYHNLAKEVA VGVTLTVPASIVKQTICFKCRQPDPLQHETCTVRVTVL PPAAQPTTTTTTTTTTIKPTTTTTTRKPTTTTTTTRRP TTTTTTTKKPTTTTTTTRKPRTTTTTTTKKPTTTTTTTR KPTTTTTTTKKPTTTTTTTKKPTTTTTTTKKPTTTTTTTT TRKPTTTTTTTKKPTTTTTTTKKPTTTTTTTTKKPTTTTT TTKKPTTTTTTTRKPTTTTTTTRKPTTTTTTNKPTTTTT TRTPTTTTSKPPVVFELPSEFIEETATTTRATISLRARSA AAPTSTTTVGPTTPATSLFDWSSKVASLAGQWSFSG ALQGTAGSRSGVYEAPITQLPPPLLVPFKIDCCGPPVI QKGCCPPPLIQVDCCRPPVLQQKPGCCCCGHE |
| TGME49_270240-t26_1-p1 | MAG1 | 80 | MDCGQCRRQLHAAGVLGLFVTLATATVGLSQRVPEL PEVESFDEVGTGARRSGSIATLLPQDAVLYENSEDVA VPSDSASTPSYFHVESPSASVEAATGAVGEVVPDCEE QQEQGDTTLSDHDFHSGGTEQEGLPETEVAHQHET EEQYGTEGMPPPVLPPAPVVHPRFIAVPGPSVPVPFF SLPDIHPDQVVYILRVQGSGDFDISFEVGRAVKQLEAI KKAYREATGKLEADELESERGPAVSPRRRLVDLIKDN QRRLRAALQKIKIQKKLEEIDDLLQLTRALKAMDARLR ACQDMAPIEEALCHKTKAFGEMVSQKAKEIREKAASL SSLLGVDAVEKQLRRVEPEHEDNTRVEARVEELQKAL EKAASEAKQLVGTAAGEIEEGVKADTQAVQDSSKDV LTKSQLALVEAFKAIQRALLEAKTKELVEPTSKEAEEAR QILAEQAA |
| TGME49_280570-t26_1-p1 | SAG-related sequence SRS35A | 83 | MTKNKILLRAVLCFLILFGCSAWRVRAKSWTYDFKKA LDDDETKKEIITPGDSVSIENSGSRPLEYIPPNPSQVLK VEKGDHCKDEPVELAALFKGSAPTPAWTEAQSTRSLT VPAGSVPEKETIPFCFKVKDTGKNKTLTAVIKVAGAH GLSAAVGVSIGIPALAFVLISM |
| TGME49_320190-t26_1-p1 | SAG-related sequence SRS16B | 93 | MERTGNMQQRLGGFKSQVRKLMAVCIGGILLLSSG QGLADSLHEGLQSLSLKSQVGVSSKVDVTGTHAKCEF STAGAGSSTAVSKQSLTLSKGSLTATLECTAKDGGTIS SIPNPLAGNVCVTKQKQSETPCNFGENGGAAASVQV PLKDVLGTTSAVQWMNTTAEQKAIQSQKWTLKLNE EDLPISDKTFDVGCTYTTTGKAAANPAVCQLTVNVKA RASSVADENVVTCSYGAESNSEALKVDMTTTRNTVTI DCGSEGTLNPAADSAQYCNPQNKDLDDCKKKFVDIL PNFVISWWKTPTPGNASILTIPAADFPESEQQFRLGC VPNKAPQSDSQDTRVDNQSQVQSRTSAPTTCSVLVT VKAASSASYASPSLQTLIAVSGAAVVSGFVVASL |

| | | | |
|---|---|---|---|
| ¹Accession number and protein description for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format); ² Example of predicted sequence for the corresponding protein on the ToxoDB database (ToxoDB-51 TgondiiME49 AnnotatedProteins.fasta, Last modified: 2021-03-06 09:21; Size: 9.2M | | | |

In another embodiment, the protein composition of the present invention further comprises at least one native protein specific of the acute phase (specific of *T. gondii* acute infection) selected from Table 3. Preferably, the protein composition of the present invention comprises more than one native protein selected from Table 3, such as 2, 3, 4, 5, 10, 15, 20, 30, 40, 43 or more. More preferably, the protein composition further comprises all native protein specific of the acute phase listed in Table 3.

**Table 3: List of proteins associated with T. gondii acute-phase infection (native T. gondii acute phase infection-specific proteins).**

| **Accession** | **Description (transcript product)** | **SEQ ID NO.²** | **Amino acid sequence** |
|---|---|---|---|
| TGME49_201780-t26_1-p1 | microneme protein MIC2 | 1 | MRLQREAVFGLMFACGMWMWPSEVAGGGWSIVDALRKRYDTSR GGDANGVDTSGVEDVIQSESAIGAAEGCTNQLDICFLIDSSGSIGIQNF RLVKQFLHTFLMVLPIGPEEVNNAVVTYSTDVHLQWDLQSPNAVDK QLAAHAVLDMPYKKGSTNTSDGLKACKQILFTGSRPGREHVPKLVIG MTDGESDSDFRTVRAAKEIRELGGIVTVLAVGHYVKHSECRSMCGCS GTSDDDSPCPLYLRADWGQLATAIKPMLKEVCKTLPQDAICSDWSA WSPCSVSCGDGSQIRTRTEVSAPQPGTPTCPDCPAPMGRTCVEQGG LEEIRECSAGVCAVDAGCGVWGEWSAWSASCGNATRKRERTRYND PPPQGAGRRCENQDPPVLQEQTEEATLAPCITIPPTPPEWAAWSDCT VTCGGGNRHRVRNALPPGLGSQNGESDESLVSKLWPGTDLRQEEAC NTSPCPINATCGQFEEWSTCSVSCGGGLKTRSRNPWNEDQQHGGLS CEQQHPGGRTETITCNPQACPVDERPGEWAEWGECSVTCGDGVRE RRRGKSLVEAKFGGRTIDQQNEALPEDLKIKNVEYEPCSYPACGASCT YVWSDWNKCVCPMGYQARHAAVKFDYRNKPCDLPTFETKACSCGE TNPVPSEGTTPGAPGATGPEQPNQRPLPEGSDENETPTNEEGEQSKK ESGSGIAGAIAGGVIGGLILLGAAGGASYHYYLSSSVGSPSAEIEYEADD GATKVVMEEEKETLVPVDDDSDMWME |
| TGME49_204050-t26_1-p1 | subtilisin SUB1 | 2 | MGSSHAIVACAALIVLLSTNARGLRVRKDKDVLLATSFLSHHGEYQNP TGTYNLIKEIRRVEAEIEDEVETLNRDRRLHRGHNKYADDDIRQGLKGE QDMGASENIPVAELEPQDLDREAKYPVRMLIVDKRSDDDDEETKTSF VETALHSDLAQRVVKELNGHVDVLRESGVVLVDLPAQTTDKQLQELIE TARAQGTIVEPDHLVQSVNTSSKGSNDPLLDRLWGMDALNVKEAW DIITTGEPNMGGRRPLVCVLDTGIDYNHPDLRDNMEVNQAERDGTL GVDDDNNGEVDDIYGANMLSKENDPADDHSHGTHVAGTIGAHGN NGIGVAGVAWAPRLLPCKFLAYTGRGYSSDAVRCIDYCVKRGADIVN HSWGGSWPSEALREAVVRTANNGLIHIFAAGNDGVDIDQTAFYPAA FSTEADGLITVANVKGDPDHGGKRIIELDRSSNYGIQRVQVACPGMW ILSTVPTSGSSQEPYAEKSGTSMAAPALSGIVALMLAVNPGLSTRQVR EGLRQCSVQQPLLQGKVEWGSMPDAKRCVEYALTTHAEGRHKSFIR |
| | | | EPSTETSTPPPPPPAQPTPQPQPHPPPQPETPPCAPSPPPPTPPCAPS PSPPTPPPGSPHKPEPQTPVYPEVPRSTRSPPPSPPPTESAPEAPPSDT PSCRVPPCSSSPRSGSQPKPPQDNTTTPKMPSLSSPPTEHSTAQPPKH ENDAREQEPPTDEDDFSSVKGKKLGAYESDGSPRASSCAGAGVLGVF FMVVGLTV |
| TGME49_205470-t26_1-p1 | translation elongation factor 2 family protein, putative | 3 | MHPSGGQFVPTPQRHGPDLTKSWGLINRDFALRTREWAVSQRSLVF PVAVAAVRGETAPIAGRASQCPRLLVPFSWVLFLEQRPELSLTKVVFLL WNLYFVSLSPLRPFTGSLHPRPVVKMVNFSVEQMREIMGNPKNIRN MSVIAHVDHGKSTLTDSLVSKAGIISAKAAGDARFTDTRADEQERCITI KSTGISMYFEHDMEDGKGAQPYLINLIDSPGHVDFSSEVTAALRVTDG ALVVVDTIEGVCVQTETVLRQALAERIRPVLHVNKVDRALLELQMDG EEIYQTFSRTIENVNVIISTYNDELMGDVQVYPEKGTVSFGSGLHGWA FTVERFSKIYAKKFDVPKEKMMERLWGNNFYNAKEKKWTKTQSENT RRAFCQFIMDPICTLFTSIMNDEKEKYTKMLGSLGIELKGDDRDLTGK ALLKRVMQLWLPAGDTLLEMVVRHLPSPFAAQKYRVENLYEGPMD DEAAQGIRNCDPNAPLMMYVSKMVPTSDKGRFYAFGRVFSGTVAT GQKVRIQGPRYVPGEKTDLYIKSIQRTVIMMGKYVEHVQDVPCGNTC CLVGVDQYLLKSGTLTTLDTAHNIADMKYSVSPVVRVAVKPKDNKELP KLVEGLKKLSKSDPLVVCTTEESGEHIIAGCGELHVEICLKDLRDEYAQI DFTVSDPVVSYRETVSAPSHMTCLSKSPNKHNRLYMVAEPFPDGLAD AIEAGQVNARDDPKERANALAEKFDFDKNAALKIWCFGPETTGANM LIDTTQGVQYLNEIKEHCNSAFQWASKEGVLCEENMRGIRFNLTDVT MHADAIHRGAGQIMPTCRRVLYACQLASAPRLQEPMFLVDITCPQD AVGGIYSTLNTRRGHVFHEEQRSGTPLVEIKAYLPVAESFGFTTALRAA TSGQAFPQCVFDHWSTLNGDPLEKGSKMEELVHNIRTRKNLKPEIPP FENYYDKL |
| TGME49_210370-t26_1-p1 | hypothetical protein | 4 | MATVPMVSLSAARFTLSFLFFQLCLSRLFRPEGRNELEGPKDVDADVI RALDLASVFAFAEAVNNPPPLEGASVSPENATDPPETGGSRRWRTAR YVLRRPFTHIRRVSSAVRRYISRGTSSLASSFAWKRRLRKLIRGMAKDL KQNRVRDAQQKPGSDAVASFVKTPHALLTSLGTPPAELANAVGLLLP VQHPVPMKSKVTGKSLLVSHAATVGVSSYGSVLLRCEGSGESASYFLE AFPVLRGSREASVSVRKQYLAAIKELRAASGVAQVGVVVDLLLLKRVR NTWCPRPHESHFAFPNVFLKRAVEGETLREVLLHIASTAATDTPGTGP AHDARVSLTEQVHKLARSAAELGSRGGKINIDTFFVTLDGIVHLQPVSL VESPPVHRGSAGLESPIAAELEEGEEKPSPEKRMTDDLALAIVLAQIWC PQRRFDDVAYELLLLHRFEREVTEEFFERCADPPEAVQVLVTMLAF |
| TGME49_211290-t26_1-p1 | rhoptry protein ROP15 | 5 | MLKTTPAFFLFLTWMFPRCDGFVLTHKNGTTDAYDSAVLSFLLTTEGV REGAAKSSGTEIPSTLACPSSSPLKTAKDKHDASLLGLVSSVPDSSILKPE FVGAGAALVAVTRSVKWTQLKQSLKALVDTGVKCKVNCKHNNKKLS KNTDFKDQQEFPQTSARILPTSDREPLIAGPQKVTEEDLQVLLFEIAHD CKMSAPQAAITKPLTILLAGIPGAHISKRPFEAAIPHFILQKEDTVDLLLT VDRSWCGKGLSHLVRYHVGYMSFNLEVNGRRLADDDTFEDDRLYRQ VVLTTKFTAKSLGGTLKQVRSLTDIHKKTGKHGENRPHSSPF |
| TGME49_213280-t26_1-p1 | SAG-related sequence SRS25 | 6 | MRTSRLLCAFGAVLLCWVPSWTAHAEEHFAVFSTCRTSFGRKRILVHS GDSVTIQCPGAIASNPQDVSKYVCPGEEPNCTDATKATYQTMFPIAPP DFQFWSGGDSKSNYGILKIPEVKDPVTISYSWQAAHSTFTGHAANSH LILDVNPKEEEVTRTVTSTVVVVSGCETQSLFTFLLVVASGIAMQQVL |
| TGME49_214940-t26_1-p1 | MIC2-associated protein M2AP | 7 | MKLAAVSSAAFAAAFFPAVVNARKVGNPAAQPSVLVNEPVALAQLS TFLELVEVPCNSVHVQGVMTPNQMVKVTGAGWDNGVLEFYVTRPT KTGGDTSRSHLASIMCYSKDIDGVPSDKAGKCFLKNFSGEDSSEIDEKE VSLPIKSHNDAFMFVCSSNDGSALQCDVFALDNTNSSDGWKVNTVD |
| | | | LGVSVSPDLAFGLTADGVKVKKLYASSGLTAINDDPSLGCKAPPHSPP AGEEPSLPSPENSGSATPAEESPSESESVPSLPLSQIPSEVPLSQESPVPP QDGENPDSPQNEDNSMQEVENSASQSDIEAQRQAENGRLPSDDEA |
| TGME49_218410-t26_1-p1 | ribosomal protein RPP0 | 8 | MAGPKGKSDKRKTYFSRLFALLEKYPRVLVVEADHVGSKQMADIRLA LRGKAVVLMGKNTMIRTALKQKMSEMPQLEKLLPLVRLNVGFIFCIE DPAEVRRIVAENKVPAPARQGVFAPIDVFIPAGPTGMDPGSTSFFQA LGIATKIVKGQIEIQNEVHLIKEGDKVTASAATLLQKLNIKPFEYGLAIQ HVYDDGSVYKASVLDITDEVILEKFRAGTMNVAALSREVGFPTTASAP HSILEAFKFCTSLVLESDYSFPQMQRIKDILENPEAFAAVAAAAAPAAG AAAAAEAPKEEEPEEEEDDMGFSLFD |
| TGME49_219540-t26_1-p1 | cytosolic tRNA-Ala synthetase | 9 | MLDVPILCALLVLLLSSLPAGAGALCVSLHRVSPSCLHSWEGGGCGFS SVFPPGRFASDCSERRRLPQPSLTTENVSASLNSGSALSSPPRRLSDFV RMQPRLLQERLFTDLSFLSRPRQTFSSSRLGVFSSPALSPLRSSSSATRV LLNFSRPARPRLCSRSPRHPSPSFCSSSIFPQSPSRSSLSSTWISLLLSDV RLASGAASLSSPWESFSLITSAARRPDSRAFARIPLVPLSIHSGKMAMT EEERRAKIEEISAKGKAKGWSGEAVRGAYLEFFEAQGHLIYPSSPVIPR GDNTLLFINAGMNQFKPIFLGQIDRSSPLSKLRRVADTQKCIRAGGKH NDLEDVGKDVYHHTFFEMLGNWSFGDYFKREAIAWAWQLLTEVYKI PKDRLYASYFGGDPRLPSCPPDEEARDIWLQYLPANRVLPFGAKENF WEMADTGPCGPCSEIHYDRIGGRDASSLVNADDPSVLEIWNLVFMQ YNREKDGSLTPLPAPCVDTGMGLERVASVLQDKQSNYDTDLFTALFD KILELSPGLRPYGGKVGEEDKDMVDMAYRVLADHARCLTAAIADGAE PSNEGRGYVLRRILRRAVRYARQTLGMAEGNFPKLVPVVVSLLGETFP EMKKNEEKVVRILLKEEQQFSRTLDNGVERFKKVANSLQAKGQKVFP GGEAFTLYSTYGFPLDLTQLMAEEVGLQVDAEEFERKFDAHRAASEN TSFKQGGAGAVYALPPDQRHLLAQSRKVPPTDDSAKYEWNVKDQA ALMTQDAVEAAPIKAKVMAIWDGKRFAPEVTAVASPTGENLVAVIL DKTCFYAEQGGQIGDVGRLVDLSSQAEFEVVDSQKSGDFILHIGSMSK GTLREGTELVPAVDYVRRFRLAQHHTGTHLLNFALREVLIRKLRSGGK ASANGDKREDTNGIRICESCLNEKELEDGEDIQQKGSLVEVKRLRFDY AGEKPLENEDIIAVENVVKDCIAQARPVHYKEVPLEQAMKIPGLRAVF GEVYPDPVRVVSVGEDIESLVALADSSQPETPFASIELCGGTHLHNAS QLSDFCILSEESVAKGVRRVVAAAGDAAFEARKELEELEEAFRSLASAP RELAALEKEVAKARGLLDGAGPLPLVGKRSLVKQLEQAQKALLELQKA RSKELVKEAKKLGEELGTEHKTKPFLVLNLPQLLGDGKALDAASQTLH QAAPDLPFLLVTASAATGTAAIAFVPAACSQLHAASWLTACVGLLGG KGGGKGERAVGAAKEGAADKVDAAVAEATAFAEKKYGA |
| TGME49_221480-t26_1-p1 | hypothetical protein | 10 | MSSHDLRLCRPRRQSKGSRQSPAFSAGRLCSSWGSLCVCRRVPRTKL GGSPAPRTAGIHRDSKCCATAMMPFPKRSLSLVCLLFCVVTAAGTLPR QPVADAFRLENQGVQDSHANSDAMQPSLGSATNHKATGTTSFVSV NENEEGMSQKAIIVFVVAISAMVICIGVVLVMWWMSSEREKKRRHV ARDAERAPGLFFSEDEGPMSSDEKSAPKKPAGGNSPNTRDAGESISR ERQIIEEDLGKVLVVDPVSGVMRSRQKWQRNGNSVPDKRMLAGTER TCRTGQQKFKK |
| TGME49_221620-t26_1-p1 | beta-tubulin, putative | 11 | MREIVHIQAGQCGNQIGAKFWEVISDEHGVDPTGTYTGDSDLQLERI NVFYNEATGGRFVPRAILMDLEPGTMDSVRAGPFGQLFRPDNFVFG QTGAGNNWAKGHYTEGAELIDSVLDVVRKEAEGCDCLQGFQITHSL GGGTGSGMGTLLISKVREEYPDRIMETFSVFPSPKVSDTVVEPYNATL SVHQLVENADEVQVIDNEALYDICFRTLKLTTPTYGDLNHLVSAAMSG VTCSLRFPGQLNSDLRKLAVNLIPFPRLHFFLIGFAPLTSRGSQQYRALT VPELTQQMFDAKNMMCASDPRHGRYLTACALFRGRMSTKEVDEQ |
| | | | MLNVQNKNSSYFVEWIPNNMKSAVCDIPPKGLKMSVTFVGNSTAIQ EMFKRVSEQFTAMFRRKAFLHWYTGEGMDEMEFTEAESNMNDLVS EYQQYQDATAEEEGEFDDEDGGMDAEGEA |
| TGME49_222170-t26_1-p1 | dense-granule antigen DG32 | 12 | MKSGSCPFTFSPTLSHPLEIAWRARPLQWPEIERQPEPAAPCGALYPA SRVLSFSPTMRAIRSVVGLPVMGAAVFLGLLSGELPNSSVLPVPEVAST PFLLSVAADGDGGKNYAEVAKVEALTNMISTPIELFVKDVWQQLFHK SGKPVWENMLFKFGSMIRHRAAHKATLVIMWELRHFLYGTAKVNPS AWKKLETKFETYLREWWMTVPEDPWAALHAGAWKSLLKLYNEDLE PLLRGSPKLKDLESILFDSKLATIRRWTDEAHIEVMKGRTSNMVPRLEA LSAKMAVKQKAMQGKQ |
| TGME49_ 224460-t26_1-p1 | aminopepti dase n, putative | 13 | MVTLSATTMNATVSPCRISGGVSSRLCGWGVLSFLLASVITVQVSRA GEEKEEPKAVVRLDYKPPNFYLEDIVLDFNLDDEDTTVEALLTLYRRAG TEPMNLVLDGDESVILKSISLATTSSPSLRGQGRVTFTELDGQMPTDS QFQFRIVDGNLMVSKFLLPREAEVRFYIRTLVSINPKANLALFGLYKAG DIFVTLNEPSGFRRITYGVDRPDVLATYTVTVTAPRHLPILLSNGDKVLS GYAGENRHFAKFVDPFPKPSYLFALVAGDFASVSGEFVTMSGRSVTV TIYAQHHQRNQLQWALRSLLRAMRWDEETFGREYQYSEFRVLCVEV FNPGAMENTSLNIFTCSLLLADPKLTTDADHRLIVDVVSHEYFHNWTG NRVTVQDWFQLTLKEGLTMFRNNSFTEETTSRAMKRIGDVADILSIQ FREDSGPFAHPIRPETYKSIENLYTRTVYLKGAEVNRMYRTMLGPKGF RRGMDLYFKRHDGKAVTCDDLRAAMADANDKDLSQFERWYSQAG TPHVTVSSFIYDAAERKMHLTLKQHTPPTPGQDKKLPLQIPVAVGCIG KTSKRDVLSPPTQILELTEEEQAFVLVDVGEDCVLSVLRDFSAPVKLIQP LQTDEDLAFLVTYDSDNVNRWQAAQTLSRKVLLTRISEYQQRVEEIEE GRVSESDLFSKLPTQYVQTVRDIVIAPESSMGKDIKSLLLSLPTKAQLEL AVDSIDPDAINAALASVRRDIVDALGEEMLQLYTELTLPAGTEESGADI EHWGRRALRNELLRFLTASFDQKSAKLASAHFDRAMVMSDKVAALT VLTEIPGQERDEAFERFRQEVAGNAVLMEKWMSLQAMSAAADTLE RVAQLRQHSAFHPFIPGSAYSLFGTFSSSSPQFHSKTGAGYALVLDFLT MIDEHNSLVSSRIAGVAFANWKRFTSPRREMLKEVLKKLLKKEKLSVG LKEVLEKALAGEDD |
| TGME49_226570-t26_1-p1 | hypothetical protein | 14 | MSQPTSPTVSPGTPLNCGASERQLEVESSPMEGRVYTDNEIHSDGGH SVECPPPGSPIPQTTTFTSQAAPALQPRMMYPSTDALTIPAGHQQM MYFNPAAAGYTDGSHGYDPREGYPVVYPYVPMTLPIETKKKRGFCC |
| TGME49_230180-t26_1-p1 | hypothetical protein | 15 | |
| TGME49_232350-t26_1-p1 | lactate dehydrogen ase LDH1 | 16 | MAPALVQRRKKVAMIGSGMIGGTMGYLCALRELADVVLYDVVKGM PEGKALDLSHVTSVVDTNVSVRAEYSYEAALTGADCVIVTAGLTKVPG KPDSEWSRNDLLPFNSKIIREIGQNIKKYCPKTFIIVVTNPLDCMVKVM CEASGVPTNMICGMACMLDSGRFRRYVADALSVSPRDVQATVIGTH GDCMVPLVRYITVNGYPIQKFIKDGVVTEKQLEEIAEHTKVSGGEIVRF |
| | | | LGQGSAYYAPAASAVAMATSFLNDEKRVIPCSVYCNGEYGLKDMFIG LPAVIGGAGIERVIELELNEEEKKQFQKSVDDVMALNKAVAALQA |
| TGME49_233460-t26_1-p1 | SAG-related sequence SRS29B | 17 | MSVSLHHFIISSGFLASMFPKAVRRAVTAGVFAAPTLMSFLRCGAMA SDPPLVANQVVTCPDKKSTAAVILTPTENHFTLKCPKTALTEPPTLAYS PNRQICPAGTTSSCTSKAVTLSSLIPEAEDSWWTGDSASLDTAGIKLTV PIEKFPVTTQTFVVGCIKGDDAQSCMVTVTVQARASSVVNNVARCSY GANSTLGPVKLSAEGPTTMTLVCGKDGVKVPQDNNQYCSGTTLTGC NEKSFKDILPKLSENPWQGNASSDNGATLTINKEAFPAESKSVIIGCTG GSPEKHHCTVQLEFAGAAGSAKSSAGTASHVSIFAMVTGLIGSIAACV A |
| TGME49_233480-t26_1-p1 | SAG-related sequence SRS29C | 18 | MATRASLARQKTGAACLAVLTASPSVVAAKPWKRWVGVSVILASAF AVGVSAGPPYRYEPEKFTCRPKKGILSQWVSLLYQVQHNITFACEEAT PVPTTLISEEHGLMVCAESMTPEECEANPAPLSAFLPGATKEWVTGDS VLTGLKISVPESQYPANAKSFRVGCRHNTKTGNTCMLTIHVEPRDPAV ERQEARCSYTENSTLPKIFVTKDSNTMTLACGPHGAPMPESYTENYCS TPDTCDEKPFTSVIPGYLSKWFFGDPKSPLGAMLRIPPEQIPSSPQIIYL GCTGPTDGEGPKYNCTVPVQLGGEDPSEGSRPGGGSGGGKRGGGQ GGGGGLAGSDSRQGSARHSLPSGMALSASALLAMTALAY |
| TGME49_243570-t26_1-p1 | ribosomal protein RPS26 | 19 | MPTKRRNGGRAKHGRGHSAVVRCSNCGRSCPKDKAIKRFNVRNIVD ASSQRDLREASVYNTYTLPKLYIKQCYCVSCAIHSRVVRVRSVEQRKVR ENPQRRPQGGQQGQGNN |
| TGME49_243730-t26_1-p1 | rhoptry protein ROP9 | 20 | MTHPNPLQTRFSRSASLVSRMPLVRLFFTIAAPLLFSPSPCPFLPLKTHC LAIQLGKPQGSPPASQKEAIRDTGVSHQKGEPSDSSSEPKPQGTVAET PGAASAAAAEVGRPSRSSAGPGKKKRGPSLFTEETMGPSKRHPCLKTL AAAVERFQAQHLTGRDAAKAFVDAIQECGIQVVASDYDRTAISVHSG GSARRDDLSVLGALTPDFKLLGEELTRRNIPIYFVTFSDKGENRGDRIA AGPLVEATLKASNANFEAQGVFGYYPPFYSEPEDYAPLGLSAPMPTD KSFHIQQVSKASGVPEDKILLLDDDRANCVNFCRSGGAAIHVSGHEGF DFGAVRVVVKPSLIMQ |
| TGME49_246540-t26_1-p1 | cytochrome c1, heme protein | 21 | MGGGGGGALNKLFPGYKDKIWMKVPVQWRQQMIQHWNKSYEKQ VYSESVALNRTFQARNQLVLDRLKPSGAYRLPAVDYKRQLSRGTLVEG ADFYLPTAQEQQRLARHFEPYSEQEQEERRKFRFQSISVYLAVALGAS FVHDYFYQRRPVAWCLEKEPPHPPSYPFWFKSLFHSHDIPSVRRGYEV YRKVCATCHSMEQLHFRHLVGEVLPEKRVKQIAAEYDVTDGPNDQG EMYTRPGILGDAFPSPYPNEEAARYANGGAYPPDLSLITAARHFGPDY LMALLGGYRDPPEGVELRPGLYWNVWFPGNAIAMPPPLMDEMIDY EDGTPCNISQMSKDVVNFLTWATEPTADERKLYGLKCVSAIAIGTVL MTLWWRFYWAMYATRRIDFGKLKYL |
| TGME49_249180-t26_1-p1 | bifunctional dihydrofolat e reductase-thymidylate synthase | 22 | MQKPVCLVVAMTPKRGIGINNGLPWPHLTTDFKHFSRVTKTTPEEAS RLNGWLPRKFAKTGDSGLPSPSVGKRFNAVVMGRKTWESMPRKFR PLVDRLNIVVSSSLKEEDIAAEKPQAEGQQRVRVCASLPAALSLLEEEY KDSVDQIFVVGGAGLYEAALSLGVASHLYITRVAREFPCDVFFPAFPG DDILSNKSTAAQAAAPAESVFVPFCPELGREKDNEATYRPIFISKTFSD NGVPYDFVVLEKRRKTDDAATAEPSNAMSSLTSTRETTPVHGLQAPS SAAAIAPVLAWMDEEDRKKREQKELIRAVPHVHFRGHEEFQYLDLIA DIINNGRTMDDRTGVGVISKFGCTMRYSLDQAFPLLTTKRVFWKGVL EELLWFIRGDTNANHLSEKGVKIWDKNVTREFLDSRNLPHREVGDIG PGYGFQWRHFGAAYKDMHTDYTGQGVDQLKNVIQMLRTNPTDRR MLMTAWNPAALDEMALPPCHLLCQFYVNDQKELSCIMYQRSCDVG LGVPFNIASYSLLTLMVAHVCNLKPKEFIHFMGNTHVYTNHVEALKEQ LRREPRPFPIVNILNKERIKEIDDFTAEDFEVVGYVPHGRIQMEMAV |
| TGME49_253430-t26_1-p1 | asparagine synthetase, putative | 23 | MCGILAILMSSLSRDQLRQLALERSRLSVFYRGGLDQECMGKSLWLR HRGPDSNGVHVQEYSTTLCNALAHERLAIVDPATGKQPLCDASGTFV CVANGEIYNHVELTKTLLPPEEVAKWKTASDCQPLPSLFKFHGPSICDK LDGIFSFVISNGATGEFIAARDPLGVCSLYVGYASDGSIWFASELKALTR DCEQVTVFPPGHFYLSTMNEGKGGFQRYYNPSWWGVDKPLPTYRC DLGQLREALEAAVRKRLMCDVPFGLMLSGGVDSSIIAAIAAKEFQKM SATEKGSHIWTNQIHAFSIGLKDGPDRKFAKMVADMLGTVHHEFTFE LEEGLDALDDVIYMIETYDITTVRASVPMYLLCRMIKSLGVKVVLTGEG ADEVFGGYLYFHSAPNREAFHRELQNKLNLVHFYDCLRANKTSMAW GVEARVPFLDRGFLDFAMSIDPEDKMCTNGRMEKQILRDAFKGYLP DEVLYRQKEQFSDGVGYAWIDHLKSYAEKKVTDTMMRNAKLLFPTN TPVTKEGYLYRAIFAKHYNKGSAARTVHTGPSIACSTAAALEWNEKFQ ECIDQSGRSVDVHQFHQGHRD |
| TGME49_254720-t26_1-p1 | dense granule protein GRA8 | 24 | MALPLRVSATVFVVFAVFGVARAMNGPLSYHPSSYGASYPNPSNPLH GMPKPENPVRPPPPGFHPSVIPNPPYPLGTPAGMPQPEVPPLQHPP PTGSPPAAAPQPPYPVGTPGMPQPEIPPVHRPPPPGFRPEVAPVPPY PVGTPTGMPQPEIPAVHHPFPYVTTTTTAAPRVLVYKIPYGGAAPPRA PPVPPRMGPSDISTHVRGAIRRQPATATTTTTTRNVLLRTAILAAAA TLIALFRQRPLFTEGVRMFPDFQYRFTVQTTQN |
| TGME49_262050-t26_1-p1 | rhoptry kinase family protein ROP39 | 25 | MSKPFFPQVWVSCTGGVLNLRRHWLRVGNMSRAFAPTVLFVLLNV GGSLWVPRTLPVSAFQDAVAPHHPCIGRPCDQVASVVAVPPANNLG FKREPSVGSPGHFSFLQAVGGVGGPPYAGDGSPLLASRLDWQFLQQ WHTDEDENARDGFVAALSRVTRLSQILEDCNMEMVLLLDRVFSRSPR ITLSAVEPRTHRRRTLKLKIRSVAGTGGYNIVVKAFVIKSWFFRLGHWL GSLGRQRNTARAPGGETGQQTGTSGRRSSRSRGQSGREEGRREPQ MVSLRIRVGGAPDCQVANEMQAAEVVQKCVSEADRIMHVFPEDVT PDVLVSRFRFAVPLYSGRIRHRSALLGQSRGYALLNYMDVLPPMLCDL FAAQSVVQPSMTRAIAKQLLELVAHLQALGVVHSDLKPENVLVDEAG NLFLADFDKAHKVGSRVRCSSLPHSLFADPQTVICRETTPDQEVTLAFS QDAWALGMLMYDTFCRNWPFMGMDYNNDSDTSNTIVLAGLLDRA AAVDTRAPIDWIGCDNPRVESEIKDLISRLLDIDPENRLDVLSFYMHSD FFNEGRNEANRSPPQRGAPILPLRESIV |
| TGME49_262400-t26_1-p1 | lipase | 26 | MVRHWRSPTFSWSPSVHFLLTSLAVFFLFSFFSPRFLDSSAATVQDAE EVERRRAPDNSPGVEASDALREGDRKPLSCEEDGRQPCTVSDYRVGL TDLQPLLQRVQALPSGVNLADVSEAATWISRPNSGSGGNCEGEADC AGEKTRAPFSADTVHGLTLPGPSQTTSTLSEETVAPGKGTFQMGVDP FSTVHGRGLSSGYDGVYLSNSGEDMSIERSFPDVVDEPGTPTSLPLH WFHPLYHFYPPEAFREEDSRSTSSKRTSAPLVWGARFLSPSRAGPRKS KASSAKHAGAVPASETRRPSHDEWLEPLEEREGGAREKKLRLSQSAEK TSSSIRSQSRFESQGTSRSSAAAHSSLVPSSLVSRIPSASQALHMQAPV DSSSLSHPRPVEVQGDLPAAVSESHAGDKAESPTLCLGRKAVVDSRSS QSRSASAEPSPERPAKTLSEPGSPDLASQSVVASDSRDECTGGNSSCV TSTPAAAVPAVQLPVSSFYPVVFHSSQTLAAVTEVSDLRRLGEDPLFD AEAVRHQEVCGGLFQWKRPMKASSSSDLACVWDRFADAFERTIPPR TPLPFRPGGFTYRTVDTVSRWYSRINSSSVPEVKSEQHREKAEASPSSP NQDAETLEPLSSRGTIHRSDSSSESSLSHESDAGVVAGSPHTPTPEKAK AVRRTTRSESPTGILGRSHAFLSKASRLISKRLTSFTWGLVQRLVTVLKN QQREGALMNLGKAINSMLESEGVTDTRAIESALFLPLGCKSHLHYSEL YTEGYDVSPTDPTSWPDSPFFAWLIQDRARGEMLLPSNLPHHLTVTV SPRLPDDVEDKASRLRLYEMWHTIWFLIAEVSRSDYVWPWVKHKDV NQFFPWWTVTTVALHRVTEHRPVRYATGVWRFKNIDPETLEVIPQP |
| | | | DGSESNPFEVLPPPRSHLVEMQPGDNLATSGGDDQNPWGFLAEGLY ANMAAAEQSANSRDDEDNAVKPKTFTYRGKSLSIYPDLCGEDATFSY LYDAPFAMLFTPSNLLERIRTRLHGRESDHPRWRQPRRTASRTSRQRK SRETVQGNLVDAIWIFKPTTMPKMWLANRMAKPFEPELSLSNHPEA LVHRGYGLLFRTSYRPRMEQFISQLTSPQRPSSPLFPSSSLRGRDAMW LAYQHRSRAHPFTLVFAGHSQGGVAAEMSAFFVAKAMKDAIDNGT VRVYVIVFGTPSWCTQKMYEEFRETGLVIHEVASSVDPVPFLFTSTGF VADRKMVTLNLEDLYKVSFRHPFPFDGSLWKSPRKKTDKKTRLISATL GIRPLLDPTLSFTLTHSHVYAASLGLLSQRYPHIGWASFLGYPLVPPEV QTVPSHTPEGMRGLQEGHRLLLMGAGVEPPKSSSAAARGLSLLRSLR LRKRSREPDEGEEPPKKRRVVDKTLPEHDADRQ |
| TGME49_262730-t26_1-p1 | rhoptry protein ROP16 | 27 | MKVTTKGLAFALALLFCTRCATARYMSFEEAQKASEAAKRQIATLPSP DSTLSNPGSKHRNRGGSPAAGQPSQSTLQPEQAAAEVGLGAGGSTQ GQGRTGGSAGAREERRSPSPQSAYPATSSASLRGYQTQLSPSHLPPRS SGPGGWFPTESIFTPWSSPPQPLTQRKPSLSGVVVTEFQEPQEQYGA ASSLASSPKRYVSGASSSALSGKAVPTPASLGQENPLFPVQSATLDSGI QSPAQERRGSPQRQIAMSTENPADSGASQLASSVSSYVAVQTPHVK RSERIRRVRLSEEGLEEVQQLKAAAAQLLVAVPDYEAMRAVLQEAVLS EQRVATRKRKRKQPPGAVESAVDEVFPPNERVMMINANGVPIALYN RGHLGSGHFGAVIKASLDDGTLYAAKVPYSQIVPNADATSAELEAEISS ARAELVKTIRQELDVRDKLVAKGLTLTETAEQYGLPLCQMTLTLPENK ATVVRRGSRLVVVSKEVMLLPLIDGSPSNSLVQSQPPFLFQRAVAREA IIALAKLHELGFAHGDVKLNNMMIDVHGFGHMLDMGSVRPVDSCVS EEDKYYLRLWAPELAKSQHTSQQTCLKRGALDVWALGLAIFEFVCFN RLPYSLSNLPSSLWSRVEHLSRLRLSDFSAKDCNESDPAVMGIVAQFL NPNPEERPELPKFVSSYTFFRQAPGVTSHLTRIPTTELSSHRM |
| TGME49_263520-t26_1-p1 | microtubule associated protein SPM1 | 28 | MSGGNSNTPKKLPSEEGSDYGYPQKPQKYLPKSEQAEPDYSACCKGN DAYKGASHGTVQFSHPEEAQKYAGAAAGAETIQRGRERVAADRQPR AAGDVPARRLHLSDVDEAHRGQSPSRHPGYCVEELCTCGMHKCIPSR APVPFTGSTQYRQEFVPKPLPPPTQVSQVTLPPSLPFEAESSYRTEFVA KPLPPPAKFSEVKLPPTLPFHGESAYRTDYVPKPLPEVAKPVEVKLPPTL PFNAQSCYRSEYVAKPLPPPVQTVEVKLPPSLPFEGSTHYRDEFQVKPL PPATKVTEVKLPPSLPFDATSMYRSDYVAKSNPICPVSKLPQYPAATYP QNHVFWDPDTKQWY |
| TGME49_266970-t26_1-p1 | hypothetical | 29 | MKLDVRVINLEDDSKQTVTVESWETVKTVKNLIEQKTGILESEQHYYL GVQLIEDSMPADQLRKKAKAEDRVPPDDSDGVHVVEVKMYTGKAPI DFDDSFFKDIIKKLQTHEQIMKHISVRVHRKGDDAKYNFHLDISFLTVE SLKEDLINRLGPSSLTGLNADNILMYIRQEQPGRPLEGLQIRDRFGTRS SMEIEVFPINPEVTVVCKVKKWGLKTEEEFNIRIQDHFLLDDLKQILVT RLRSSANPKILQRDYIVGNLKFKLNGISINSKKEAALKRKYGIPAGELWL RAFNLKEGSLVVIPL |
| TGME49_268850-t26_1-p1 | enolase 2 | 30 | MFRLLVPTYYSYSVPLRFPFCKIYRYSTSNKMVAIKDITARQILDSRGNP TVEVDLLTDGGCFRAAVPSGASTGIYEALELRDKDQTKFMGKGVMKA VENIHKIIKPALIGKDPCDQKGIDKLMVEELDGTKNEWGWCKSKLGA NAILAVSMACCRAGAAAKGMPLYKYIATLAGNPTDKMVMPVPFFNV INGGSHAGNKVAMQEFMIAPVGASTIQEAIQIGAEVYQHLKVVIKKK YGLDATNVGDEGGFAPNISGATEALDLLMEAIKVSGHEGKVKIAADV AASEFFLQDDKVYDLDFKTPNNDKSQRKTGEELRNLYKDLCQKYPFVS IEDPFDQDDFHSYAQLTNEVGEKVQIVGDDLLVTNPTRIEKAVQEKAC NGLLLKVNQIGTVSESIEACQLAQKNKWGVMVSHRSGETEDSFIADL VVGLRTGQIKTGAPCRSERLCKYNQLMRIEESLGSDCQYAGAGFRHP |
| | | | N |
| TGME49_271050-t26_1-p1 | SAG-related sequence SRS34A | 31 | MSFSKTTSLASLALTGLFVVFKFALASTTETPAPIECTAGATKTVEAPSS GSVVFQCGDKLTISPSGEGDVFYGKECTDSRKLTTVLPGAVLKAKVEQ PPKGPATYTLSYDGTPEKPQVLCYKCVAEAGAPAGRNNDGGSSAPTP KDCKLIVRVPGADGRVTSGFDPVSLTGKVLAPGLAGLLITFV |
| TGME49_275810-t26_1-p1 | ribosomal protein RPS10 | 32 | MTVPGMKFSLIPKANRKAIYEYLFKEGVIVVQKNGKIERHPEVPVPNL HVMMTLRSLQTKKFVEEKFNWQHNYYFLTNEGIEFLRTYLHLPPTVFP STLTKKSTGRPTRGAGEGMGEGGDMNGDWRPRGGFGRGRGRMG DRPDRAERAPRYA |
| TGME49_275860-t26_1-p1 | hypothetical protein | 33 | MDVRYQTRGAVAAVYVLFRTVVGAVLGLHCLKIVSAEVGLTHGIMST EFEVELRGHNWVIDSGACLLGQPRNLVVDPVLDSEFMTPQKLLTDRT GPELCSWLEKIHRQHMEQKPAWLAAYQKFEKRFFLLRLLGMVGKPIT FFPEFTVRIIVKHFSLWGDDRWGNAQPWIAATFEYAHPEGAGGELFL HIEKVFSMEFSGRVAKLVHILLNPSATFNQYVELHRRKSSGVPGDSGV SGIRRTLYERDLNISHFPTADTSQISHSLAPDILTLRFAKQDFWMEDGS CYILSRFVATSSIRGKTLCKIVSDYGHRESWMPFVGSRKKTVALAVTAA DFFNTRRPSLVAHIKEGVKLEALDKTPVVLELVTDPVILRSYVEAARKA |
| TGME49_277080-t26_1-p1 | microneme protein MIC5 | 34 | MLRPTVRSSLLSLGLTVILYLALTGSADALASHLRSRHMEAGRRTMDT QNDVESAGRQSEPMEAADRQAEHPGAPTQSEMKEFQEEIKEGVEET KHEGDPEMTRLMVTEKQESKNFSKMAKSQSFSTRIEELGGSISFLTET GVTMIELPKTVSEHDMDQLLHDILAAGGVVGLDSEVKLA |
| TGME49_285870-t26_1-p1 | SAG-related sequence SRS20A | 35 | MVSCKRLAASRRQCLFFNACLFCLFICTVQIRAIAGADPEKCDQDGSLL NLRVTGNSSVEFKCGGAVPNLHPNPGKTPPIVCENSSCTNEVPLNIPN ATLVKDSSGKATFTVSDASVGATTVYLKCSSLANTAARVGAGQPGQE AEKEKTCIVQTSVWGPPIKGSKEYPTPDACKNGTLSLEVNSSKKSVTFA CGDNETLSPELFDMVFSTKECTTSSTLAEHLSGASLVQHARTNAADDK KPAYTFSVTSLPTEEKTLCYQCTKKNAQMKGEAQNTCKVLIKVAKQET DTGSTTVAPPNESRAEHGQWNTALMISLITIPVVLMKML |
| TGME49_286770-t26_1-p1 | hypothetical protein | 36 | MDTRGAGDLLIVTRWLGLIAGLLTLLQWCFILPSKAVSLSVDNGDFLK DINHDSWRFALFSFVPEVFIDIWTPFVMGMISVLCHFDFYPIDFNSKN FALFFVWNCLQALFGNLGYCGGIGIISGSFSLLVSLLSLICFVLDRNADA RLHIDKRS |
| TGME49_288720-t26_1-p1 | ribosomal protein RPL10 | 37 | MGRRPARCYRYCKNKPYPKSRFCRGVPDAKIRIYDAGRKRADVDEFP GVAHLVSDEYEQISSEALEAARVCANKYMIKNCGKDNFHLRIRVHPFH VLRINKMLSCAGADRLQTGMRGAFGKPMGLVARVDIGQILMSIRTR ESNVATAVTALRRAAFKFPGRQKVFVSNKWGFTKFTKVEYKKWQAE GRIVSDGVGAKWISTKGPLANTFPSAADITLPVA |
| TGME49_289630-t26_1-p1 | microneme protein MIC16 | 38 | MVVSCLCTAMSSALSQRNCILISCRLGRQRSRPPDAMKVYAPVFTCR RQFLYEKYTRRRCTTLGGLVALCVIFTAEAISRSQHPYGTSRSDLSPPAT VDSTSYGMSILEAGGPSDEVAIPSDRLQTFRNASLDFDRAIRFPNQAS VAESRQLRLMQMGECQNVYWGKKELISRITGKWKSALHPSCHESWF TYTSWSRTKTWRECAQALDNSYLYFSWVRRHNEADGACIWVKPSRR DVSKSDVEDCLVDVNPQKEVAVTGIVGSHHGCTTCEVGEWSETTCD SWCNDGVKGRFRELTGHTNSAHCSTSADANCDVCPNLWEVQRCST AVECAQGIKPRVRFCPNEGREYTAEGWRACAERCQRKFNATNGDTA NRDTFYKAECFRYSFNNDTSACILNPLHACPDAESASDVLWISGDVLS MPSADYTTVTWQEWQSWSSCSEVDPATGWKKRTRRPKTWGYRPD NVLSPLQWISVTPCASNVTLTRSELDLAMNPSAMCWVYSDFADWST VSCEPRCGADRKKTRARAVRQMPVVQPDGRISESCASTEVSLEARQQ TKDCPGFVSCGTGCVFGEWTAWSDCRCTAEGSTARTIRARELVSGSK ESCTGLEEERKCEKVCEESIFSNGVTYAVAGGIGLVVVIGLGFIGRKFYR |
| | | | ALPAERTRDNYL |
| TGME49_290200-t26_1-p1 | NAD/NADP octopine/no paline dehydrogen ase, alpha-helical domain-containing protein | 39 | MGAPSPVAAGVAARTKSPLLTVCVCGGGNSAHAVAAWLGQAHKN VRVNVLTRQPEKWQKEIRLETKICRWDHLGSITGRVNAVSSDPAEVV PEADLCLICAPANAHFPLLEKIRHHLKAGGIIGTAFGQGGFDWAMLKA FEAEDCRKNLGCYFALQNLPWLCRIIQYGSAVELIGPKDFLNATVVPG NMGQPVRLLISLLFDMPCRLLPNFMAITLSPSNQIIHPARYYSIFHKW DGKTPMKEDEIQWGLYNQFDEMSAEWLEKLSTELQAIKKALTARFPE LDLSSVLPIKERVIKHYGADVKDTSTLQTVFATNRGYAGCRTPATKVEG GYVPAVNGRLFNEDIPFGLCVLKDIAEQMDVPTPSIDFMIEWHQKLM GKEFLKDGKLNPEMIHETSAPRAYGLTTPEEIVAPSLMAQNATLPFAH IDMLGRLLN |
| TGME49_291890-t26_1-p1 | microneme protein MIC1 | 40 | MGQALFLTVLLPVLFGVGPEAYGEASHSHSPASGRYIQQMLDQRCQ EIAAELCQGGLRKMCVPSSRIVARNAVGITHQNTLEWRCFDTASLLES NQENNGVNCVDDCGHTIPCPGGVHRQNSNHATRHEILSKLVEEGVQ RFCSPYQASANKYCNDKFPGTIARRSKGFGNNVEVAWRCYEKASLLY SVYAECASNCGTTWYCPGGRRGTSTELDKRHYTEEEGIRQAIGSVDSP CSEVEVCLPKDENPPVCLDESGQISRTGGGPPSQPPEMQQPADRSDE RGGGKEQSPGGEAQPDHPTKGGNIDLPEKSTSPEKTPKTEIHGDSTKA TLEEGQQLTLTFISTKLDVAVGSCHSLVANFLDGFLKFQTGSNSAFDVV EVEEPAGPAVLTIGLGHKGRLAVVLDYTRLNAALGSAAYVVEDSGCSS SEEVSFQGVGSGATLVVTTLGESPTAVSA |
| TGME49_291960-t26_1-p1 | rhoptry kinase family protein ROP40 (incomplete catalytic triad) | 41 | MTPSTCCFGVLFQSFRGNSASASEESCLGGGHPDGTTATVFRPKTFQ ALGLATDSDSEPEGRGGYQRLTESTRRKKKATGRIASLFGRGRGKGDE EASGDEGASGGKEQPLGPPSGIPRRRRFWQKEHVEPEDAVVRAFRRF LKYRVLDVETIEGAPSQLDRSSWTPSPPFFQPGEGSSKAVEYFVKQQT PALTDEMHKLLEVVDPVMPPGRPLQMSSPSSKRTTHFERGPMLRTD FFALTVDHQPFNMRIFPLPGGFEGEQKVEQYKKVMLNEQKVLSIFGV ADGKKVSNAYHCYLPVEEVFFGKGQQVISLGPSLKMPSVVFLYPSASA TLGQVAEAIKLAAQQQGTALVAQAAQLHITLQLIKLVAITTSKGILLRRL SLENFLLRGDGVVFLSGFSELVNEKEIFYETEDGTLVTQPPTRTSRGRRF TPADNSCDLGLVIFSLWCNGSPPEREPSGWVNLDFSSCESEVPVLVQ QIICGLCRAHGYPPQNAFQTLGSHEFKQLRQLYRDVEQRAEVHLLVE |
| TGME49_292110-t26_1-p1 | formate/nit rite transporter protein | 42 | MCSIPPLRLLEVVDDLTCLVNSSQYSRWNFFTFTLPGFTLPMRKEFTSS CKGSFSNHPVPDHPCKLVVFFRPKMVVTAGADAYLKILEYGVKKTQLR IDRLLLQAFMAGIFVAMAGHCCTVLAGSYPTDPGDPLAVAKPTQKFIY GALFPVAFICIILTGAELFTGNTMTMLICYFQKRVTMLQLGVNWLGSL AGNWLGALFGAYFLSYLTGALGDEHVRQFLFRTCVNKISYGWGECFL RGVGCNTFVCLAVWAVIASENVAGKVLVMWFPIVAFCVGGYEHIIA NMYTLQAGLMAGAPVAILDVIAFNFLPTLLGNIVGGCLLVGAVYAYN FYPTLSYTETTGAKVYVQEVGPVLDRRSSMQVSMTEREPDGQVVTEY EAVPFESFGGEYIVNKHATMAAPIPSRASSFLYPFQWQRQRSQSGNL STHARLDLPNRPVEPPSDGLEVTPQSQTAESVAQQV |
| TGME49_293430-t26_1-p1 | hypothetical protein | 43 | MPATLEEQAVVAPPGDQTAMSSLPFEDTAAGRQGDSVVKGAKDDT SKQPTAVAYATEDPPLPSEVKILRQQPVEANETTVERGSQVAREKLEK RAAGANCMIFLVVVWLVCTGVLLIFAFSPVNADLGITSMLLGVFIGIIG ILSVSAKSKTGVQLYLVLGTVLLLLTVALWGYYIYILFRAARAAADIAAD RASHWVSDTPPELLPDRYGSGAAELTSIEAVARQGGLLPDVPLDETVE PCRKGFEEYGANYFKSATSVFLGLSTRGQMCNYSHYDILDFKKWPVS CWRTCGFDHLAVDRVLDNTRLDDQKVMADFLAHLESLSPQETSACV LYSVRTACVAENTAYHYILLAVIAFLIFWTCCACMCCIPASLCVAGKYLS ELKSYQKDKEAVERHTPPGADQIELAGA |
| TGME49_300260-t26_1-p1 | threonyl-tRNA synthetase family protein | 44 | MSFSLLVSCLRSPPFRPRFLWGSPRACRRVPEARSPSRLLFSAQRRLPF SPRGPRFSTMASGRRAEKGTVAALATEEKIGDPHFQVSENSPFLQKRL QVFEDLYEKQTRRLKEKPRREISIELPDGSKKSGTAFETSPYDVALQISK GLAEASLVAKVLYEQPSAESEAITAADDEEEEEAECPCGEHAEESPAEK WILYDMKRPLEGSCRLQLLKFDSDEAKHVFWHSSAHILGQAIEATFGA QLTVGPALTNGFYYDAYMGDAKVTEESYGRLEAAAAAVIKEDQAFRR LVCSKAEALELFADNPFKVQLIASKIPEHGLTTVYCCGSLVDLCRGPHIP STGKVKAFQVIKHSASYWLGRQHLDSLQRVYGVSFPDKKLLKDYLKLL EEAKKRDHRVLGQNLHLFFFDTNVSPGSCFWLPDGAKVYNKLCMFM REEYRFRGFQEVISPNIFSCDLWKVSGHYQNYKENMYLFDVEGKEWG LKPMNCPGHCIMFKHLAPSYRQLPLRLADFGVLHRNELSGSLTGLTRV RRFQQDDAHIFCRLDQVKEEVADALNFLFFVYDQFGFSFELFLSTRPK KALGERAVWDSAEAALKAALEETGRPWQLNPGDGAFYGPKIDIRLW DALKRPHQCGTIQLDFQLPIRFNLQYRTQDEAVAGARDKAKEDKETG EKRTDSEHEQKEEEAFGVELPLRPGMARPVIIHRAILGSIERMCAVVIE HTGGKLPFWLSPKQCIVLPISDKVNDYAASVRDVLHSCGYEVGLDVSN NTVNKKIREAQLQQWNYLLVVGEKERADKTVTVRDRADPEHQKVLS MEDLLTLFEKQGMPNSRKTLTLDAWKKSQQ |
| TGME49_305050-t26_1-p1 | calmodulin, putative | 45 | MSSVEQKAREAFKLFDRNGDGELTHQEAVLAVRSCGIPLRIQELDLPE QVTYPQFRQWMMNRVARSDPLEDLIKLFAPFDRKNDGTISTEELAQ VMKTLCSSMTEEDIDHLIKQADPNNSGNIKYAEFVHQCF |
| TGME49_308840-t26_1-p1 | SAG-related sequence SRS51 | 46 | MELNLATQNASLGVTQATRAKSVSLNVMQTTTGSVMVGQEGWLE DGAAMTDNKHDCQLSVVQFVPLGSISVKQWCYFKVGYVYACLEYNG GLAKRQQGAEPPSLSVTALFITMAVPREISWTQSQREMRLQIPYGRLL TRQSTLFFVALLAVVLHDSFVAFHGHLCHFAEAAAHQGSCTSQGTSSP MQVCTCNVPEQGETSNHAVVVSAEKNQLMAKCAIGTVTLPVDLME KQVVCPAGMTDPQSCVSPSGTIPFASLLSGDGASAVAWVGDEVNYA AVLQVPKQNLPPVDKEFIVGCTQEGKGHCLLHVTLSARASSKDSQTVT CAYGSASNADVHRVTVSPKDPTFTLDCSSDGIMQPASFTTSYCPATGP LQENCDEKYASILQGYSSSWWQVSQAGTSNKAVLTIPHDNFPEAQKK VLVGCKASTAIPNSTLCTVELTITSSSPSSAWFLSLSVTMTLAVAGAVSS LV |
| TGME49_309590-t26_1-p1 | rhoptry protein ROP1 | 47 | MACRQLLCSVQNLLFYFLPDIYCTDFDTMEQRLPIILLVLSVFFSSTPSA ALSSHNGVPAYPSYAQASLSSNGEPRHRGIRGSFLMPVKPHANADDF ASDDNYETLPSFVEAPVRDPRQVPGRGEAALGTEETPGQQPPVALGS AEGEGTSTNESASENSEDDTFHDALQELPEDGLEVPPPNAQELPPPNS QELPPPNSQELPPPNSQELPPPNAQELLPPTEQDLPPPTEQELPPPVG EGQGLQVPGEHGPQGPPDDDQQLLLEPTEEQQEGPQEPLPPPPPPT QGEQPEGQQPQGPVRQNFFRRALGAARSRFGGARRHVSGVFRRVR GGLNRIVGGVRSGFRRAREGVVGGVRRLTSAASQGLRRAGEGLRRG FTRVRGALRGRGRAADGASSVRERFGAASGRVRDAFSAGLTRLRRRG RTSGEEGRPLLGEGREQDDGSQ |
| TGME49_310780-t26_1-p1 | dense granule protein GRA4 | 48 | MQGTWFSLFVVVMVSHLAFGGECSFGSYLADMAGLTGREDKEHHA AKKRYYHSMYGNKTPYPYADGQQGSPPPQGQLLIIQNPDGSFMIVD QQGVPQAPQAAGGPGSPMNGGYYMPAGVYTAQVVPGTPGHPVQ AIPQQPLRTQATATYYHPAAAPPPGPSVFVFTPSSVQPGAEVTPGYSG LQLRQQSQYDYSYPGTTSTPTPPRPASYGYPAFPAFPRLPAFSDSVSVS TEDSGLTVVRDSSSSESTVTPADEAASESEEGDKTSRKSKVKKGVLTGL GVAATLAAAAAAAKAVKGFGGTRTSTAPAEAGKTELDDGYRPPPFNP RPSPYAELLKDLERMRKE |
| TGME49_318430-t26_1-p1 | malate dehydrogen ase MDH | 49 | MQDPSGHGNRPRAALVQLQESWVCCAAPNSVFSADIRTVAQMFSP |
| | | | PCASRRPRVGFSQATLCAGRLPVSRFCSLLSFSDLPPRQDTRLFPSSFL NGVASEKTLAFLPYPLPRESVSGGSVQAPGAPVSGVRHDKFAFRLSPG SRPGSVSRFRFHRPSSSATAARHLQGESRPFASCPVTPEATPFPQPVP SSFFKMSRRKIGLIGGGNIGATLALLSAVKELGDVVMFDVVQDLPQGK CLDLYQLTPISGVDVRFEGSNDYSVLKDADVIIVTAGVPRKPGMSRDD LLAINAKIMGQVGEAIKQYCPNAFVICITNPLDVMVYILREKCGLPPHK VCGMAGVLDSARLRTFLSERLNVSVDDIHALVMGGHGDTMVPLPRF TTVGGIPLPELVKMGMISQQEVDDIVQRTRNGGGEIVSLLKTGSAFFA PAAAGVLMAEAYLKDRKRVLPCAAYLNGEYGVKDMYVGVPCVIGAG GVEKIVELDLTPEEKKMFERSVESVKTLLAAAPKSA |

| | | | |
|---|---|---|---|
| ¹Accession number and protein description for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format); ² Example of predicted sequence for the corresponding protein on the ToxoDB database (ToxoDB-51 TgondiiME49 AnnotatedProteins.fasta, Last modified: 2021-03-06 09:21; Size: 9.2M | | | |

In a preferred embodiment, the protein composition of the present invention further comprises at least one *T. gondii* chronic infection specific protein, preferably more than one, such as 2, 3, 4, 5, 10, 15, 20, 30, 35, 37, or more, and more preferably all of the proteins of Table 4.

**Table 4: List of proteins associated with T. gondii chronic phase infection (native T. gondii chronic phase infection-specific proteins).**

| **Accession** | **Description (transcript product)** | **SEQ ID NO.²** | **Amino acid sequence** |
|---|---|---|---|
| TGME49_201840-t26_1-p1 | aspartyl protease ASP1 | 50 | MSPSSRFRNLVSVDSSSQDFGKRSSLYASLLDSASVSSL PGICSAAEDRDVEDESWKDPSSSSHCAKTEGGACAFPR LNQVLSSLRDPMGVFSRAKRRRSLGKAVGLSTSVICVV ALFGIVCLCLYGLVNFSFTSVETSPLDDPRNSPVMGELG NPQASTPSSARADTPARHDRQNQMFSPWSVQNEQF LGEDSDALPHAGPLFRSGVMVMPLRKMKSLRQIGWD KNTITVPDLQAHLVAALRMQEGLSKKDDGNLSGLSNG DDISIHDYMNSQYYTEIYVGSPGQKVRVVVDTGSSDL WVCSASCGILCMMHKTYNHGKSDTYHADGTPYHVQY ASGPVGGFLSADDVALASLKTKNFLLAEAVDLKGLGTA FFFGKFDGILGMGFPSLATKGLKPFMQAAVEQNVVKN WVFAFYLASANGVDGELAIGGVDQERFIGDINFSPVVD SRYWMINTKGLKSDGDLIAPTTKMIIDSGTSLIVGPLDE VKRIATMMGAFSVPLMPEGMFFISCEKAKVLRDLQLEI EGQDYPIKIKDLLISASAAAGTPCLFGMMGLKALEGGR PTPQKNGFGIFPPSQLVASAKGPIGRTWILGDLFMRNV YTVFDYDNKQIGFARLKN MNTGCLFLPATRHAAIQTICVVALARQVADPDALVFER DFLEFIRILSSTQTVFSSFGCPRSHVALLAQSVERVREAN |
| TGME49_203290-t26_1-p1 | hypothetical protein | 51 | MNTGCLFLPATRHAAIQTICVVALARQVADPDALVFER DFLEFIRILSSTQTVFSSFGCPRSHVALLAQSVERVREAN |
| | | | AVGLFSEFARRLGFPTSGDDAGCSCAFFVFTGRAMVW RVSIGCAVLATVLVAGHEPVASAPYKAYGIPAESTDTTS GDDVEEAFRKRSRGNVLKDAAKEEKKLPLFFIDVEDNK GQGRNSEKGATDDDVAVDKTKRSSPSAPCTRLAGKEK KYPCDRYVVKGKMSFLEAIGAFFLASVIHAAVSEQLRAR STGGDIWRPRLAGSVMQAALAATTLWMALRRRLPTF YLLQRRESSEEKKKAKENSTRAASAV |
| TGME49_207160-t26_1-p1 | SAG-related sequence SRS49D | 52 | MAAAHSAAACRYSTFWPCLLRERQSGTSSVSFVYPSQ FLSLSLLVILTGSFAQQSAGNQANSQSVTCESNASPLVL RITSKTNEVKFKCGTDLQLRENPAGSNKFWGNAACTK EVDASSVTFTSSPSPAKVAGNKGTEYSLALKNSSLPLSPF TVYFSCDPPSTTGVGETGKAKVPASATTCIVQVSVFSQT AVTVPETNKCKNGQVTVAVTSKSKSVTFGCSEGATLKP ALLDHVFIEKATEKSGGASTGREEEVVLQDLVPNSSLVE NAANTGNDTVGYTLSCPDLPSSPQNIFYKCVSPASARE QVGTQTECKVLINIEEKPEAETPATPEPSRGEQGVVLGS AFMIAFISCFALVAGNMF |
| TGME49_207710-t26_1-p1 | phosphatidylinositol synthase, putative | 53 | MAGTSASREDSQSPAVRQRVGSASGGKSVQAASDSD LSHEQKWARELKVFLYVPNIIGYVRIALLLAAAFAWREA YTSLFFVSYVTSQCLDAVDGAAARRLGQVSIVGACLDQ VVDRLSTCLLYVLNASVYPDFAFAFFIALFLDMGGHWV HFFAAAVVGAKSHKTMDPEVNAILSVYYRSRPVMFSAI VGFEGFFMCLLLLASPVFQASAASLSSALLVIVYVTSPL MIFKTLTNLLQGLYGARRLVACDIFGAPSS |
| TGME49_208730-t26_1-p1 | microneme protein, putative | 54 | MVPLSRMAMVSVAYFILGALVYGSANVFGEMQPRQL AKSSTDDPTIHDEGHGAAAPSTPRAGTELMTPHPLASR AKAVTSAIIQESKSTLFNTGHVTTTEHAPKNPGLLGKPV KQTEALQLASPEKGVTTMEPKAASQHADRSGNPAVKK STSGAQQAAAQSGKVLTKLTPIEKNPAPLHRQPMTRA TPVGPKPADPAGKAVKKAATGIPKPAAPGGKAVKVTP VARKPVAPKAAAPDGKAVKKATVVVPKPAAPSGKAVK KPVVSVPKPATLGGKAVKKPAAGVPKPAAPDGKAVRK PVVGVPKPAAPDGKAAKKPASGVPKPADPAGKAVKKA ATGIPKPAAPGGKAVKVTPVARKPVAPKAAAPDGKAV KKATVVVPKPAAPSGKAVKKPVVSVPKPATLDGKAVRK PVVGVPKPAAPDGKAVKKPVVGVPKPAAPDDTGINKA TLVTRKPEAPDVKVVKKATVVVPKPEAPDIKVMTDQTP TTPPSPPVRARAASATTIVTAPAPVPTRPVLTTTSTAAT VTTIKTETPRAASRLQQDLNAFCTTQFQKLCSETIDGGR YCGVSGAVARFGPGGHDSDSQWRCYEESIMNTTTKG VQCMDDCGNQMQCVGAVSVKTVATPPLRALSAFISQ RRWIYCSSFQRSADALCNFKRTGNVARYDPGRTRWM CFNPVDISLEGQSYCANNCGGFTPCAGGLSPGEIPTRT VYIIQQSDTAEAFAALHPCRHDEICIPSTVYPPACVKAH KIIAIQQLSKQQEAMDNYCQTEMDQMCRVGKWTTYC YQLWLSRMVSTEEDEEPKWRCYPFALLNFSDLSTCIDG CSNRVPCHGAPSPTSTLTTEFSKLKTIAENIAFCSPFQKA ANDHCNKRYGEGWVARQDVTTDDWACFQTTKLPTY RWTGCVDNCGNTSLCAAGVPEQLHSAAAEAFVHPDP ALERVVESVPSPCLTGESCTPTAMNPPYCSETRPNPQP APLRDHTIRNALDCSKGAPLARRNPTSK |
| TGME49_209985-t26_1-p1 | cAMP-dependent protein kinase | 55 | MRTSVALFFAAAGGISLVCAPQVSLAAPVQGTLGPDV ADTAGEPVVLQVARGADSLPDSRGNIDEEGITEVATTG VPPQTQSSGRSFGQRVMSWFTSARNAIRRPRTDEQG AVPGPSQPIPSGTDSTPEGWETVQSGDVPPGEPEPKA PTRSPRQRARDMFGRVGRFFGGARRFVGRGWRRFTT GMAGVTDRVRGRLALRSSFEFRNEEAVGQSELASLASS MTVTAPEGEALEVAREAGLRSGASINLVSATTGEQVTV SLRAPLGIGEYTAVFSGFISGIDSELAVKAFLGKTTESMS VAETEARILKIVSLKDPVDAMHRLRLLAPIETLVYRGDG DSADQSEGRPVVLVPKASSSLVDVINFLRSGDASAFDD RAKKIVLLGATVQLIQLLAALHTRKVVHGKLEPKSVLLFS HGLLYLSDLGRARRHGERFTTSRPSRYGAPEVLEHPETP YTYSRDGYSLGIILFELWCGRLPFDLGTPGVDSSEKLENS HPATLYQTVRNLDQRLSFDVCPSDMPESVKTLIRKFLTR RRWTRLVPQAALRDANFRRTMQLLQETVQGNGETQL |
| TGME49_210810-t26_1-p1 | hypothetical protein | 56 | MVKLRWKSASCTDRALQLMDVTLQRLEEEEENADKK GDNGTDRQRHIPTAINDLLYPSCIAVAVTPNVGEGACF RGMQCAQYSVLGKVYNIAVIMKPEEVLRSNGQE |
| TGME49_214410-t26_1-p1 | hypothetical protein | 57 | MRGSVLVVTRTVDCRLSRFQGKCYFRQWPVSGTTRSH RTRGWYLTLLLPLQSAVMAHVLISSPSTVELDEETQVFT SLQNAALIPRDRRKTRTHVRLRRSRSGGTGSWKPLATR VLIVTFLVALAAVALRRYVLRTRSRSMSTVSQDSDISASP RETVLDDRARFRADDGGNEGEFAFPSSPVENVAAAVA RLNHGSTTADEQPHRGGQADAALELLRPPTPSWGNQ EAPEGVTGRITEAVAMPLPAIDSLGERPHNGEQASAAL EPPGPATSPWGNEETPDGATGRITEAVAMPLPAIDSL GERPHNGEQASAALEPPGPATSPWGNEETPEGATGRI TEAVAMPLPANDSFGERPHNGEQASAALEPPGPATSP WGNEETPDGATGRITEAVAMPLPAIDSLGERPHNGEQ ASAALEPPGPATSPWGNEETPEGATGRITEAVAMPLP ANDSFGERPHNGEQASAALEPPGPATSPWGNEETPE GATGRITETVATSTVGSIAPAEQSHSGEQAGAVLEPPA APKPWGILDAALEKVREEISKQQVQLHFAMRRGDSAE VDARKEMLLDLLKGRALLQSIPDEPGDGEIP |
| TGME49_215910-t26_1-p1 | hypothetical protein | 58 | MAARLRSLSLGRFCLLLLLATWSAWSAAGSSSASFSPEE ESLVSHSRVPPEGKEASSAAAFEPTFRLVAFSPPSSSQQ ESVPVLRGRQPIAAVFSVPVVPLGSSLFSENASAPSEPR TPDAFRLSTNSAAKTVKGRGFWVTTSIFRFDPEEPWPA DLEVYVHLNAELRSASGQLVTLPEDCGRGVAPQGGLK NHGMIAEKLDGEDERRSLATSNSRSACIVHLRTDSLRVS VASVTSEFASAVTDNEWKPVLGNSADPLAALFEVPRD GKVRVKMNSPVALAVLKKTSLAERLLAVGREERNGEFS FVPFDIRTCAENEREVWAASDEVDFTTDSEEGWVSWR DDSSEDSRQEGRVGGNRRGEEVLCVELSFPERPLQTRE VYELFVKKGTSYSRYSGPLGVASAFLSSSSPAPHFAFFGA VRVSPAFIDQSVYAVVSHRHRRLKEKKGSFTGPRPFRL HGGRRALKHVSYRRLTLVLPHSLALPVARHSEANTDTLS PSPSSPSSSSSPPSSPSSSSSLSSSSTPSPPSTSSPPSTSSSS RSVARLAVEQTSATLQKAAEKLMARMQLVHVASGQA LSLRATMQSKVHLVLWCSQLQPGETYKLRIVGDDAVA GESDFSPLLDGFGLALESSEIEFSMAQLRPNLDFLRCQE |
| | | | TLWILHDRQLEHLGASHRADYGECDSGLPMAVMTVR HGKKEGEEGEGGHTATWGDVDAVALEAEPFAACIRSG DAKCVARLLPPWSHRHPPLKRQPSHELFYFDGATGVV SHESPSQLSTAVVAASSTSSSVVAPCLTDFSSNSLYLVST HTQHYRSASISTDKHLLGALSISVFHSLHIHHDTNRLVLN FQVLSLRTAQPISGATIRLFAASVAETREATLLECDSEEP RPREKARGAWRSSGRKNACLAVQTDSNGFATWVSREI LEEGIEVFATVVASIENSSPSGESATAVFVTDRLNWPW EFRHKPRLGWEAWQGGPNVLAGYDRRFFVPANEIRFF LLTDRSSFRPGEKVSLHGLLSTVNASLCGFHGACLLHHA LRLDAPEKLRVLVGAQWPPETGGHYESPGVHASSEVG SSHSCTSAVVSLNRFGAFSLSLKIPADAALGSRAFLEYRIF KAGDVDEAALSETACPETWYQLRNAGNLKHIDPAGGF SIVVEDPKPPSVVVSPLKLPAVVDPASALTVSGSVQTYA GLPVQDHRVEVRFAFQPSFGETTLRKAAARLQDSWNR EGKRAGQGASERTSDCGRDFSKFLIGSDQVGVNVVTT GEVSSRDTVHLHAEVRTDAGGQFELPLVLEKLTVAPSK CVGIETETALGVHPLEWEEGTEISVTVRVTGLTGDVLPP QTASVVVASSEFSLARKLSVSVRPVLPGVPFAVTAVAKP YPGVKATQLPTGSWLEVSVLRVDPQKPLGSSWEAQAD NWNFWNSDQGLASCQPSLFSEAGASESAERVPNTEY RAVTEEAMEAFTDGAVDLALFVEKHEDRFSIVKKCRGH GERLSCPVTLPLDAAQYIFLTTMRVNGRRVRTCEYFLPT AGNLLTRALKPDLQLYSDVVAPGQAVSLVLPALFRPGS SIRPRIYEVRRGGGEKTGPSRQLRGALSVWWHTQSNR RLFFSVSLDSTDDELRIPIGRVPEDCPASCSVRVVLVPPL SETPLPISQVDTSSNALMPKRDYFPIETEVGARYGPFLID ETLSVMVRPAASPFVIPENVIRVSFNESGASAREPRDAE LKPGKTASVRVALLTRTLEKKSALASFFSRLTSPQRQIRA YAFVGIVDKRYFDLGPVDLPQVEEEFRQRLVDQNVRQ SVSSSFAEAASLATYLYLFQFLKVLKERDPWVSDLHWP QAGLLGATALTSSWAPWSKTLRNVLLGRTSTLTGSFAA SPRGENLVDGVVFAQMSREATAMPMMGAGVMAQA KRSALTASRSGDAEVSVDSGETHGVAAAAKLLLSDTPLI LWKSVELSEGETPGELIGEVVVRVPDDSNRYLLRTQVV VEVEESEAPALSFFRRLWTRAEALKLSRVFYGQFEKEVV AKKRVKLTPFRPKLLRKGDLARVGAILQVDETLVSEGRE AVVSCWFPERNETQETGHRQRVKLTKTALPVTVQVDT DDPSLILNNRLQVNCFAQLASDASFSHGIAFAVPVVPM APRLSLSSVWAMVSPRAFKQLSPGDGGAGGQQLEPP REDSTSVTSVEEKIELPLPVLEGVGGLSASVGVGYGAVL LGKIHSFLSAEICPFLPAKQAHEDLLWDVDGRRRTDLGS KKILCTCCLEAFPAAATPFFERRSPSLSALLLVLLARQVVK TAKLQMHGELVASARFAETKLTAYLPADPEIFRRVGFLP RPSTEYTPSALHELRVDLDVNLLVLLVAKRQASSVVLPY VASVAETIRAFLRDAEARFLALDSGPSPGDLWPQPVPLI AASEPGVDMPDSQVVAQAASRVGRADAAKKKREDFT GFLAFVGPDLLARIRYVLGSATPLNLVHPEAEEALAFPSL LAWTLSAESRDRESAKEQRLSPHLLWALVVGLEHREEA NGRQLLALAHAVLKAAVGVLRFLPDSAAYFSRVAGGSV PASDAQHALLIQIASFLLSATSAERPAPSTVFSTLDPELF |
| | | | ASGSERMSPESIVHVFSKVLLYLARGGMSGQQPLGRM VPWTGGLFRNPWEDLLVMDAVAQWHTATKSDRADL SVSVELGSEKRSTWADGDKRLMQPPWLSVLRGQLSLR RQTAVLEKKLSWTDLDASEILKPVSFVSSNFMHANDAV SDDAEQCTWHGKVRVNVHGEGVALVSIAMDFVPDRS HMLPTFMGALLQKEFLPFNSATEQCENAPVASVVRGN RVCVSLRVTVKDELRDVTIRDILPAGLELSSRDPSMSPLA VVDDAVSSVPPSSHFPTVGKRGSASFFWFLPRCEPRLL GNAVEWKCPHLAAGTHTLRVIAIATVSGFFAVPMATV EVGTDEEGGGETLSGQKQADKVMLLGASGAAKRAFV VLEEGERQLAANEVAIFKEAGLQPPPAWLLGSAPKGCG VCPAGTVCSPALGKCVS |
| TGME49_224170-t26_1-p1 | SAG-related sequence SRS60A | 59 | MARVCRVAVGVRQQLAASVVAGLLVTLLTDVDASPV NALPPDPAESTVPTCDEGQEMTITLSPEKNAASFMCGT AIGNISPPLRPAKEGFEAFVDEAATTVVRFHSPDVQLIE ENNVYTVTAGIFPPVGGTWYLFCLEPGIGSAAVNTVKK KCRVEVVIKGTSIIPQQCTTVGGSVDLRIRGPGYAVTFG CGLSFPQLDPPLSGKEVYVGRECSEKQQLSALVPSAALE VGPAGQLYTLFVDKLPPVGRLLCYKCVNDLTAACKALIV VPPSAEEVATTVASTSTFGPSSDATIALHSSSSYLLIAVVF LLRLM |
| TGME49_225540-t26_1-p1 | hypothetical protein | 60 | MDTDLLVDLDRDNLLESICTEDILAGLNEDESKPDKGG NEEKLKDDSEKSAGGKVKVEDTEGDKSSRGSVDCAHL DKMTGLNKFRDLEKRREEERKQHQMELEAEKAKKPYS GKKWGITTPAGPVRPQEAAGSVVAEAVRSNKTKISARE EIEQQTRKALEKQGLLTHPVSPRPSCGEVPAVSVTSPIE QNSDDAGAASQVAVAGAASVEASGEGKHEDGAAHCS TTQSTGSQHSEHDEHEDAKDDKGAQEDGHNATDAHE VGQDDHGGQEVATGDTNANGEAGVTVVAATEVLDN AGSAEQSQVSVSGAGKKTELQKIKHKMLGLFACYNKK GVKEPIRPEESAVTVEAVTIEAVSAAGPGGNALPGSGT ASTDQKEQSSGENMKCDVEQGKPEETQNAAEAEKLD SGKESPVTAGKPSQPMVDEAAKPAAEDAAKQTVEEAS KQTVEEASKQTVEEASKQTVEEASKQTVEEASKQTVEE AAKQTASEVTKPTEEANTS |
| TGME49_225790-t26_1-p1 | PDI family protein | 61 | MLAADCFFGPDVVKRTQQGNYVPVRPDHFAGVSVAL FFAKAGHSKCAQIVPVVRQFYKTTNFSGEKAVIEIIYVSL DKDEQDFERVRALMPWCSVEYKSCLRKKLIERYRVPNG ELAFGTVRIPSTAIPLLIVIGPNGEEAGRMNFQQSDEFV LQRWDYRFNKWPGSAQRLRTLNDATDPWKKRLPQN V |
| TGME49_225940-t26_1-p1 | hypothetical protein | 62 | MSGNNPSPGYSDSGCYYLCVCCRPAPPGKRIDEEELVP DSTTTEEALKEVDEESFGEEAVEDGVEIEEGETAPSSAG AEVTREAAADGPTVRTRVVTKKRAKVHPKSAVKSQPKL FTGSAAGTPPPPPEACTGPLPEPAEPTAPLGMGVYLVY ATENGGTMYMKWSNTPLSGPGVLAYIEPKKEVGAFKF QKKDGVQHICGGLEKDMQSSFVADRKKYYDGWATFL KQMDAADGTLVLLPATTLQPPPKVKVAVYSQKKIRILEP DTPLEKAGVELMAVVPDTFVKFDVPTMEVSEFVAFAN NVGSLISFVPAKK |
| TGME49_226420-t26_1-p1 | peptidase family M3 protein | 63 | MISPVASVFSTMPHWKQGARLHAPGGRFTFAARIYLF |
| | | | LLLSCLGFSCSGEIANGKGTDTTPARLDPRTLPRYNLKR HFPYSSIFDPAIDRDIEALSRRAREFKERFKGTLETTLLEA LIKHEQLDEAVDFVDYYIALVAAVNTTNEEIRKRVNQLE SAIAADVQPYRLFVNLELGAMPEAALQKQMAANGNL SFYSGFLGAARRRAPYVLSEDVERALLVRKPMTVQVAT EYYFKQLGDATFDMNGEKLILSMILAFIMDNSQEKRW AAQKAVKQGLERHKITNFAALSLNVVAGSWHMERKE RGYERLRSSRNVANNVSDKTIDALIHAGETVAVSLTKRY FKLKKKILKNRAGVEVFDFADRLAPLPLKSSDRLYDWKE STRIVRDAYKSFSPTMALMFDKLLEEERIDAPAVPGKRT PACYLGTPTTGPFIILEHVGQPRCVETLAHEAGHAIHSM LSYEQGNLQLAPPLTIAEMASLMGERIVFNDLLRRTTD PEERLSHLLRYLDGWTSTVTRQLMFDDFERRVHDARA QGTIPDGAFTTMWKESLEKYFGAEGEVFDSYANSEAD WARIPHFHNTPFYVYAYAFSDLAVGSLYRVYKAHPEGF EQKYLDILRSGNKKSFEEIMKPFGLNPSAETFWADAVE ATGGTVIDEAESLALKLGFV |
| TGME49_227020-t26_1-p1 | histone deacetylase SIR2 | 64 | MNLLVISCCFLLTLACLFEPHEQFLVAVPTRASTTLRVQP IQKASCHLRKQRTATFSHFHSDVRIVWMHSVYGSEVA DSLMSGTEPAGSDYHQRDDMGQAATYFQKKNTTFISF EDLADDVRKAKYVVALTGAGVSAESGIPTFRDPSDGL WKKYDPTVYATIWGFWRYPHKIWELLLDFLRTNDPMP NAAHVALTDLQRLGYLKFIVTQNVDNLHQDSGSTNVIE YHGSLLSATCRQCGKKMRLSKSMLQDENFAKDLPPKC ACGGIFKPDVILFGEGIPANAVRDANREVDKCDLLLVV GTSASVSPASDLPYRAMRGGAKVVEVNLETTGLTNRIS DKFVQGRASQLAQTVKALQRR |
| TGME49_227380-t26_1-p1 | hypothetical protein | 65 | MMTPECGDSPAWSHSISGNASINGDPSCVDAATGED PTELRCYYIDVAFDCVSEVNTTSTETKMLSKLWGDESFS VSEADGRADLPAPKQKPSSEIVSLKRQERVSRVARRLYV HLPRLVRSPMTRLQKKTEKRVVASLPSLARSESVVTADS TGEASCRRLSSVGGALSASGSSLKRSCSRGRSQAGGKV CSCESIFGVFTLRVFLAACILMLALLIQLVLLVLARNLPFA HHSKGSEVAAVVSEQVTAKVGEFAQLSRAAQPHELSG EDTAVSSSFITLPESGFRDVERDGLGTTSRFHRAEPAPR SVFACNVAFLIFLFCSVLRMYFLTPNLASAVTIRFFLCVS VAFLFRGLSCLLSALSNHSAVENRETQKDFFPHIVLEAT GLWIFGGEAGSPVTSQKLVLWILVSFWGYYTSKHVFLA YIVIPLVAVIVCVIPTTAACDLPGHAAATLFISSVCIIYHLL LDIAAQRYLRTQSFRPSGGSIAAFCGRYVVGDVLIRVLA FLEALHLRLEGVMCVSSPTVVAHVKLPDRAEEESVSTP HSTPHSLTYEDLASRVVLAYAGSGDFDATNCRRAFLCLL KKVKAIGEQERCRESGGEIPTAAECEMEVFAASHAYRS NVNASLELGESGLYLSTSVRLGCAFRLARVFLRTGICFTP MAASPREETFYDDGLSGMPLLPRRTFLVENRRREDVC VPVCRFVTRMQVSRISKL |
| TGME49_234380-t26_1-p1 | hypothetical protein | 66 | MLFPPGIFSEATAFSHKRSIRSISSHSTFDDVTAETDEAP VLAEVEKMPAVVKKVKHGMRKVLESLGILDSKCEQKKK HNKIPSAAAGDSPIKTFGYPFYDTSLGRSLLRPQVGLSFI EGFMQPIKVGAFDWMVQGTVDDVLSFLVCFDDYEAI AEKLGEDGAWHSEGNIGPLYLQLQNTSQYQGDASSIL |
| | | | MAYSVEQILGRIDTWGVIDQTIGHWWKPAASLSEKAK SVYDTYQSLKERVPDLTTEKFQEVWNSASTKSGVGKLK ELAKLFTLNPETRDLRDSWENLDRVDRGRKISLYVTSYP KRSRTMATFAFGENRVKLGDMAAAVAAATKDSTLDN LLKKYV |
| TGME49_234530-t26_1-p1 | hypothetical protein | 67 | MEKMSVSLKALCLAPFNASDFKAAVFGGLFGGLASFLV THLLLELTFSPFFSVFFAILLASIAGLVIQRALAIDPPVPPP ALGFADEDDSLDLDPDTSLHSLLAARLGRGPAPDAAPL SDNAWTGRTGSSGPGGRGNAADAKQMSTEEFLNDD EEEGAMLFGHNALVA |
| TGME49_236070-t26_1-p1 | pyrroline-5-carboxylate reductase | 68 | MIPPLLLNSLLSRKAIILQHSLPCKKKRRLAAVDSDPVLL ALCSCARILVHSSPVCAAETRMTATLDKKIAFIGAGVM AFALAKGFVDSERVKKEQVYMLVRNETKRSLVEDLGYK SFQNIAGLADVDIVVISVKPQDAVQVLCDIRMVVDKEK HLLISICAGLSLDTLARALDPSALNRRICRVMPNTPSQV ARGVSVFALGEGCTDEDRVTVESLMTAVGSCYRVQEK HIAAAGAISGSGPAFIFTLLEALSDAGVKNGLPRKLAQD LALDMVEGSAALARVEFGRRHFAELKDQVTSPGGTTIA GIAALERWGFRNAIIQAIDATTDRAKDLDC |
| TGME49_236860-t26_1-p 1 | haloacid dehalogenase family hydrolase domain-containing protein | 69 | MNRAVCTAAGVTRLPSVTAFPDFRLRLPLRVRSVRFIVF AVVTVFTFSVDPERSFWRVQNDRLVTHFLGHGHSSEK TLFALSPVLAHAITRETPRKTLEERHHVRAFSSQHAGRA RHDEAELQPVSSKDSTARRTAAAHEESKAHNREGKGA SEGFQGRSKVENDGQESPDSKNSGAPSAPSSQAKPER NYQTGFLGFFSGQCPATPLTTPIPPHFVRFVIICYSVIAGI AILWLLVQASRRKHHQRKQKQALQELVRHGELWHRS GKVVQRLSSGMRVATEGDPDEDNSLHSGPLTFVRGM LIQQEGCYRSLPGSMIKTVLKASFALACVLPILAISVSVVT HSNRSLTSFLMPPVFYDAQEPPAPPSNFRLGSESVKSSP SDSSRAIPKTRPPASLEGSSNDFITQYWISDVECMQTRE EVCVSLMLFSFLYLALGQALWVRFLRDLDLTPAPLASCS QLRLRVTYIPLHRVEQLRRSWQREHRCHAAKTCSDEL MRRRFTAVAASVTNVLSAEGAENSSQALERLGAGEAC TSAVGHPGKATREGSEEGLEGKKDWTDGGMFVEEED LTRWSMGEINLQSLQTQTFVADVWSTVSVQHRQRFV EVEGLSFVYESRTGVFVLATTFQEKQGVGEAVDLAEAL TAEDMDGSEDKATRLLNTFQRVLLKPEEVADRQYIGKN RLVVANTTFRELVWSSVTGNAFYFAVLVAMWYYSFTR NFLVLGTLLVFVVFEGLYRIRLLVRQQKRLQSLAAEREK AVSAKPALVLRLLRTASGLKTGAEPRSATPPQSGQGSH SFVPTWIRIPAADIVPSDIVRLSRGDVVPCDMLLVNGT VAVDESSLRGDGTPQRKGPLRVSVRKLRGKLHGGDKG TPLPEDGRLADSVLHAGTSVISCTEARGRSRTLDEEAIQ YVFTVEDETANRSAGKAFRETLGVEQRAKLEKNLATVE RTQVAAAEALMDAGASGQGGICWGVALRTGIDTAAG QQLRQLQLPEQEMFRYDRKLIYVCSIVLILWLGLVAVH AHYTESLLLSFRFAVDSLLKLLPLWLPALCGLFATLAARR LRVCSAKEIDGFRRRVEQATGRNCLRVFLSAASQARML ASLPSEQPVDTREPQGMSPVSPKSRSGLSSPLDPADRS STVVDSRPFTVVCPAPGRLPIAAKVRIVCFDKTGTLTRE EFSFVGCQPVEAASMLSFVSYEPDGPAMQMRPRGAG |
| | | | APLPNVKNSPQTFQAAGGDRQVPPRLFEGLVCCHGLT LDNAQMVGTLLEQQMFESTGWKMKWTVFPQQRAD GPTEIAGLTRLAFSPANAKGYYSDEDGDRTGGFVLLQR LVFDPVRQVMSVVVAKVKDERRGDQNEGEVFVFCKG SYEAIASLCRPETVPGQFLHRAGTYAKEGVYVLAAASKS LGVRTDLNAWKDLPRSEVESDLDLEGLLLFRNELRPDA ADTIHQLKVAKIRPVILTGDSPLTAVAVARRAGMVGGS GGVFGSDTGRLQRLSRGDTVDGLHPMVLGDLVDDDL SGEDVEAFQSGQDRVGDSRAGKRCTGGSQGWHPAK ERTSAVCQGEKSKAAAGLCERVKWTNVDTGEEIDRWT VFFTKEYKELALTARAVEALTETPDILMAGDLSGEDFAV GDEGDSDQLEEESEGLLWSQGHSGVPPGSESTPSNTG GSSGSQSPSSASLGQTECWSPNSKRYGGPVEDPSSLFR VRQRRPTMFDRILFRVRVFARMSPHGKALIIERYQERG LIVSMVGDGTNDCFAIRQAQVGIAFGPGSLCQSIAPFAI LTNQREEPTHSASSSDSSASAGQSKASLVVHHCPRANR FDSCGLLGVITLMREGRATLVTSFAVYKLQILYGLLSAVS YLMLTVGAYGTPNAFSLFFSSVAMLLGLSLSMLWSVPS VTPLTKRSPTSNPLGVRTVAEVMSILIVDLLALFLLFGLLY GPAGRPLPCAWVQREVRHELLQRQQIENLLDLHSFNE PHIAQMEDAVTHAKKKLVEYNPDKEADGGHTKQRTA VHDKAIAEVQRWRVEVTPSLSFPTHEANRVTDLVGNV SDKTISSQQQTLNGNHSTAMLKNLEETTKGHNGATAF LEVNQQVATRFRTEVNAVPFREKSIRKQSVFSSGLVED VELRNMIPAAKRNRAVSHNGNAHEDGEVRHFSIPESS AQSWIEKTRGTAPPPHPDVDTLMGHNLRPGAAEHNL PCSRDGRTEASVIFLWLGACLLNAALIFSSGRTSGKAFW SNPVLMPLSFIFAVFFVFLVTAPKNRVSCVFMVNCPAN EIRMQQQRRNAIGVAKQLVTDEEAKDMYRLLARNAE MDVVKVRAILSVDMAAPNERLPTLPSTSLPAHSNLGG ASETSKSGQLETAGDLAKSFRARMARRAQIGIHGSRKP MIPGQFNGATEERNEGVNNVQVDEVQPKQGDRGRV KGNNGSVISAWYGQVLLYVLCSMSVLNGMIHMYVIG DQMGRDTIANWWRKAHPSRYKGEVIAV |
| TGME49_248990-t26_1-p1 | hypothetical protein | 70 | MRWSGGPATASRPQTTLSTQSVPEMVTGFLVFWLNG PTSKEEHLFVYCGADVGKFSLLPRPLVSECFHVHWPGA RASPRDVRHPGAATSAFLKALEQQILSDFRRRGLWNP NKEDGTKPFEFEIDLYERGSHYGGRLDYAVVDGNVVET GGADIEESNLWLSTFHKMLNNHISENGNKAAADEDV GSTGDAAGPMPLAEFESSVTRTFLITRDGGVRDVSIPAF FLALRGNGQGILELMQDTQKGWKSLYSKLAGIPLCVYV TRDLFLQPDFEHGSGEKYEELCAAPKGFTSGLEMVDSV GMKPLTEKTFATQAAETGLTPDGMDFMSGVTRSIYSQ DIEEINAAAGVVAMLGSFGSFFQGKRYSGRVLVEYLIAR FTSNAFLNCGIRDVLEEPQSGARQVTKSRKRYRLRPRLE GDSCTAEAQRRLESILYDFVVVAAPLELSGVRLYRHRED SWKEGERTRSEASALERVTVPPRSWRTVHLVYFVADAI RWTNSRSVSSEEGSRSGDMSHLILKARPSGASAGAFPE FYVAGCYGHAVEDAGQDVFRRKVSSDKTAMGLDLGE NPRDYTATPESDDVAPGKKTLCRVASPQWLTDETLEST FVNPQHIYRRRWLAAYPNPAPFDPVSTSATAEQFSSSA |
| | | | SSAQAFNLNASPSEHASRKRTTTQHAVPEQSNFILADN VFYPSAFESIFSCMEGQAVSAQNAVNLISNSLDIYSGEV |
| TGME49_251540-t26_1-p1 | dense granule protein GRA9 | 71 | MRSLKSIVVPLSAALVAAAELDLFLGESGVYLFGKASES DVALKVPEDPVPEEPRREPEKHVDLFGEDWKQFGGSG FGDFSKVEFENLFSQVHEMMRRLMGRGVDGFGPSLL GDSPGFHFPRLRALQPKTKLEKTGTCQYVVTWAPEVT AENVRVILHLQRRQVEVQYRAATRRDEKTEGGESHSM SKEQSSQLMSVDPQCIMTREVVAQKLAGWTDNTHTA TAGTPKKLLISFPSPDHIKEMVKEGYLPDNALERVLAGD FEGFSRTQMCLVSGRNRTECAFAEGQEVELEEKPLPSD SSPVTSVELPRLSQEDRGL |
| TGME49_253330-t26_1-p1 | Rhoptry kinase family protein, truncated (incomplete catalytic triad) | 72 | MANTSVRRRQLLSSVLLLQWLTTVLGVAWGPNPIDGS KHGQFPSLRRTEGVSQSGSGHRGSVYRIPLADMTFWS WMSYLKELPDIDESRNPILKRLLSGSFLRRDGSTTVNVR VPARELVRLLSLTPEQQREGVSAKVRLINLLDPKYSVYEP YLYREILPKRSPLLLPSLGEYRGAFLTYIFHPLSKGLVGDM LETGRSHPDVQVLAANMVAALKSLHNLGLLHRSIELNS FSVLPDGTVVLGGLDTAAPIGTETRLWVGFFGQETAPE IDRNFLADLALTQGRHTVKSDVYSLGVAFRNLVQLLGN GNLGPEDRGAVRQDHLELLDKLSQKMIEEEPGNRPTIE EIMKDPLFEGLNFEDIEEGKARPFRYKQNRL |
| TGME49_255900-t26_1-p1 | Bax inhibitor-1, putative | 73 | MNVFEQYNQRQGAMNLKHIFNFAPLSPIQQEHLTRVY GALMTNIILTAVGVYVQQRILALPIFLLLGVQVFCVWGL GASSTEAVYSGKVTTPLRAAYFGGFGFTTGMMLGDYL YFVNVLNPRIIPTAFLVSMGIFASLSAAAIVAKDRKFIFLG SILGTGLTLFTYVSLFSVVWRTKLADDVLLWGGLLLYIGF VLFDTQVTLEMARRGSSDYLVQAIQFYVDLVGIFIRVV QILADKERRKRQRDEE |
| TGME49_259020-t26_1-p1 | bradyzoite antigen BAG1 | 74 | MAPSASHPPGACPPGCTKHPATATAISPSGVCPMRAF HPAGPHSHFSCYDDLRNRLSHDKNVRPVASQQLDYLD EVSPFALAYYPPPFWGGVGLNPIDDMLFETALTANEM MEDITWRPRVDVEFDSKKKEMIILADLPGLQKDDVTIE VDNGAIVIKGEKTSKEAEKVDDGKTKNILTERVSGYFAR RFQLPSNYKPDGISAAMDNGVLRVTIKVEDSGGAKQQI SVK |
| TGME49_260190-t26_1-p1 | microneme protein MIC13 | 75 | MVGVFQLCLLTCLLCLLKGSDAIQLRQHRNGLGVEQSF VSEETDIVGGRISSLQAELDSYCHNRYKQLCDGGNKKF CDKTGVARYGTGIQSQQTAEWRCYFAEVLKPSGRKVQ CVDDCGNYFPCLGVVDPNDTFHATAHNQILNFIKDGV EKHCSPFQMAGNNYCQGVLADTVARKDTGTASQKAK AWRCYKKDSLTYEARSICVDNCGGEIECPGGRSETSGEL LSQHYTREKELQKVIESQSAPCHDACVATPINPPICADT ESLLQSTKRSRAQRFIDDACQKLFNCHCKKDKKFCTTVV ARKDRGRAGSQNDAEWRCYSLEELDFQKTNPSCIDEC GNELPCQGAVPEESTHHITWTKLPSKIDEATQKFCTER QRAANDYCASEFADSLARYGPGAGDQTSQFRCIKKAA MRADAEGQCADSCKGTETCGGGRSRLDGPLSVDDVL NAHEGISEAMKSASTEC |
| TGME49_261710-t26_1-p1 | ankyrin repeat-containing protein | 76 | MATTHLNKSVIAGGSIKTIKALKKYWNSPVSDIDDNSSE AKYTMLQMAILLNKKNVVEYLISQKDVDLTTRATDGTT ALMTAVGKNVPVDWIRAMLERGAADNINVEDNEGH |
| | | | TALDRCEKDSEVYKLLVQHGAQPRPSPPPTPPPEPDVA GGAGSTDTNAAGQNGASKPVDGITGVGGMSPGLDE HPGIWATMCGCGGRQGKNGTEVVEVVDAGKAELNP AQAS |
| TGME49_263270-t26_1-p1 | glycerophosphodies ter phosphodiesterase family protein | 77 | MEDSAVRYSSNVPLQETPAWGGGSEERHRQGSPHSP CAPWCRVRCRLSVFAWHFLSTPSFSFLRKVFCCFRNPP ESSESSAPRNAATSGTEEHGTLVADSIPPRPDTAPAAP WDVCTPRGVAWKQSGRQSCLFAALSASFLGSFLACVV LYLLCVWRNDWQSIDTTLFAWSGKYWSWAWILCSLF FFVWSYNVVLSVSILLVLRPPPCWTRAAESSTSFPAAM SSSASRLSSACLPVLSPLHYILLPGSLALCAAFFVITFLLW GPEYRGVPLWFEFYFPSLYAVSLPVVTLCFGGVIVFFSPT RSSSPSRGVVFPESSQQRPGCHPHSSAPLLDTAGPAAF PGPPGSAYQPYSHSDLCLFDGQECVFTKTGTARRASAG EADLTAPLTGSGSRDVCSPDSPSAAARGRASGTHSGCL GVSATEEKSEDRAGDGGDREACSEEDSETEGPVTAVH QWRHVVFCLPQWVALLVLAGVWTFFLAAPFVAPWLF SSPCLLRDPPASSPENERRRVKFSGEEETWKTKSFFAFG SDSSSSLNTHRSKRANLHPWLPEKPLLIGHRGLPELRPE NTEVSFEAASAMGCSGLESDVVISADGVPFLLHDLTFA RTTNVADVFPSRAKTRADLFTWADVAELNAGRWWLE KDPYGTVKLVDPVVQKEISEQKIPTLEWLMRKAVSANR TLIYDLRCPDCDSNTWGCGTKCVDLTIDLVRKTKCSRHL WWLQDKRQEVKKEFPDVTIVTAADRHFRHDADNLTD VLNVEWVQLDAQLIKMWNERGYWVNAYVVSQPWLL SYFWCAGVDSVTTNSCSRLNAMDAPVQHISWNAFLW YVAILDLICILLFIGLLYFCRRPPSDCPCSVTVVNMSSF |
| TGME49_268790-t26_1-p1 | hypothetical protein | 78 | MMIRISGHVCGGGRPGIPRWKSVLRKYIASFCFFVACK TWPGFVSCSIWRPQGTPEVGSLGHDAADTAAAEAAR DVLSAWLQEVKSLIETAAYGVRGVANDVVDGHIVEEAL GMTYDIHDQLQKRIAEEAKASEVRIAQAREKASQILERL QKLDLSSISSSIQQVPARELRELLSLLAVPRMPSLSLPQIS ISFNKDPNSETDENNQDKGAEEAQRPKQTASDATAED VEAEIDKSQVPPSDDIVQSTSDV |
| TGME49_268860-t26_1-p1 | enolase 1 | 79 | MVVIKDIVAREILDSRGNPTIEVDVSTEGGVFRAAVPSG ASTGIYEALELRDKDPKRYLGKGVLNAVEIVRQEIKPALL GKDPCDQKGIDMLMVEQLDGTKNEWGYSKSKLGAN AILGVSIACCRAGAASKGLPLYKYIATLAGKTIDKMVMP VPFFNVINGGEHAGNGLALQEFLIAPVGAPNIREAIRYG SETYHHLKNVIKNKYGLDATNVGDEGGFAPNVATAEE ALNLLVEAIKAAGYEGKIKIAFDAAASEFYKQDEKKYDL DYKCKTKNASKHLTGEKLKEVYEGWLKKYPIISVEDPFD QDDFASFSAFTKDVGEKTQVIGDDILVTNILRIEKALKDK ACNCLLLKVNQIGSVTEAIEACLLAQKSGWGVQVSHRS GETEDSFIADLVVGLRCGQIKSGSPCRSERLCKYNQLM RIEESLGADCVYAGESFRHPK |
| TGME49_270240-t26_1-p1 | MAG1 | 80 | MDCGQCRRQLHAAGVLGLFVTLATATVGLSQRVPELP EVESFDEVGTGARRSGSIATLLPQDAVLYENSEDVAVPS DSASTPSYFHVESPSASVEAATGAVGEVVPDCEEQQE QGDTTLSDHDFHSGGTEQEGLPETEVAHQHETEEQYG TEGMPPPVLPPAPVVHPRFIAVPGPSVPVPFFSLPDIHP |
| | | | DQVVYILRVQGSGDFDISFEVGRAVKQLEAIKKAYREAT GKLEADELESERGPAVSPRRRLVDLIKDNQRRLRAALQ KIKIQKKLEEIDDLLQLTRALKAMDARLRACQDMAPIEE ALCHKTKAFGEMVSQKAKEIREKAASLSSLLGVDAVEK QLRRVEPEHEDNTRVEARVEELQKALEKAASEAKQLVG TAAGEIEEGVKADTQAVQDSSKDVLTKSQLALVEAFKAI QRALLEAKTKELVEPTSKEAEEARQILAEQAA |
| TGME49_273320-t26_1-p1 | hypothetical protein | 81 | MKVLFFLGLLAGGLLCSADTPSLDSDPSASPLRAVLHLR GPSPVPAQVEPHEADGSYLEATTGNEHENTLSDESEQE EADIEEERAAVSALQMTQKEAPNTGATGVAGPEQCSC EFAGSCLPKGSCLGIAFTLVAATLFFVIFLAWLLQWIIAIP SEWILAKRLSKRDTSTKNSPREEDDGRQALLQENDSLD GRQDEAKATTEGTAISREEEEERERTIRVGPSGLPPPSP KESGRL |
| TGME49_278080-t26_1-p1 | *Toxoplasma gondii* family A protein | 82 | MKAVALRGFWLTILLGVFIPRTASKSSGLTSAAPDFTVTI PKEGLPADRQEVFSLRPSGILRVIDETGEAVYMPQATA DSDSSSVDPSKETLKASDAVSTQTYGDAYAFVNGKCDF RETIKYKDTFAGYTAPLWVRVTSDASMTPETSSSAVVN YTFTNPPKEYLSEVVSFCVIFKTRLASGSTSSSPAVTKPTE PSGAAPQSTSATLSVPAAEQNTSQHYGDSGTGHESVP EGKPDLGKDDKVNKEIHGPKGGPGPTGPVVPLQKEPG QSPDTPGPLTPHDGTASPKRKTEEDNGKSGVAPKQPV PVKPAATKPSTTRHPYASESPSDRAAHPADDDRGYSIS GSKYHKQGGDEKSVRDSEVAATGPGEHLHVEQDRRK GDDVAHNAEEEAASEAASKGGSMVNDEVQGGKLGN DDHPESAETSLQGGHRNILQEEHVDSKDPAEIEKAHTR RLSDTVTPESAHLTIVIHSAAWGLAGGRAAIPAFVLSCA AALFSTL |
| TGME49_280570-t26_1-p1 | SAG-related sequence SRS35A | 83 | MTKNKILLRAVLCFLILFGCSAWRVRAKSWTYDFKKAL DDDETKKEIITPGDSVSIENSGSRPLEYIPPNPSQVLKVE KGDHCKDEPVELAALFKGSAPTPAWTEAQSTRSLTVPA GSVPEKETIPFCFKVKDTGKNKTLTAVIKVAGAHGLSAA VGVSIGIPALAFVLISM |
| TGME49_290300-t26_1-p1 | hypothetical protein | 84 | MSCSCPSLCEIGDFRFPAGASSLAGRLPCLTSPRQRSFP EHKMVSHTAGCSLPWRRRLGVFSLVATVQGALYPSAV LSLRMSVGTDTRDSSLSPPVAVPPSAAAASSASSWFNS SPSELSAPSSSAPEPPFFSPSALGFGQLAPPLVMKVPQ MPASSLPPVPDNSETHEPCLLPSPISTQSASLSLLEEAAD HTVPPQESTASTESAASGEAVSSPPSDLSCTVAFEGPIS RAPTLTLSSSSDAGPFLAIFRDTHPSANGWIHGFHKELR TGYKGPFGGENGEYVRCHPPAGAHDYEVLVFDQDYEL GPKLKAMAHTTWNTRARALGTEEDLIKDIAHNAPSGA TPKIVTRCSVTVSHEQIAEGQ |
| TGME49_290970-t26_1-p1 | 8-amino-7-oxononanoate synthase | 85 | MFGSVFVLDSDPMGFIGNRNVEWTTNLDFFYCAFFSA SLLGVLLAFFTDDVSSGSLRWSWIVMELLPVPRLSNHV AVKDVEGALITAAKQASGKSQVFAKIVTAAHEGTLKVLL AQWCTKLHLRCWFCWHTLKLRYTAESRRQLLYQVNK VLLRLENRKGEKEVQSYLDIKRYMQTNNLWYFAFRISD VKSQYITCEGKRAYHMSSYSYLDFMREPLVQEAALAAG RMWSTGNHGARMLGGNPTVIRELEQIIGRFFGREDAL LCATGFLAAMSSICAVAKKGDLIIGDNRLHTSLRVGMK |
| | | | LSGAKEVLFRHNNWQHLTQILGSMRRKYIDCWIVIESV YSMDGDIADLPTVRRLADQYKCQIIVDEAHGLGVLGKS GRGLEEHFNMPGAADIIVGTFSKSIGGVGGFITCGKDLI EFLEYHALGSVFSAPLTAYSAGGAKKAFELMQGEHRW RIAKAQENAIYLRRALKTGNGNWPPDYPADKKYEVEGI ECTTVIPVVFPNDPYRLCCVTRALFSKGWVVGAAMYP ACPLMRPRIRITATAAYTKEIMDKFVRDLVKTTVDVPLT TEVEDGPITL |
| TGME49_291040-t26_1-p1 | lactate dehydrogenase LDH2 | 86 | MTGTVSRRKKIAMIGSGMIGGTMGYLCVLRELADVVL FDVVTGMPEGKALDDSQATSIADTNVSVTSANQYEKI AGSDVVIITAGLTKVPGKSDKEWSRNDLLPFNAKIIREV AQGVKKYCPLAFVIVVTNPLDCMVKCFHEASGLPKNM VCGMANVLDSARFRRFIADQLEISPRDIQATVIGTHGD HMLPLARYVTVNGFPLREFIKKGKMTEAKLAEIVERTKK AGGEIVRLLGQGSAYYAPALSAITMAQAFLKDEKRVLP CSVYCQGEYGLHDMFIGLPAVIGGGGIEQVIELELTHEE QECFRKSVDDVVELNKSLAALG |
| TGME49_293690-t26_1-p1 | profilin PRF | 87 | MSDWDPVVKEWLVDTGYCCAGGIANVSAEDGVVFA AAADDDDGWSKLYKDDHEEDTIGEDGNACGKVSINE ASTIKAAVDDGSAPNGVWIGGQKYKVVRPEKGFEYND CTFDITMCARSKGGAHLIKTPNGSIVIALYDEEKEQDKG NSRTSALAFAEYLHQSGY |
| TGME49_309930-t26_1-p1 | melibiase subfamily protein | 88 | MTEDRASDPGWETVAFQAPSRLPRKPQRTSARPMFH CLLRVTFLFALSFLCLTASEGTMPAYENTLQRAQINDGQ SHARHGSERLTEESAAYVQREALHEEGAAEQATVEGN QERKPDSRTRRDVDGKIPCLTQAWSATGEQKEDSCTN LLKAAEAGVRRSASSVLAEGSEERSNLGFLQWNEGGG ARPYSWRVAQTAGRDDSVPVGEKLMRRDREQVGSIN HHGHQEGNREGKAGDQVLESGTMDLFDEEDDPNPSL YLSDGDEPAALADPEDEDFIWGDEDEDGPFLDGDADE YSLSYDVDAAEDALNLRENEDPPNEGGMNVDQEADE PFGVDAEADTETANAAHTRPPDHFLQRYNDDVVPSPR ATPLPADAEEPFVSASTAEVNGLAERKEFAKWSQDTAP LVVPNKHEEGDGIGYQGLVNISENESSSPARTPRGKANI PKPPMGWSSWNRFECDLSELNEKLVMDTALALEQSG LHAAGYEYVMLDDCWSIKKDEERGAREPIVWDAKRFP NGMPALASFLHSRGFRFGLYTDSGSRTCMGYIGSADH EEQDAMAFQSWGVDFLKIDGCYAEPSEMGMLYSRW SPAFEYAAAANPDHKQTVVLSCSWPAYVHNPTDFDFK LIGEMCDTWRIFDDIQPTWESLSAIMKFWGDNQGIFA NVVAPGRFNDPDMLEVGNGNFSTAEGRTQMSVWSI VAAPLILGNDVRNMTPQTLQILANPEVIAVNQDDLILE GLRVFESPNTMSIWMRPLAAGSTAVAFVNLSSKPRNV TINLSELQKAYWESWLPWRRRKLLRLLDNIPPADRERL VQVHFPPVDNDPTAPFGCTVRDVWLGLNLGFKERYIV SPYILAPHDTFMVIISGCTLTGEPHRFSSANDETQSPELR KAGVRERDENTGVRRENNNGEARGTEPDIDILSGHVS VAGHVEQVRGITGSGEQQRNSPRQAVGEEAETYPAV VANQGNIPAHSLRSRIDREM |
| TGME49_309990-t26_1-p1 | hypothetical protein | 89 | MGNMCAICFNPGKAASCSCFNLAVAVLLFAVYYGLVG GMDLYQATKSHPVLVTQKYNAIFTLSVATALLVSVVLG |
| | | | VLKLGIVSYIFTMMVFVLQLSDAIFKLVTFILNWVDWGI ALHDGTMSFQSAMVTSSISQVLTIILTFSMANILYSAA MVYKVGGNGCEYRNYKQIESSKNASSTKKEDNEAATE V |
| TGME49_310670-t26_1-p1 | glycogen phosphorylase 1, putative (GP) | 90 | MANAADENVFANDSSYHWEMRRKASFSKLTGAVPRA IPGMYNEEDPHADEKKEKLWKLMETYISSDIHSIQRSIV NHVEYTCARTRFNCDPESCYRASAFSVRDRLIETLNDTN AYFHEKDCKRAYYLSLEFLLGRAFQNALVNLDIENNYKK ALAELGFNLEQLYEFEHDPALGNGGLGRLAACFLDSMA TVNLPCWGYGIRYTYGIFEQKIVNGRQVEHPDYWLTM SNPWEIERPDCTYAVRFYGSVKEYRDVQTGRMRSKW VEGEIVQAMAYDNPIPGFDTYNTINLRLWKAAPGKEF DFHLFNVGRYLESVRERQRAESISAVLYPNDNTLEGKEL RLKQQYFFVCATVQDVLRRFKKVSNRDWNDLPSKVQ MQLNDTHPTIAIPELMRILLDVEGLEWEQAWDLTKQIF NYTNHTVLPEALEKWSAELIGKLLPRHLLIINEINFRFLNE VRGVFGDDWNKISRMSIYEEGEEKRIRMANLAVIGSR HVNGVAAIHSELVKKDLFPEFVEFYSRQGFNNKFLNVT NGVTPRRWIYCANRGLADLFSNWLGSDSWLKELDMV AGLQNHIDDPQLRKEWRAVKRENKKRLAAWVEQRCN VKLDVDRMLFDIQVKRIHEYKRQLLNCLYTLHRYLTLKK MSPHERENVVPRATMIGGKAAPGYFTAKNIIKLVNNIA QVVNNDPDVNQYLKVVFLPNYNVSHAQVIIPASDLSQ HISTAGTEASGTSNMKFVMNGGLILGTLDGANIEIREE CGDDTMFIFGAREHEVAKIREQARNGNYPIDGRLREVF DFIRSGKLACGDGQAQSDFVAIVDQLCNNGYGHNGD FYLLIHDFQDYCRAQHVVDETYKDVEQWTTLSIKAASS MGKFSTDRCMREYATEVWDIEPCERPPPDEFQRMRS FANDIPNKVSA |
| TGME49_312600-t26_1-p1 | heat shock protein HSP21 | 91 | MASPTSASSKAPGTESEACGHRHPGHVGATHTYNWD EFRQFLDSQGSLWHSAVLGAPASAAPNTIFELAPTAAE AGRAIAFRPRLDAYYDTAAHKIVMLFDLPGFEKKDISVE VTDHAIIISGTRDRLNDKVLYGEKSRELIKERAFGHFCRK FQLPTNAVEDAVTAAMTGGVLEVTVETREEEASHTKK KIQIA |
| TGME49_313050-t26_1-p1 | oxidoreductase, short chain dehydrogenase/red uctase family protein | 92 | MDIQAVGSVLASALASPLLLAVACCASLKLAAGALNFI WSLCAAYLLRQADFWTMLLPEKTGYWRNRVAWVTG SSSGVGLSLCRLLAHRGCRVIMSSRKIEDLQAAKQDVV TFCSLKGVQKSENDFLLLPFDLLQLESFEDIVVKATAWA PNGRIDFLFNNAGIASRGLFMSYEAAEKIIKTDLLAQMK LTKLLLPIMSKAGFGHIIFTNSGRSKLVACGHEPYSVSKV GLLCFAEALSRDLKAKNMNIFVTSALPSYIRTKISYKTLG PDGTPLTDDTSYMHTDTAAGLPPDTVARYMLKAASNK LRECWIATTPVLFYLYLQAYLPDLASSLLDLRAEAHALEI EQEAQELEEIAQRVSRCPPHST |
| TGME49_320190-t26_1-p1 | SAG-related sequence SRS16B | 93 | MERTGNMQQRLGGFKSQVRKLMAVCIGGILLLSSGQ GLADSLHEGLQSLSLKSQVGVSSKVDVTGTHAKCEFST AGAGSSTAVSKQSLTLSKGSLTATLECTAKDGGTISSIPN PLAGNVCVTKQKQSETPCNFGENGGAAASVQVPLKD VLGTTSAVQWMNTTAEQKAIQSQKWTLKLNEEDLPIS DKTFDVGCTYTTTGKAAANPAVCQLTVNVKARASSVA |
| | | | DENVVTCSYGAESNSEALKVDMTTTRNTVTIDCGSEGT LNPAADSAQYCNPQNKDLDDCKKKFVDILPNFVISWW KTPTPGNASILTIPAADFPESEQQFRLGCVPNKAPQSDS QDTRVDNQSQVQSRTSAPTTCSVLVTVKAASSASYASP SLQTLIAVSGAAVVSGFVVASL |

| | | | |
|---|---|---|---|
| ¹Accession number and protein description for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format); ² Example of predicted sequence for the corresponding protein on the ToxoDB database (ToxoDB-51 TgondiiME49 AnnotatedProteins.fasta, Last modified: 2021-03-06 09:21; Size: 9.2M | | | |

In a further embodiment, the protein composition of the present invention further comprises all native tachyzoite-specific proteins (also referred to as **"native tachyzoite-specific protein"** in the present description) listed in Table 1. As described above in the present description, the skilled person is able to identify native tachyzoite-specific proteins, see, e.g., Buchholz et al., 2011 (Eukaryot Cell. 10: 1637-47) and Tu et al., 2018 (Microbes Infect. 20(9-10):466-476). In a further embodiment, the protein composition of the present invention further comprises all of the native bradyzoite and/or cyst specific proteins listed in Table 2. As described above in the present description, the skilled person is able to identify native bradyzoite and/or cyst specific proteins. For instance, Tu et al., 2019 (mBio. 10(2): e00469-19) provides a data set of potential *T. gondii* cyst wall proteins, identifying known cyst wall proteins and validating several novel cyst wall proteins, see, e.g., Table 1 on p. 5. Hence, in a preferred embodiment, the protein composition of the present invention comprises all of the native specific tachyzoite proteins of Table 1 and all of the native specific bradyzoite and/or cyst proteins of Table 2.

In a further preferred embodiment, the protein composition of the present invention comprises all of native tachyzoite-specific proteins listed in Table 1, all of the native bradyzoite and/or cyst specific proteins listed in Table 2, and at least one native *T. gondii* acute phase infection-specific protein selected from listed in Table 3, preferably all of the proteins depicted in Table 3.

In another preferred embodiment, the protein composition of the present invention comprises all of the native tachyzoite-specific proteins listed in Table 1, all of the native bradyzoite and/or cyst specific proteins listed in Table 2 and at least one native *T. gondii* chronic phase infection-specific protein selected from listed in Table 4, preferably all of the proteins depicted in Table 4.

In another preferred embodiment, the protein composition of the present invention comprises all of the native tachyzoite-specific proteins (listed in Table 1), all of native bradyzoite and/or cyst specific proteins (listed in Table 2), at least one native *T. gondii* acute phase infection-specific protein selected from listed in Table 3, and at least one native *T*. *gondii* chronic phase infection-specific protein selected from listed in Table 4, preferably all of the proteins depicted in Table 3 and all of the proteins depicted in Table 4.

In a preferred embodiment, the protein composition of the present invention comprises all native specific tachyzoite proteins of Table 1 and all native specific bradyzoite and/or cyst proteins of Table 2, all of the native proteins specific of the acute phase (acute infection) of Table 3, and all of the native proteins specific of the chronic phase (chronic infection) of Table 4.

Hence, in a more preferred embodiment, the protein composition of the present invention comprises all of the proteins listed in Tables 1, 2, 3 and 4.

Preferably, the protein composition of the present invention does not comprise live tachyzoites and/or live bradyzoites, that can infect and multiplicate in host tissues. The skilled person is able to verify the viability of tachyzoites/bradyzoites under bioassay of a highly susceptible host to *T. gondii* infection (e.g., experimental infection in mice) and later detection of the parasite in target host tissues out of point of inoculation (e.g., such as the brain) by microscopy identification of cysts, histology techniques or parasite DNA detection by a *T. gondii* specific PCR *-Polymerase chain reaction-.* Alternatively, the skilled person could also determine tachyzoite and bradyzoite viability *in vitro,* by means of inoculation of the parasite on permissive cell cultures and after successive passages for allowing multiplication of the parasite, under microscopy observation of the growth of the parasite in cell cultures or parasite DNA detection by a specific *T. gondii* PCR.

In one embodiment, the composition of the present invention as described herein also comprises variants, such as functional variants or functional equivalents, chimeras, functional equivalents and/or or truncated versions of the proteins described herein.

By **"functional variants"** or **"functional equivalents"** is referred herein to amino acid sequences that differ in their amino acid sequences, respectively, from that of SEQ ID NO referred to in the specific embodiment, but which perform the same function and provide the same utility or technical effect as the SEQ ID NO: referred to in the specific embodiment. Preferably, the functional variant or functional equivalent is a protein with at least 80%, preferably at least 90%, more preferably at least 95%, or at least 99% identity with the SEQ ID NO referred to in the specific embodiment, which perform the same function and provide the same utility or technical effect as the SEQ ID NO: referred to in the specific embodiment.

**"Percent (%) amino acid sequence identity"** with respect to proteins or polypeptides described herein is defined as the percentage of amino acid residues in a candidate sequence that are identical with the amino acid residues in the reference sequence (i.e., the protein or polypeptide from which it is derived), after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for example, using publicly available computer software such as BLAST. Those skilled in the art can determine appropriate parameters for measuring alignment, including any algorithms needed to achieve maximum alignment over the full-length of the sequences being compared.

Preferably, the "percentage of identity" as used herein is decided in the context of a local alignment, i.e., it is based on the alignment of regions of local similarity between amino acid sequences, contrary to a global alignment, which aims to align two sequences across their entire span. Thus, in the context of the present invention, percentage identity is calculated preferably only based on the local alignment comparison algorithm.

### The method of the present invention

The present invention provides a method for producing the protein composition of the present invention. The method of the present invention comprises, or alternatively, consists of, the following steps:
a. Providing *T. gondii* cells in a hypertonic solution;
b. Centrifuging said solution obtained in step (a) under conditions suitable for separating the soluble fraction (supernatant) and insoluble fraction (precipitate);
c. Recovering the precipitate from step (b); and
d. Mixing said precipitate with a non-ionic surfactant.

Preferably, the *T. gondii* cells comprise both *T. gondii* tachyzoite and bradyzoite stages. As discussed in detail above, *T. gondii* transitions in the host from the fast-growing tachyzoite stage to latent bradyzoite stage. Hence, in a preferred embodiment, the *T. gondii* cells provided in step a. of the method of the present invention comprise both stages of the parasite, i.e., tachyzoites and bradyzoites. As also described above, the skilled person is able to identify and select *T. gondii* cells with the tachyzoite morphology and with the bradyzoite morphology inside cysts, e.g., by identifying tachyzoite and/or bradyzoite-specific proteins expressed by a certain parasite cell. Dubey JP. et al., 1998 ("Structures of Toxoplasma gondii tachyzoites, bradyzoites, and sporozoites and biology and development of tissue cysts", Clin Microbiol Rev.,11(2):267-99) provides a detailed account of the biology of tissue cysts and bradyzoites including in vivo and in vitro development, methods of separation from host tissue, tissue cyst rupture, and relapse.

In particular, it is preferred that the *T. gondii* cells provided in step a. of the method of the present invention:
(i) maintain/retain the ability of forming bradyzoites and/or cysts; and/or
(ii) maintain/retain the ability of spontaneous conversion from tachyzoite stage to bradyzoite stage under conventional conditions of tachyzoite growth *in vitro,* and/or
(iii) maintain/retain the ability to produce proteins comprising cyst- and/or bradyzoite-specific proteins, associated to the chronic phase, and proteins comprising tachyzoite-specific proteins, associated to the acute phase.

Hence, the *T. gondii* cells of the present invention are capable of forming cysts. Additionally or alternatively, but not limiting for invention, the *T. gondii* cells of the present invention may maintain/retain the ability of spontaneous conversion from tachyzoite stage to bradyzoite stage under conventional conditions of parasite growth *in vitro.* The ability of spontaneous conversion from tachyzoite stage to bradyzoite stage may be present under conventional conditions of tachyzoite growth *in vitro,* as explained herein. Additionally or alternatively, exogenous stress factors, i.e. alkaline pH, IFN-gamma and other proinflammatory cytokines, chemicals or drugs, heat shock, and deprivation of nutrients have been shown to increase the efficacy of bradyzoite development *in vitro* (Ferreira da Silva et al., "Stress-related and spontaneous stage differentiation of Toxoplasma gondii", Mol. BioSyst., 2008, 4, 824-834). Hence, the *T. gondii* cells provided in step a. of the method of the present invention may maintain/retain the ability of forming bradyzoites and/or cysts as a stress-related response to hostile environmental conditions.

In the context of the present invention, the term **"conventional conditions of tachyzoite growth *in vitro"*** refers to standard conditions that are generally used for culturing *T. gondii* tachyzoite cells *in vitro. T. gondii* cells can be grown *in vivo* or *in vitro.* Typically, there are two fundamental reasons for doing parasite cultivation *in vitro.* The first is that it provides long-term preservation of strains without having to resort to laboratory mammals. The second is the cheap production of a large number of parasite cells, especially for applications such as antigens, since it is possible to produce a large quantity of pure, extracellular, or recovered intact parasites, see, e.g., Thais Alves da Costa-Silva et al., 2012, "Toxoplasma gondii antigens: Recovery analysis of tachyzoites cultivated in Vero cell maintained in serum free medium", Experimental Parasitology, 130(4): 463-469). *In vitro* protocols for the maintenance and growth of *T. gondii* are also described in, e.g., Khan A. and, Grigg ME., "Toxoplasma gondii: Laboratory Maintenance and Growth", Curr Protoc Microbiol, 2017, 44:20C.1.1-20C.1.17 or in Saadatnia G. et al., 2010, "Optimization of Toxoplasma gondii cultivation in VERO cell line", Trop Biomed, 2010; 27(1): 125-30. Various cell lines are used for *T. gondii* culture *in vitro.* For instance, as described in Jabari S. et al., 2018 ("In vitro culture of Toxoplasma gondii in HeLa, Vero, RBK and A549 cell lines", Infez Med., 2018;26(2):145-147), *T. gondii* cells can be grown, *inter alia,* in HeLa, Vero, RBK and A549 cell lines. *T. gondii* is also routinely propagated *in vitro* in human fibroblast cells - HFF - as tachyzoites by successive passages (Ferreira da Silva et al., 2008, Mol. BioSyst. 4: 824-834). In a preferred embodiment, *T. gondii* is grown *in vitro* in Vero cells. Originally, Vero cells were established in Japan from the kidney of a normal adult African green monkey (*Cercopii-hecus aethiops*)*.* Studies have shown that Vero cell lines are free of oncogenic properties, not presenting risks to humans when used as substrate in the production of vaccines. These cells have been used extensively for production of human virus vaccines in large-scale production as microcarriers or cultures in bioreactors. These procedures have also been recognized by the WHO (Thais Alves da Costa-Silva et al., 2012, "Toxoplasma gondii antigens: Recovery analysis of tachyzoites cultivated in Vero cell maintained in serum free medium", Experimental Parasitology, 130(4): 463-469). For example, the cell line ATCC-CCL-81 can be obtained from the American Type Culture Collection and can be used for culturing *T. gondii in vitro.* The Vero cell line ATCC-CCL-81 was initiated in 1962 from the kidney tissue derived from a normal, adult African green monkey. The cell line can be used in a variety of applications, including the detection of verotoxins, detection of virus in ground beef, efficacy testing, the study of malaria, media testing, and mycoplasma testing. The cells are grown under conventional conditions for growing the cells (i.e., with suitable cell culture medium, possibly in the presence of antibiotics-antimycotics, supplemented or not with serum (e.g., foetal bovine serum 10%), at a suitable temperature (e.g., about 37°C) and under a suitable CO₂ pressure (e.g., about 5% CO₂), and the *T. gondii* cells are then added to the culture to infect the cells. Foetal bovine serum concentration could be diminished to 1-2% after medium change previously to *T. gondii* inoculation. For instance, conventional conditions of growth *in vitro* are described in Example 1 in this description, e.g., culture in cell (such as Vero cells, as explained above) monolayer in culture medium (such as Dulbecco's Minimum Essential Medium (DMEM) supplemented with antibiotic-antimycotic solution and serum (such as foetal bovine)), and incubated at a suitable temperature (i.e., about 37°C) in about 5% CO₂. Hence, DMEM at pH of about 7.2 with 5% CO₂ can be used to maintain and culture *T. gondii.*

Induction of tachyzoite to bradyzoite conversion of laboratory isolates is preferably performed under exogenous stress factors - non-conventional conditions-, i.e., alkaline pH, IFN-gamma and other proinflammatory cytokines, chemicals or drugs, heat shock, and deprivation of nutrients have been used for bradyzoite development *in vitro* as it is reviewed by Ferreira da Silva et al., 2008, "Stress-related and spontaneous stage differentiation of Toxoplasma gondii" Mol. BioSyst. 4: 824-834). *T. gondii* tachyzoite to bradyzoite conversion and cyst formation can occur also spontaneously without applying any stress factor in Vero cells in isolates recently obtained from infected hosts (Colos-Arango et al., 2023, Int J Parasitol. 53(9): 491-504).

As explained in detail above, the skilled person is able to ascertain whether a certain *T*. *gondii* cell is able to maintain/retain the ability of forming cysts, maintain/retain the ability of spontaneous conversion from tachyzoite stage to bradyzoite stage and maintain/retain the ability to produce specific tachyzoite and bradyzoite proteins associated to the chronic or acute phases (chronic or acute-stage specific proteins). For instance, the presence of bradyzoites, tachyzoites and/or cyst structures can be detected in *T. gondii in vitro* culture. See, e.g., Sahm M. et al., 1997, "Cyst formation by Toxoplasma gondii in vivo and in brain-cell culture: a comparative morphology and immunocytochemistry study", Parasitol Res., 1997;83(7):659-65 and Colos-Arango et al., 2023, Int J Parasitol. 53(9):491-504). In addition, as already discussed, the skilled person is able to specifically detect proteins associated to the chronic and acute phases, comprising cyst- and bradyzoite-specific proteins or tachyzoite-specific proteins, respectively. Bradyzoite and cyst production may be for instance detected with protein production of specific bradyzoite marker such as TgBAG1, and/or differentiated from tachyzoite by absence of specific tachyzoite marker such as TgSAG1, and the formation of cyst wall rounding parasitophorous vacuole (Lane et al., 1996. Parasitol. Res. 82: 340-346).

In one preferred embodiment, the *T. gondii* cells which are provided in step a. of the method of the present invention are not older than 40 passages, preferably not older than 37 passages, more preferably not older than 33 passages, even more preferably not older than 31 passages. As described above, for *T. gondii in vitro* culture, a cell line (such as Vero cells) may be used for *T. gondii* infection and multiplication for parasite growth. Parasite growth in culture cells follows the lytic cycle (also in tissues of the hosts) governed by adhesion of the parasite to the cell host, cell invasion with the parasite internalization, host cell adaptation and formation of the membrane of the parasitophorous vacuole where the protozoan will survive and multiply in successive divisions until egression with the lysis of host cell for initiation of new cycles of multiplication on neighbouring cells. Also, within a few hours of infection, tachyzoites localized within a parasitophorous vacuole may gradually begin to change their metabolism, slowing the division rate and converting into bradyzoites (see, e.g., Attias et al., 2020, Parasit Vectors. 13(1):588). *T. gondii* cells are recovered from cell cultures *in vitro* during egression and subcultured, i.e., harvested when parasite egression occurs, completing replication and reseeded into multiple 'daughter' cell culture flasks. Hence, each passage of the cells may correspond to one lytic cycle of the parasite. In the context of the present invention, the terms **"cells that are not older than x passages",** or **"cells that have been passaged nor more than x times"** (or the like) refer to the number of times that a *T. gondii* infected culture has been subcultured.

As used in the present invention, a **"hypertonic solution"** may be defined as a solution that has higher osmotic pressure or has more concentration of solutes than another solution to which it is compared, in this case the solute concentration higher than solution inside the cells, the *T. gondii* cells.

Preferably, the hypertonic solution comprises osmotic shock elements such as sucrose and/or sorbitol, and/or mannitol. If the hypertonic solution comprises (or, alternatively, consists of) sucrose, the amount of sucrose is preferably about 10-80% (w/v in PBS), more preferably about 15-40% (w/v in PBS), even more preferably about 20% (w/v in PBS).

Preferably, the centrifugation takes place at about 8000-15000×g, during about 40-90 min and at a temperature of about 1-10 °C, preferably at about 10000×g, during about 60 min at about 4°C.

As used herein, non-ionic surfactants are surfactants comprising a polar, but uncharged hydrophilic group. Preferably, the non-ionic surfactant is selected from the group consisting of Cetomacrogol 1000, Cetostearyl alcohol, Cetyl alcohol, Cocamide DEA, Cocamide MEA, Decyl glucoside, Isoceteth-20, Lauryl glucoside, Monolaurin, Narrow range ethoxylate, Nonidet P-40, Nonoxynol-9, Nonoxynols, NP-40, Octaethylene glycol monododecyl ether, N-Octyl beta-D-thioglucopyranoside, Octyl glucoside, Oleyl alcohol, Pentaethylene glycol monododecyl ether, Poloxamer, Poloxamer 407, Polyglycerol polyricinoleate, Polysorbate, Polysorbate 20, Polysorbate 80 (Polyoxyethylene (20) sorbitan monooleate), Sorbitan monostearate, Sorbitan tristearate, Stearyl alcohol, Tween 80, Triton X-114, Tween 20 (Polyoxyethylene (20) sorbitan monolaurate) and Triton X-100. Preferably, the non-ionic surfactant is selected from the group consisting of Polysorbate 80, Triton X-100, Tween 20 and Nonidet P-40 (i.e., octylphenoxypolyethoxyethanol, branched, also known as Octylphenoxy poly(ethyleneoxy)ethanol, branched). Even more preferably, the non-ionic surfactant is Nonidet P-40, or octylphenoxypolyethoxyethanol, branched, also known as Igepal^{®} Ca-630, which can be obtained from Sigma-Aldrich, with product number I8896 and CAS Number 9002-93-1.

In a preferred embodiment, the method of the present invention further comprises the step (e) of homogenising the mixture obtained after step (d). Homogenising procedures include those carried out by physical methods such as sonication, hydrodynamic cavitation, and high-pressure homogenization. Homogenization methods are described, e.g., in Balasundaram et al., 2009, Trends in Biotechnology 27(8): 477-485.

Preferably, the *T. gondii* cells are *Toxoplasma gondii* tachyzoites and/or bradyzoites, and even more preferably they belong to the PCR-RFLP genotypes ToxoDB #2 and/or #3, see, e.g., Su et al., "Globally diverse Toxoplasma gondii isolates comprise six major clades originating from a small number of distinct ancestral lineages", PNAS, 2012, 109:5844-9.

In a particularly preferred embodiment, the *T. gondii* cells belong to the isolates TgShSp3 and/or TgPigSp1. These two isolates have been deposited on July 28, 2023, by SALUVET-innova S.L. (address: Veterinary Sciences Faculty, Complutense University of Madrid, Avd. Puerta de Hierro s/n, 28040, Madrid, Spain) in the Culture Collection of Algae and Protozoa (CCAP, SAMS Limited, Scottish Marine institute, OBAN, Argyll PA37 1QA, Scotland, United Kingdom) under the Budapest Treaty, with accession number CCAP 2074/1 and CCAP 2074/2, respectively. Hence, in a preferred embodiment, the *T. gondii* cells belong to the isolates with deposit number CCAP 2074/1 (TgShSp3) and CCAP 2074/2 (TgPigSp1).

### The pharmaceutical composition of the present invention

The present invention further provides a protein composition (directly) obtainable or obtained by the method according to the present invention.

The composition is preferably a **pharmaceutical composition.** The present invention further provides a pharmaceutical composition (or pharmaceutical formula) comprising the protein composition of the present invention. Preferably, the pharmaceutical composition (or pharmaceutical formula) comprises one or more excipients and/or one or more pharmaceutically acceptable carriers or diluents. Suitable pharmaceutically acceptable carriers or diluents are for example water, culture fluid, a solution of physiological salt concentration and/or stabilisers such as SPGA, carbohydrates (e.g., sorbitol, mannitol, starch, sucrose, glucose, dextran), proteins such as albumin or casein, protein containing agents such as bovine serum or skimmed milk and buffers (e.g., phosphate buffer).

The pharmaceutical composition (or pharmaceutical formula) of the present invention may comprise immunomodulant-immunostimulant substances (i.e., substances that stimulate the immune system by inducing activation or increasing activity of any of its components), such as cytokines.

In one embodiment, the pharmaceutical composition or pharmaceutical formula of the invention is a **vaccine.** In the context of the present invention, the term **"vaccine"** refers to an antigenic preparation used to establish immune system response to a disease. The vaccines described herein can be used against pathogenic organisms or to induce tolerance against antigens which cause allergies or against autoantigens triggering autoimmune diseases. Preferably, the vaccine according to the present invention is used in the treatment or prevention of infections caused by *Toxoplasma,* preferably by *T. gondii,* (e.g., toxoplasmosis).

The vaccine may preferably comprise one or more adjuvants. Vaccine adjuvants are chemicals, microbial components, or mammalian proteins that enhance the immune response to vaccine antigens (Spickler & Roth, 2003, J. Vet. Intern. Med. 17: 273-281, which reviews modes of action and adverse effects of adjuvants in veterinary vaccines). The adjuvant useful in the context of the present invention may be an inorganic or organic chemical, macromolecule or whole cells of certain killed bacteria which enhances the immune response to given antigen. In the context of the present invention, the adjuvant that may be present in the composition of the invention can be any suitable adjuvant which e.g., enhances, accelerates, and prolongs the specific immune response as known in the current art.

Major type of adjuvants comprise alum and calcium salts, oil emulsion adjuvants (comprising a mixture of oil and aqueous phases, stabilized by a surfactant), liposomes and archaeosomes, nanoparticles and microparticles, saponins, immune-stimulating complexes, nonionic block copolymers, carrier proteins (such as diphtheria or tetanus toxoid, KLH, and bovine serum albumin), bacterial products and their derivatives, derivatized polysaccharides, cytokines, complement derivatives (Spickler & Roth, 2003, J. Vet. Intern. Med. 17:273-281).

Adjuvants useful in the context of the present invention may include for instance:
- Mineral salts, e.g., aluminium hydroxide and aluminium or calcium phosphate gels.
- Oil emulsions and surfactant based formulations, e.g., MF59 (microfluidised detergent stabilised oil-in-water emulsion), QS21 (purified saponin), AS02 [SBAS2] (oil-in-water emulsion + MPL + QS-21), Montanide^{™} ISA-51, ISA-720, IMS (stabilised water-in-oil emulsion).
- Particulate adjuvants, e.g., virosomes (unilamellar liposomal vehicles incorporating influenza haemagglutinin), AS04 ([SBAS4] Al salt with MPL), ISCOMS (structured complex of saponins and lipids), polylactide co-glycolide (PLG).
- Microbial derivatives (natural and synthetic), e.g., monophosphoryl lipid A (MPL), Detox (MPL + *M. phlei* cell wall skeleton), AGP [RC-529] (synthetic acylated monosaccharide), DC_Chol (lipoidal immunostimulators able to self organise into liposomes), OM-174 (lipid A derivative), CpG motifs (synthetic oligonucleotides containing immunostimulatory CpG motifs), modified LT and CT (genetically modified bacterial toxins to provide non-toxic adjuvant effects).
- Endogenous immunomodulators (immunomodulant-immunoestimulant substances), such as cytokines, e.g., GM-CSF or IL-12 (cytokines that can be administered either as protein or plasmid encoded), Immudaptin (C3d tandem array)
- Inert vehicles, such as gold particles towards the desired response to vaccine antigens.

Preferably, the vaccine of the invention comprises one or more adjuvants, preferably a saponin adjuvant. Saponins are complex chemical adjuvants extracted from plants, most often the tree *Quillaia saponaria.* The crude extract from this tree is called saponin. Sun et al., 2009 (Vaccine, 27(12):1787-1796) reviews advances in saponin-based adjuvants.

The saponin may be any saponin suitable to act as an adjuvant. Preferably, the saponin is Quil-A^{®}, a saponin obtained from *Quillaja saponaria* (soap bark tree or Soapbark).

The adjuvant is preferably present in the final pharmaceutical composition (or pharmaceutical formula or vaccine) in a concentration of about 0.001% to 50% w/v with respect to the final volume of the composition, preferably 0.001%, 0.005%, 0.01%, 0.05%, 0.1%, 0.2%, 0.5%, 1%, 10%, 50% or more (w/v, i.e., weight in grams contained in a final volume of 100 ml). More preferably, the concentration of adjuvant in the final formula is about 0.03% w/v with respect to the final volume (w/v, i.e., weight in grams contained in a final volume of 100 ml).

Hence, the protein composition and/or the pharmaceutical composition (or the pharmaceutical formula) and/or the vaccine according to the present invention may be formulated in any pharmaceutical formulation able to achieve the desired effect.

For example, the protein composition and/or the pharmaceutical formula (or pharmaceutical composition) and/or the vaccine according to the present invention may be formulated as tablets, capsules, pills, emulsions, suspensions, or solutions.

Preferably, the protein composition and/or the pharmaceutical formula (or pharmaceutical composition) and/or the vaccine according to the present invention comprises (or, alternatively, consists of) an amount of about 0.0001% to about 0.5% w/v (w/v, i.e., weight in grams contained in a final volume of 100 ml) of the protein composition of the invention, preferably an amount of about 0.0005% to about 0.2% w/v, more preferably an amount of about 0.001% to about 0.02% w/v of said protein composition, even more preferably an amount of about 0.002% and 0.004% w/v of said protein composition.

The pharmaceutical composition (or pharmaceutical formula) preferably further comprises a suitable adjuvant (preferably saponin, more preferably a saponin obtained from *Quillaja saponaria* such as Quil-A^{®}), preferably in an amount of about 0.001% to 50% w/v, more preferably in an amount of 0.015% to 0.03% w/v, with respect to the final volume of the composition.

For example, the pharmaceutical formula (or pharmaceutical composition) and/or the vaccine of the present invention comprises an amount of about 0.0001% to about 0.5% w/v of the protein composition of the present invention and an amount of about 0.001% to 50% w/v of a suitable adjuvant (preferably saponin, more preferably a saponin obtained from *Quillaja saponaria* such as Quil-A^{®}), with respect to the final volume of the composition.

For example, the pharmaceutical formula (or pharmaceutical composition) and/or the vaccine of the present invention comprises an amount of about 0.0005% to about 0.2% w/v of the protein composition of the present invention and an amount of about 0.001% to 50% w/v, preferably about 0.01% to 0.2% w/v, even more preferably about 0.015% to 0.03% w/v of a suitable adjuvant (preferably saponin, more preferably a saponin obtained from *Quillaja saponaria* such as Quil-A^{®}), with respect to the final volume of the composition.

For example, the pharmaceutical formula (or pharmaceutical composition) and/or the vaccine of the present invention comprises an amount of about 0.0005% to about 0.2% w/v of the protein composition of the present invention and an amount of about 0.015% to 0.03% w/v of a suitable adjuvant (preferably saponin, more preferably a saponin obtained from *Quillaja saponaria* such as Quil-A^{®}), with respect to the final volume of the composition.

For example, the pharmaceutical formula (or pharmaceutical composition) and/or the vaccine of the present invention comprises an amount of about 0.0005% to about 0.2% w/v of the protein composition of the present invention and an amount of about 0.015% to 0.03% w/v of a suitable adjuvant (preferably saponin, more preferably a saponin obtained from *Quillaja saponaria* such as Quil-A^{®}), with respect to the final volume of the composition.

For example, the pharmaceutical formula (or pharmaceutical composition) and/or the vaccine of the present invention comprises an amount of about 0.001% to about 0.02% w/v of the protein composition of the present invention and an amount of about 0.01% to 50% w/v, preferably about 0.01% to 0.2% w/v, even more preferably about 0.015% to 0.03% w/v of a suitable adjuvant (preferably saponin, more preferably a saponin obtained from *Quillaja saponaria* such as Quil-A^{®}), with respect to the final volume of the composition.

For example, the pharmaceutical formula (or pharmaceutical composition) and/or the vaccine of the present invention comprises an amount of about 0.001% to about 0.02% w/v of the protein composition of the present invention and an amount of about 0.015% to 0.03% w/v of a suitable adjuvant (preferably saponin, more preferably a saponin obtained from *Quillaja saponaria* such as Quil-A^{®}), with respect to the final volume of the composition.

For example, the pharmaceutical formula (or pharmaceutical composition) and/or the vaccine of the present invention comprises an amount of about 0.001% to about 0.02% w/v of the protein composition of the present invention and an amount of about 0.015% to 0.03% w/v of Quil-A^{®}, with respect to the final volume of the composition.

Preferably, the pharmaceutical formula (or pharmaceutical composition) and/or the vaccine of the present invention comprises an amount of about 0.002% to about 0.004% w/v of the protein composition of the present invention and an amount of about 0.015% to 0.03% w/v of a suitable adjuvant (preferably saponin, more preferably a saponin obtained from *Quillaja saponaria* such as Quil-A^{®}), with respect to the final volume of the composition.

### Medical uses of the present invention

Preferably, the protein composition and/or the pharmaceutical formula and/or the vaccine according to the present invention are used as a medicament (medicinal product), preferably in a method of therapeutic treatment (after infection or after the clinical manifestation of the disease caused by the infection) and/or prophylactic treatment (before infection or before the clinical manifestation of the disease caused by the infection) of infections caused by *Toxoplasma,* such as infections caused by *T. gondii* (e.g., toxoplasmosis).

The protein composition and/or the pharmaceutical formula (or pharmaceutical composition) and/or the vaccine according to the present invention may preferably be used as a medicament. A **"medicament"** in the context of the present invention is understood as a compound or composition used to diagnose, cure, treat, or prevent a condition or disease. Hence, the present invention provides the use of the protein composition and/or the pharmaceutical formula (or pharmaceutical composition) and/or the vaccine according to the present invention as a medicament, or the use of the protein composition and/or the pharmaceutical formula (or pharmaceutical composition) and/or the vaccine according to the present invention for the preparation of a medicament.

The protein composition, pharmaceutical formula (or pharmaceutical composition) or vaccine of the invention can be used both in asymptomatic patients as well as in those who have already shown symptoms of the disease. The protein composition and/or the pharmaceutical formula (or pharmaceutical composition) and/or the vaccine according to the present invention may be administered before the infection, and/or after it.

The protein composition and/or the pharmaceutical formula (or pharmaceutical composition) and/or the vaccine according to the present invention may be used in a method of therapeutic treatment (after infection or after the clinical manifestation of the disease caused by the infection) and/or prophylactic treatment (before infection or before the clinical manifestation of the disease caused by the infection) of infections caused by *Toxoplasma* such as infections caused by *T. gondii* (e.g., toxoplasmosis). Hence, the present invention provides the use of the protein composition and/or the pharmaceutical formula (or pharmaceutical composition) and/or the vaccine according to the present invention in a method of therapeutic treatment and/or prophylactic treatment of infections caused by *Toxoplasma* such as infections caused by *T. gondii* (e.g., toxoplasmosis). The present invention thus provides the use of the protein composition and/or the pharmaceutical formula (or pharmaceutical composition) and/or the vaccine according to the present in the preparation of a medicament to treat infections caused by *Toxoplasma,* such as infections caused by *T. gondii* (e.g., toxoplasmosis).

The protein composition and/or the pharmaceutical formula (or pharmaceutical composition) and/or the vaccine according to the present invention may be administered to a mammal, including marsupialia and placentalia. Clade mammal groups comprise a high diversity of orders involving marsupials, carnivora, primates and cetartyodactila that includes cetacean, ruminants, and suidae. The protein composition and/or the pharmaceutical composition and/or the vaccine according to the present invention may be administered to a mammal selected from the group consisting of domestic pig *(Sus scrofa and Sus scrofa domestica*)*,* sheep (*Ovis aries*), goat (*Capra aegagrus hircus*)*,* non-human primates, felids such as cats (*Felis catus*) and marsupials. Marsupials are any members of the mammalian infraclass Marsupialia. Living marsupials include opossums, Tasmanian devils, kangaroos, koalas, wombats, wallabies, and bandicoots among others. Non-human primates are a group of mammals composed of simians - monkeys and apes - and prosimians, such as lemurs.

The protein composition and/or the pharmaceutical composition and/or the vaccine according to the present invention may also be administered to human beings (humans).

Hence, protein composition and/or the pharmaceutical composition and/or the vaccine of the present invention may be administered to a mammal, preferably to a mammal selected from the group consisting of domestic pig (*Sus scrofa and Sus scrofa domestica*)*,* sheep (*Ovis aries*), goat (*Capra aegagrus hircus*)*,* non-human primates, humans (*Homo sapiens*)*,* cats (*Felis catus*) and marsupials.

The protein composition and/or the pharmaceutical composition (or pharmaceutical formula) and/or the vaccine according to the present invention may be administered to a mammal in an amount of about 0.001-10 µg of the protein composition of the invention per kg of the individual (mammal) (µg/kg) to which the pharmaceutical formula (or pharmaceutical composition) and/or the vaccine is administrated, preferably an amount of about 0.01-2 µg/kg, such as in an amount of 0.01-1 µg/kg.

Preferably, the pharmaceutical composition (or pharmaceutical formula) and/or the vaccine according to the present invention may be administered to a mammal at least two times, with at least 14-21 days between each of the administrations, such as 14, 15, 16, 17, 18, 19, 20 and 21 days between each of the administrations. For example, one dose of the pharmaceutical formula (or pharmaceutical composition) and/or the vaccine according to the present invention may be administered to a mammal at day 0 and, after 21 days, another dose is administered to the mammal.

For example, a dose of 2 µg/kg may be administered to a mammal at day 0 and another dose of 2 µg/kg may be administrated to the same animal after 21 days.

For example, a dose of 0.8 µg/kg may be administered to a mammal at day 0 and another dose of 0.8 µg/kg may be administrated to the same animal after 21 days.

For example, a dose of 0.08 µg/kg may be administered to a mammal at day 0 and another dose of 0.08 µg/kg may be administrated to the same animal after 21 days.

Preferably, mammals are re-vaccinated (namely, the pharmaceutical formula or pharmaceutical composition and/or the vaccine according to the present invention is administered to the mammal) about one year after the first vaccination (namely, about one year after the first administration of the pharmaceutical composition and/or the vaccine according to the present invention).

For example, one animal (preferably selected from the group consisting of non-human primates, humans, cats (*Felis catus*)*,* marsupials, domestic pig, caprine and ovine, being ovine the most preferred one) is vaccinated at day 0 from year 0, at day 21 of year 0 and at a day comprised between day 0 and day 21 (such as day 0, 1, 2, 3, 4, 5, 6, 7, 8, 9, 10, 11, 12, 13, 14, 15, 16, 17, 18, 19, 20 or 21) of year 1. For example, one animal (preferably selected from the group consisting of non-human primates, humans, cats (*Felis catus*)*,* marsupials, domestic pig, caprine and ovine, being ovine the most preferred one) is vaccinated at day 0 from year 0, at day 21 of year 0 and at day 0 of year 1. For example, one animal (preferably selected from the group consisting of non-human primates, humans, cats (*Felis catus*)*,* marsupials, domestic pig, caprine and ovine, being ovine the most preferred one) is vaccinated at day 0 from year 0, at day 21 of year 0 and at day 21 of year 1.

According to the present invention, the term **"vaccinate"** may be understood as to administer the pharmaceutical formula (or pharmaceutical composition) and/or vaccine and/or protein composition of the present invention to a mammal.

The pharmaceutical formula (or pharmaceutical composition) and/or the vaccine according to the present invention may be administered orally, intranasally, intradermally, subcutaneously, by aerosol, intramuscularly, wing web and eye-drop administration, preferably subcutaneously or/and intramuscularly or/and intradermally, more preferably subcutaneously or/and intramuscularly. For example, the pharmaceutical formula (or pharmaceutical composition) and/or the vaccine according to the present invention may be administered through the mucosa of the mammal (preferably selected from the group consisting of domestic pig, caprine and ovine, being ovine the most preferred one). For example, the pharmaceutical composition and/or the vaccine according to the present invention may be administered subcutaneously to the mammal (preferably selected from the group consisting of domestic pig, caprine and ovine, being ovine the most preferred one). For example, the pharmaceutical formula (or pharmaceutical composition) and/or the vaccine according to the present invention may be administered intramuscularly to the mammal (preferably selected from the group consisting of domestic pig, caprine and ovine, being ovine the most preferred one).

In the context of the present invention, the expression **"therapeutically effective amount"** refers to the amount of protein composition, pharmaceutical formula (or pharmaceutical composition) or vaccine of the invention that allow producing the desired effect. The pharmaceutically acceptable adjuvants and carriers that can be used in the pharmaceutical formulas (or pharmaceutical compositions) and/or vaccines are carriers known by persons skilled in the art. The compositions provided by this invention can be facilitated through any administration route, for which purpose said composition will be formulated in the suitable dosage form and with the excipients that are pharmacologically acceptable for the chosen administration route.

For purposes of the present invention, the term **"effective dose"** refers to the minimum dose capable of producing the desired effect, whether the reversion of a disease state, or inducing a specific immune response, etc.

Unless otherwise defined, all technical and scientific terms used herein have the meaning commonly understood by one skilled in the art to which this invention belongs.

### EXAMPLES

### 1. Production of the Toxoplasma gondii antigen extract (TgAE) with native tachyzoite and bradyzoite proteins

The *T. gondii* antigen extract (TgAE) according to the present invention was prepared from parasites of the TgShSp3 and TgPigSp1 isolates of *T. gondii,* deposited on July 28, 2023 by SALUVET-innova S.L. (address: Veterinary Sciences Faculty, Complutense University of Madrid, Avd. Puerta de Hierro s/n, 28040, Madrid, Spain) in the Culture Collection of Algae and Protozoa (CCAP, SAMS Limited, Scottish Marine institute, OBAN, Argyll PA37 1QA, Scotland, United Kingdom) under the Budapest Treaty, with accession number CCAP 2074/1 and CCAP 2074/2, respectively. *T. gondii* isolates TgShSp3 and TgPigSp1 were obtained from a sheep foetal brain abortion and from an adult pig myocardium, respectively, and were genetically classified of the genotype #3 (ToxoDB) or type II-PRU variant and genotype #2 (ToxoDB) or type III, respectively (Fernández-Escobar et al., 2020, Parasit. Vectors, 13: 396; Fernández-Escobar et al., 2020, Front Vet Sci., 7: 604782). These *T. gondii* isolates were maintained in Vero (ATCC^{®} CCL-81^{™}) cell monolayers by successive passages at 4-7--day intervals following standard procedures (Colos-Arango et al., 2022, Int. J. Parasitol. 53: 491-504). For each culture passage, parasites were recovered from cultures by cell scraping, passaged by 25 G needle, and inoculated onto a fresh Vero cell monolayer in Dulbecco's Minimum Essential Medium (DMEM) supplemented with 1% (v/v) antibiotic-antimycotic solution (Gibco BRL, Paisley, UK) and 1% (v/v) foetal bovine serum and incubated at 37 °C in 5% CO₂. TgShSp3 and TgPigSp1 showed capacities for spontaneous cyst formation in Vero cells at passages 29-35 (Colos-Arango et al., 2022, Int. J. Parasitol. 53: 491-504).

The parasites for the TgAE were obtained from a cellular culture maintained in identical conditions as described above from parasite passages 29-35. The parasites for the extracts were recovered from the culture using a cell scraper and centrifugation at 4 °C (1350×g, 15 min). Parasites were re-suspended in a phosphate buffer solution (PBS, pH 7.4), passed through a 25 G needle and purified through a 10 µm filter (Isopore^{™} polycarbonate membrane filters, Merck) for separating the parasites from cell debris. The number of total zoites and structures compatible with mature cyst was determined in the eluent by microscopy counting in the Neubauer chamber.

The mean percentage of recovery after parasite purification by filtration was of 75% ± 10% and higher than 1 × 10⁸ zoites by T75 flask according to the data obtained in at least 6 independent experiments **(Table 5).** Structures compatible with mature cyst were observed in all productions from the TgShSp3 isolate with an average number of 6.7 × 10⁵ for each T75 culture flask. Formation of mature cyst of TgPigSp1 shown an average production of 0.5 × 10⁵ mature cysts, but they were not observed in every batch of production. Parasite production was also quantified by Real Time Polymerase Chain Reaction (RT-PCR) using primer pairs for the 529 bp repeat element (RE) of *T. gondii* as previously described (Fernández-Escobar et al., 2020, Parasit. Vectors, 13: 396) interpolating the corresponding cycle threshold values on a standard curve that was generated by 10-fold serial dilutions of parasite DNA obtained from known numbers of *T. gondii* tachyzoites diluted in DNA (20 ng/µl) of Vero cells with a slope of -3.01 and a regression coefficient (R2) > 0.994. Average parasite yield determined by RT-PCR was of 1.99 × 10⁸ for the TgShSp3 and 1.69 × 10⁸ for the TgPigSp1 for each T75 culture flask shared with parasites determined by microscopy counting.

To produce the TgAE, a total of 10⁸ purified zoites with the presence of cyst structures were re-suspended in 600 µl of PBS with 0.5% (v/v) protease inhibitor (protease inhibitor cocktail, Sigma-Aldrich). Once re-suspended, 300 µl of 60% sucrose (w/v in PBS) were added to the suspension to obtain a final concentration of 20% (w/v) sucrose in the mixture and then, the parasite was recovered through centrifugation (10.000×g, 60 min, 4 °C). The precipitate (pellet) obtained was re-suspended in 0.1 ml of 1% Igepal^{®}Ca-630 solution (v/v in ultrapure water) supplemented with 0.5% of the same protease inhibitor for the solubilisation of the components and the suspension was maintained in constant agitation during 18-24 h at 4 °C to complete its homogenization. The protein concentration in the TgAE was quantified using the Bradford method and it was aliquoted and kept at -80 °C.

Regarding the average production yield of the TgAE, expressed as the amount of protein produced from 10⁸ tachyzoites, was of 365 and 300 µg/10⁸ zoites for TgShSp3 and TgPigSp1 respectively according to the results obtained from at least 6 different extraction experiments of different zoite production batches **(Table 5).**

**Table 5: Production yield of the extract from purified parasites of the T. gondii TgShSp3 and TgPigSp1 isolates in different batches of production from passage 29 to passage 35.**

| **Isolate: TgShSp3 (CCAP 2074/1)** | | | **Isolate: TgPigSp1 (CCAP 2074/2)** | | |
|---|---|---|---|---|---|
| **Experiment/ Isolate Passage (P)** | **Purified zoites (x 10⁸)^{a}** | **Protein yield TgAE/ 10⁸ zoites^{b}** | **Experiment/ Isolate Passage (P)** | **Purified zoites (x 10⁸)^{a}** | **Protein yield TgAE/ 10⁸ zoites^{b}** |
| **1/P29** | 0.9 | 226.33 | **1/P29** | 1.07 | 351.06 |
| **2/P30** | 1.22 | 243.69 | **2/P30** | 1.44 | 230.97 |
| **3/P31** | 2.00 | 482.19 | **3/P31** | 1.44 | 426.54 |
| **4/P32** | 1.95 | 582.77 | **4/P32** | 1.14 | 188.64 |
| **5/P33** | 1.60 | 292.15 | **5/P33** | 2.50 | 302.23 |
| **6/P34** | 2.00 | 226.33 | **6/P34** | - | 351.06 |
| **7/P35** | 2.63 | 226.33 | **7/P35** | 1.11 | 351.06 |
| **Mean** | **1.75** | **365.4 ± 109.7** | | **1.45** | **299.9 ± 91.5** |

| | | | | | |
|---|---|---|---|---|---|
| ^{a} Number of parasites obtained from a T75 culture flask -75 cm² cell monolayer-. Parasite production for each experiment was carried out with at least 2 T75 flasks. ^{b} Production yield of the TgAE from 10⁸ purified zoites determined by Bradford protein quantification. | | | | | |

### 2. Proteomic characterization of the TgAE: protein pattern in SDS-PAGE Coomassie gels and immunoblotting

As part of the characterization of the TgAE, its protein composition was studied to confirm absence of variation among batches of production, passage of the isolate and the isolate TgShSp3 or TgPigSp1 as source of production of TgAE. Variations in the composition were analysed through the study of the protein pattern in SDS-PAGE gels stained with Coomassie **(****Figure 1****)** and immunoblotting **(****Figure 2****).**

For Coomassie gels the amount of 50 µg of protein of the TgAE from each sample was mixed with the required amount of protein lysis buffer 2× (sodium dodecyl sulfate (SDS) 4%, glycerol 10%, 60 mM of Tris-HCl (pH 6.8), 100 mM of dithiothreitol (DTT), and 0.048% of bromophenol blue), boiled during 5 min and run in SDS-PAGE polyacrylamide gels. All samples were run in parallel with the Precision Plus Protein Standards Kaleidoscope^{™} marker (Bio-Rad) to determine the relative molecular weight of the protein bands. The different samples were resolved at 100 V (constant) during 6 h in a bis/acrylamide stacking gel at 4% (pH 6.8), followed by an acrylamide/bisacrylamide separating gel at 10%, in the presence of Tris-Glycine-SDS electrophoresis buffer and using a PROTEAN II System (Bio-Rad). After electrophoresis, the acrylamide gels were stained in a Coomassie solution and afterwards they were rinsed in an ethanol-acetic solution until the band pattern for each sample was visible in the gel. Protein profile analyses were carried out comparing the relative molecular weight of stained bands determined with the software Quantity One (Bio-Rad) and the Precision Plus Protein Standards Kaleidoscope^{™} marker (Bio-Rad) as reference. Protein pattern of three different batches of production (passage 31, 33 and 35) of the isolates TgShSp3 **(****Figure 1A****)** and TgPigSp1 **(****Figure 1B****)** were compared. Two replicates for each batch of production were also included to stablish technical differences in SDS-PAGE gels. Only those bands detected in 5 out of 6 protein profiles were considered for protein pattern.

All samples from different batches for each *T. gondii* isolate showed an identical protein pattern with 33 and 36 common bands for the TgAE from TgShSp3 and the TgAE from TgPigSpl, respectively. There was absence of variations among replicates from the same batch, and variations were minimum among batches of production with a matching higher than 95%. Variation among batches was also limited to differences in the intensity of staining of the band. Variation between TgShSp3 and TgPigSp1 protein profiles were four and seven specific bands, respectively.

The characterization of the TgAE was also carried out through the analysis of the pattern of the antigens recognised in each of the extracts with the sera of experimentally *T. gondii* infected mice obtained in Example 4 of this document. An amount of 10 µg of protein of the TgAE from different batches of production were subjected to electrophoresis in polyacrylamide SDS-PAGE mini-gels at 150 V for 1 hour and then, the proteins were transferred to a nitrocellulose membrane of 0.22 µm at 400 mA for 1 hour using the Tetracell system (Biorad). The marker Precision Plus Protein Standards Kaleidoscope^{™} (Bio-Rad) was also included as a reference to estimate the relative molecular weight of the different immunoreactive proteins. After the protein transfer, membranes were treated with a blocking solution in TBS buffer (5% skimmed milk in TBS-Tween 20 0.05%) and afterwards incubated with a dilution 1:50 of a pool of sera from mice infected with 100 oocyst of *T. gondii.* Antigen profile was revealed after incubation with an anti-mouse peroxidase-conjugated IgG (1:2000; Sigma Aldrich) as the secondary antibody and the antigen-antibody complexes formed were detected by chromogenic reaction with 4-Chloro-1-naphthol **(****Figure 2****).** All replicates and batches of production showed a common antigen profile with 24 and 28 immunoreactive bands for the TgShSp3 and TgPigSpl, respectively. Variations between replicates and batches of production were limited to the intensity of the bands for minor antigens regardless the replicate or batch of production, and likely related to intrinsic variability of the technique. Antigen profile between isolates was highly concordant in the 80-25 kDa region and only two specific antigens of 50 and 37 kDa were detected in the TgShSp3 TgAE and other two specific antigens of 38 and 30 kDa were detected for the TgPigSp1 **(****Figure 2****).** A higher number of immunoreactive bands were detected in the 80-200 kDa region for the TgAE obtained from the TgPigSp1 isolate.

TgAE were also studied for the presence of specific bradyzoite antigen TgBAG1 and specific tachyzoite antigen TgSAG1 protein by immunoblotting. For this, 10 µg of protein of the TgAE from different batches of production were subjected to electrophoresis and transferred to membranes as above. Membranes were blocked with blocking buffer and incubated with polyclonal rabbit serum against TgBAG1 and *Toxoplasma gondii* SAG1 Monoclonal Antibody D61S (Invitrogen) at dilution 1:100 and 1:25, respectively. Anti-rabbit IgG conjugated HRP and anti-mouse IgG2a conjugated HRP at dilution 1:1000 were used as secondary antibodies for TgBAG1 and TgSAG1 respectively, and membranes were revealed by chemiluminescence using the ECL substrate (Pierce). TgSAG1 (30 kDa) and TgBAG1 (25 kDa) proteins were recognized in all replicates of the TgAE of the TgShSp3 and TgPigSp1 isolates **(****Figure 3****).**

### 3. Proteomic characterization of the TqAE: identification of specific tachyzoite and bradyzoite and/or cyst components in the TgAE using quantitative label-free couple to liquid chromatography-tandem mass spectrometry (LC-MS/MS) analysis.

In this example, the inventors proceeded to identify the protein composition of the TgAE. The identification of the proteins included in the TgAE obtained from TgShSp3 and TgPigSp1 was carried out using liquid chromatography-tandem mass spectrometry (LC-MS/MS). LC-MS/MS analysis was carried out with four TgAE samples of the TgPigSp1 isolate and four samples of the TgShSp3 of different batches of production (biological replicates). Parasites used in TgAE production were recovered from cultures at passages 29 to 30, purified and maintained at -80° C using identical conditions described in Example 1. TgAE was equally produced as described in the Example 1. Quantitative label-free comparative proteomics analysis after LC-MS/MS was employed for study variation on composition between TgShSp3 and TgPigSp1 TgAEs.

TgAE samples for proteomics -100 µg of protein of each biological replicate- were run in a SDS-PAGE gel as described in Example 2. Proteins were excised from the SDS-PAGE after running samples 3 mm in the resolving gel and Coomassie staining. Then, protein samples in gel were digested in-solution trypsin. Briefly, samples were reduced with 10 mM DTT at 56 °C for 30 minutes and alkylated in the dark for 20 minutes with 55 mM iodoacetamide. Then, samples were digested at 37 °C overnight with a 1/50 (w/w) of Recombinant Sequencing Grade Trypsin (Roche Molecular Biochemicals, Mannheim, Germany) in 25 mM ammonium bicarbonate pH 8.5. The resulting peptides were eluted with 80% acetonitrile (ACN)/0.1% trifluoroacetic acid (TFA), freeze-dried in a vacuum centrifuge, resuspended in 25 µL of 0.1% formic acid (FA)/2% ACN and quantified by Qubit fluorometer (Thermo-Scientific). Then, peptides were analyzed by nano LC-MS/MS using a nano Easy-nLC 1000 (Thermo-Scientific) couple to a high-resolution mass spectrometer Q-exactive HF (Thermo-Scientific). The digested peptide mixtures were concentrated (on-line) by RP chromatography using an Acclaim PepMap 1000 precolumn (20 mm × 75 µm ID, C18, 3 µm silica particles, 100 Å pore size - Thermo Scientific) and then, peptides were separated using a C18 Picofrit analytical column (500 mm × 75 µm ID, Easy Spray Column, PepMap RSLC C18n, 2 µm particles, 100 Å pore size - Thermo Scientific) and eluted using a 150 minutes gradient from 5% solvent B to 35% solvent B in A, 10 minutes gradient from 35% to 45% solvent B in solvent A, 1 minute gradient from 45% to 95% solvent B in solvent A and 25 minutes gradient of 95% solvent B in solvent A (solvent A contained 0.1% AF, 2% ACN in water; solvent B contained 0.1% AF in ACN) operating at 250 nL/min. Peptides eluting from the column were electrosprayed directly into the mass spectrometer (on-line) from the analytical column with an ion transfer tube at temperature of 180°C. The peptides were detected with a resolution of 60000 in Full scan MS mode on a m/z mass range of 300-1800 Da. MS/MS data were acquired in data-dependent acquisition mode of the MS and in each microscan, depending on of its intensity (threshold: 2×10³) up to 15 precursors with a charge from 2+ to 4+ were selected with dynamic exclusion of 10 seconds, using an isolation with a window width of +/- 2 units of m/z and in a maximum time of 120 ms, for its fragmentation by high collision dissociation with an energy of normalized collision of 20%. MS/MS spectra were acquired in positive mode.

Peptide identification from MS/MS spectra were carried out through Proteome Discoverer 2.4 (Thermo Scientific) using licensed version of search engine MASCOT 2.6.1 (Matrix Science, London, UK). Tandem MS/MS data were searched against home-made database with predicted sequences of the *Toxoplasma gondii* download August 2021 from TOXODB (https://toxodb.org/toxo/app/downloads/release-51/TgondiiME49/fasta/data/; *Toxoplasma gondii* 51: ME49; 8322 sequences), together with database of *Chlorocebus sabaeus* from Uniprot (www.uniprot.org; 19229 sequences) and the data base Contaminants (247 sequencies) from Max Planck Institute of Biochemistry (https://www.biochem.mpg.de/mass_spectrometry). The following constraints were used for the searches: tryptic cleavage after Arg and Lys, up to two missed cleavage sites allowed, and tolerances of 20 ppm for precursor ions and 0.6 Da for MS/MS fragment ions and the searches were performed allowing optional Methionine oxidation and fixed carbamidomethylation of Cysteine. Search against decoy database (Mascot integrated decoy approach) was used to FDR calculation. MASCOT percolator filter was applied to MASCOT results (Matrix Science). The acceptances criteria for proteins identification were a false discovery rate (FDR) < 1% and, at least, one peptide identified with high confidence (Confidence Interval Cl > 99%).

The number of identified *T. gondii* proteins for sample group of the TgShSp3 and sample group of the TgPigSp1 was 2518 and 2403, respectively. The number of proteins exclusively identified in TgShSp3 samples was 148 and the number of exclusive proteins for TgPigSp1 was 33. Analyses of sequences on TgAE from TgShSp3 and TgPigSp1 showed presence of proteins highly expressed and more abundant during acute phase of infection **(Table 6),** as well as presence of proteins highly expressed and more abundant with chronic phase of infection in all replicates of TgAE **(Table 7)** (Buchholz et al., 2011, Eukaryot Cell. 10:1637-47; Garfoot et al., 2019, BMC Genomics 20:859; Pittman et al., 2014, BMC Genomics 15:806). Proteins recognized as specific tachyzoite antigens **(Table 8)** and specific bradyzoite antigens together with tissue cyst components **(Table 9)** were also found in TgAE from TgShSp3 and TgPigSp1 isolates (Tu et al., 2019, mBio 10:e00469-19; Tu et al., 2018, Microbes Infect. 20:466-476).

**Table 6: List of identified proteins associated to acute infection in TgAE from TgShSp3 and TgPigSp1 by LC-MS/MS analysis.**

| **Accession** | **Description (transcript product)** |
|---|---|
| TGME49_201780-t26_1-p1 | microneme protein MIC2 |
| TGME49_204050-t26_1-p1 | subtilisin SUB1 |
| TGME49_205470-t26_1-p1 | translation elongation factor 2 family protein, putative |
| TGME49_210370-t26_1-p1 | hypothetical protein |
| TGME49_211290-t26_1-p1 | rhoptry protein ROP15 |
| TGME49 213280-t26_1-p1 | SAG-related sequence SRS25 |
| TGME49_214940-t26_1-p1 | MIC2-associated protein M2AP |
| TGME49_218410-t26_1-p1 | ribosomal protein RPPO |
| TGME49_219540-t26_1-p1 | cytosolic tRNA-Ala synthetase |
| TGME49_221480-t26_1-p1 | hypothetical protein |
| TGME49_221620-t26_1-p1 | beta-tubulin, putative |
| TGME49 222170-t26_1-p1 | dense-granule antigen DG32 |
| TGME49_224460-t26_1-p1 | aminopeptidase n, putative |
| TGME49_226570-t26_1-p1 | hypothetical protein |
| TGME49_230180-t26_1-p1 | hypothetical protein |
| TGME49_232350-t26_1-p1 | lactate dehydrogenase LDH1 |
| TGME49_233460-t26_1-p1 | SAG-related sequence SRS29B |
| TGME49_233480-t26_1-p1 | SAG-related sequence SRS29C |
| TGME49_243570-t26_1-p1 | ribosomal protein RPS26 |
| TGME49_243730-t26_1-p1 | rhoptry protein ROP9 |
| TGME49_246540-t26_1-p1 | cytochrome c1, heme protein |
| TGME49_249180-t26_1-p1 | bifunctional dihydrofolate reductase-thymidylate synthase |
| TGME49_253430-t26_1-p1 | asparagine synthetase, putative |
| TGME49_254720-t26_1-p1 | dense granule protein GRA8 |
| TGME49_262050-t26_1-p1 | rhoptry kinase family protein ROP39 |
| TGME49_262400-t26_1-p1 | lipase |
| TGME49_262730-t26_1-p1 | rhoptry protein ROP16 |
| TGME49_263520-t26_1-p1 | microtubule associated protein SPM1 |
| TGME49_266970-t26_1-p1 | hypothetical |
| TGME49_268850-t26_1-p1 | enolase 2 |
| TGME49_271050-t26_1-p1 | SAG-related sequence SRS34A |
| TGME49_27S810-t26_1-p1 | ribosomal protein RPS10 |
| TGME49_275860-t26_1-p1 | hypothetical protein |
| TGME49_277080-t26_1-p1 | microneme protein MIC5 |
| TGME49_285870-t26_1-p1 | SAG-related sequence SRS20A |
| TGME49 286770-t26_1-p1 | hypothetical protein |
| TGME49_288720-t26_1-p1 | ribosomal protein RPL10 |
| TGME49_289630-t26_1-p1 | microneme protein MIC16 |
| TGME49_290200-t26_1-p1 | NAD/NADP octopine/nopaline dehydrogenase, alpha-helical domain-containing protein |
| TGME49_291890-t26_1-p1 | microneme protein MIC1 |
| TGME49_291960-t26_1-p1 | rhoptry kinase family protein ROP40 (incomplete catalytic triad) |
| TGME49_292110-t26_1-p1 | formate/nitrite transporter protein |
| TGME49_293430-t26_1-p1 | hypothetical protein |
| TGME49 300260-t26_1-p1 | threonyl-tRNA synthetase family protein |
| TGME49_305050-t26_1-p1 | calmodulin, putative |
| TGME49_308840-t26_1-p1 | SAG-related sequence SRS51 |
| TGME49_309590-t26_1-p1 | rhoptry protein ROP1 |
| TGME49_310780-t26_1-p1 | dense granule protein GRA4 |
| TGME49_318430-t26_1-p1 | malate dehydrogenase MDH |

**Table 7: List of identified proteins associated with chronic infection in TgAE from TgShSp3 and TgPigSp1 by LC-MS/MS analysis.**

| **Accession** | **Description (transcript product)** |
|---|---|
| TGME49_201840-t26_1-p1 | aspartyl protease ASP1 |
| TGME49_203290-t26_1-p1 | hypothetical protein |
| TGME49_207160-t26_1-p1 | SAG-related sequence SRS49D |
| TGME49_207710-t26_1-p1 | phosphatidylinositol synthase, putative |
| TGME49_208730-t26_1-p1 | microneme protein, putative |
| TGME49_209985-t26_1-p1 | cAMP-dependent protein kinase |
| TGME49_210810-t26_1-p1 | hypothetical protein |
| TGME49_214410-t26_1-p1 | hypothetical protein |
| TGME49 215910-t26_1-p1 | hypothetical protein |
| TGME49_224170-t26_1-p1 | SAG-related sequence SRS60A |
| TGME49_225540-t26_1-p1 | hypothetical protein |
| TGME49_225790-t26_1-p1 | PDI family protein |
| TGME49_225940-t26_1-p1 | hypothetical protein |
| TGME49_226420-t26_1-p1 | peptidase family M3 protein |
| TGME49_227020-t26_1-p1 | histone deacetylase SIR2 |
| TGME49 227380-t26_1-p1 | hypothetical protein |
| TGME49_234380-t26_1-p1 | hypothetical protein |
| TGME49_234530-t26_1-p1 | hypothetical protein |
| TGME49 236070-t26_1-p1 | pyrroline-5-carboxylate reductase |
| TGME49_236860-t26_1-p1 | haloacid dehalogenase family hydrolase domain-containing protein |
| TGME49_248990-t26_1-p1 | hypothetical protein |
| TGME49_251540-t26_1-p1 | dense granule protein GRA9 |
| TGME49_253330-t26_1-p1 | Rhoptry kinase family protein, truncated (incomplete catalytic triad) |
| TGME49_255900-t26_1-p1 | Bax inhibitor-1, putative |
| TGME49_259020-t26_1-p1 | bradyzoite antigen BAG1 |
| TGME49_260190-t26_1-p1 | microneme protein MIC13 |
| TGME49_261710-t26_1-p1 | ankyrin repeat-containing protein |
| TGME49_263270-t26_1-p1 | glycerophosphodiester phosphodiesterase family protein |
| TGME49_268790-t26_1-p1 | hypothetical protein |
| TGME49_268860-t26_1-p1 | enolase 1 |
| TGME49_270240-t26_1-p1 | MAG1 |
| TGME49 273320-t26_1-p1 | hypothetical protein |
| TGME49_278080-t26_1-p1 | Toxoplasma gondii family A protein |
| TGME49_280570-t26_1-p1 | SAG-related sequence SRS35A |
| TGME49_290300-t26_1-p1 | hypothetical protein |
| TGME49_290970-t26_1-p1 | 8-amino-7-oxononanoate synthase |
| TGME49_291040-t26_1-p1 | lactate dehydrogenase LDH2 |
| TGME49_293690-t26_1-p1 | profilin PRF |
| TGME49_309930-t26_1-p1 | melibiase subfamily protein |
| TGME49_309990-t26_1-p1 | hypothetical protein |
| TGME49_310670-t26_1-p1 | glycogen phosphorylase 1, putative |
| TGME49_312600-t26_1-p1 | heat shock protein HSP21 |
| TGME49_313050-t26_1-p1 | oxidoreductase, short chain dehydrogenase/reductase family protein |
| TGME49_320190-t26_1-p1 | SAG-related sequence SRS16B |

**Table 8: List of identified specific tachyzoite proteins in TgAE from TgShSp3 and TgPigSp1 by LC-MS/MS analysis.**

| **Accession** | **Description (transcript product)** |
|---|---|
| TGME49_213280-t26_1-p1 | SAG-related sequence SRS25 |
| TGME49 233450-t26_1-p1 | SAG-related sequence SRS29A |
| TGME49_233460-t26_1-p1 | SAG-related sequence SRS29B |
| TGME49_233480-t26_1-p1 | SAG-related sequence SRS29C |
| TGME49_271050-t26_1-p1 | SAG-related sequence SRS34A |
| TGME49_285870-t26_1-p1 | SAG-related sequence SRS20A |
| TGME49_308840-t26_1-p1 | SAG-related sequence SRS51 |

**Table 9: List of identified specific bradyzoite proteins and tissue cyst proteins in TgAE from TgShSp3 and TgPigSp1 by LC-MS/MS analysis.**

| **Accession** | **Description (transcript product)** |
|---|---|
| TGME49_207160-t26_1-p1 | SAG-related sequence SRS49D |
| TGME49_208730-t26_1-p1 | microneme protein, putative |
| TGME49 253330-t26_1-p1 | Rhoptry kinase family protein, truncated (incomplete catalytic triad) |
| TGME49_258870-t26_1-p1 | hypothetical protein |
| TGME49_259020-t26_1-p1 | bradyzoite antigen BAG1 |
| TGME49_264660-t26_1-p1 | SAG-related sequence SRS44 |
| TGME49_270240-t26_1-p1 | MAG1 |
| TGME49_280570-t26_1-p1 | SAG-related sequence SRS35A |
| TGME49_320190-t26_1-p1 | SAG-related sequence SRS16B |

For subsequent relative quantification of *T. gondii* proteins between two different conditions -TgShSp3 *versus* TgPigSp1- total amount of identified proteins was normalized by recalibration of relative weights and MS spectra alignment using Sequest HT of Protein Discover 2.4 software (Thermo). The total abundance of all peptides identified in at least 50% of replicates was used for calculation of the normalization factor and normalization of total amount of proteins among samples. Proteins differentially abundant between TgAE of TgShSp3 and TgPigSp1 were recognized under a non-nested design and pairwise ratio calculation in which protein ratios are calculated as median of all possible pairwise ratios calculated between biological replicates of all connected peptides. Protein differentially abundant between TgAE of TgShSp3 and TgPigSp1 were those with TgShSp3/TgPigSp1 ratios showed a value of log₂ ≥ 1 (corresponding to a fold change ≥ 2) for more abundant proteins in TgShSp3 and a value of log₂ ≤ -1 (corresponding to a fold change ≤ 0.5) for less abundant proteins in TgShSp3 - or more abundant proteins in TgPigSp1-, with a q-value or p adjusted value < 0.05 and an abundance ratio variability < 30%. Under these criteria only 19 proteins were significantly more abundant in the TgAE from the TgShSp3 compared with the TgAE from the TgShSp1 isolate. Among these proteins, TGME49_291040-t26_1-p1, TGME49_273320-t26_1-p1 and TGME49_207160-t26_1-p1 were associated with proteins abundant during chronic infection in mice **(Table 3)** and bradyzoite stage of the parasite **(Table 5).**

### 4. Safety, immunogenicity and efficacy of TgAE vaccine against T. gondii infection in a model of chronic toxoplasmosis in mice

The safety, immunogenicity, and efficacy against *T. gondii* infection of the TgAE vaccine formulated with the saponin Quil-A^{®} as adjuvant were evaluated in a mice model of chronic infection based on oral inoculation of 100 sporulated oocysts of the isolate TgShSp1 of the genotype #3 (ToxoDB) - type II-PRU variant in CD1 mice (Sánchez-Sánchez et al., 2019, Front Cell Infect Microbiol. 8:436).

TgAE vaccine was formulated mixing the volume of TgAE containing the amount of 20 µg of protein quantified by Bradford per doses diluted in PBS buffer and a volume containing 100 µg saponin Quil-A^{®} in a final volume of 200 µl. Vaccine *in vivo* assays were carried out evaluating vaccine formulated with TgAE extracts from TgShSp3, TgPigSp1 and a mix of both TgAEs.

Forty-five 8-week female Swiss/CD1 mice were obtained from a suitable provider (Janvier Labs) and were randomly allocated in four groups of 10 mice/group and one group of 5 mice/group. Animals were placed in the animal facilities following the UE regulations in force (Community Directive 2010/63/EU) in a 12 h-light and 12 h-dark cycles-controlled environment and provided with rodent feed and water *ad libitum.* Vaccines formulated with 20 µg of TgAE from TgShSp3, 20 µg of TgAE from TgPigSp1 o a mix with 10 µg of TgShSp3 plus 10 µg of TgPigSp1 were inoculated subcutaneously in groups G1, G2 and G3, respectively, at day 0 and boosted at day 30. Control non-immunized group G4 was inoculated with a formulation of PBS and saponin with absence of TgAE. Sentinel group G5 (n=5) was inoculated with PBS. The safety of the TgAE vaccines was evaluated observing all mice daily to detect adverse effects and local reactions such as the presence of nodules in the inoculation site associated to the vaccination. Mouse weight was determined at day 0 and weekly. A blood sample from all mice was collected from submandibular (facial) vein for evaluation of immunogenicity: specific humoral response against *T. gondii* at five days before the challenge and at day 16 after booster. Mice from G1-G4 groups were challenged orally with 100 oocysts of the TgShSp1 isolate at 21 days after booster, whereas mice from sentinel group G5 were challenged with PBS. The mice were observed daily for 42 days for the presence of clinical signs compatible with toxoplasmosis and morbidity was evaluated by the following score: asymptomatic (0), ruffled coat (1), rounded back/ascites (2), loss of body condition/body weight (>10%; <20%)/moderate ascites (3), severe loss of body weight (>20%)/gross ascites with marked abdominal distension/severe respiratory distress (continuous fast breathing and chest retraction)/neurological signs and sudden death (4). Mouse weight was evaluated weekly. Mice displaying a severe loss of body condition, gross ascites, severe respiratory distress, or neurological clinical signs were humanely euthanized. All mice were euthanized at 42 days post-challenge. During necropsy, serum samples from mice were collected for evaluate humoral immune response against *T. gondii.* A sample from the brain and other target organs such as tongue and the loin and quadriceps muscles were recovered for detection and quantification of *T. gondii* burden by RT-PCR of the 529 bp repeat element (RE) as previously described in the Example 1.

The humoral immune response (IgG, IgG1 and IgG2a isotypes) against the parasite was determined in sera by in-house whole inactivated tachyzoite-based ELISA. Briefly, 96-well Maxisorp^{®} microtiter plates (Thermo Scientific) were coated with 10⁵ tachyzoites in 50 mM sodium carbonate buffer (pH 9.6) and blocked in 5% powdered skim milk in 0.05% PBS-Tween. Sera samples were diluted 1:100 in blocking solution and after washing, were incubated with anti-mouse IgG HRP 1:10000 (Sigma Aldrich) for IgG response. For IgG1 and IgG2a responses, plates were incubated with anti-mouse IgG1 and IgG2 HRP antibodies (Southern Biotech) at 1:5000 (IgG1) or 1:1000 (IgG2a) dilutions, respectively. Humoral responses were determined using a relative index percent (RIPC) employing the following formula: RIPC = (O.D. λ sample - O.D. λ negative control)/(O.D. λ positive control - O.D. λ negative control) × 100.

No adverse effects attributed to the first immunization and booster were observed in mice throughout the experiment. Also, there was absence of significant differences in the average weight of the mice between the immunized -G1, G2 and G3- and the control groups G4 and G5 throughout the experiment until challenge. Only ruffled coat and mild local reaction < 5 mm with hair loss were observed in some of the animals in the groups G1, G2, G3 and G4 after first immunization and booster that were resolved on day 15 after the booster. The severity of the side effects (ruffle coat and local reactions) in mice of immunized groups G1, G2 and G3 was only significantly higher than that recorded in the control group G4 during the three first days after booster, related to the appearance of greater local reactions (p < 0.05, two-way ANOVA test). Local reactions associated with the administration of the adjuvant in the control group G4 were smaller and in a more limited number of mice. No local reactions were observed in the sentinel group G5.

All mice from immunized G1, G2 and G3 showed a specific IgG humoral response against *T*. *gondii* at 16 days after the booster (Figure 4A), that was significantly higher than in the control groups G4 and G5 (p < 0.05, one-way ANOVA test). Similar results were obtained for IgG1 and IgG2a responses with a significant increase in mice groups G1, G2 and G3 *versus* control groups G4 and G5 (p < 0.05, one-way ANOVA test). Analysis of IgG1 and IgG2a ratios did not show significant differences among G1, G2 and G3 groups.

All mice included in the vaccine assay survived until day 42 post-infection after the challenge. Most mice of groups G1, G2, G3 and G4 showed mild clinical signs: ruffle coat and rounded back during the first two weeks after challenge, which were extended to the third and fourth week after challenge only in the control group G4 (p < 0.05, two-way ANOVA test). The analysis of the average weights throughout the experiment did not show significant differences between groups, except for the group G1, immunized with the TgAE of the TgShSp3 isolate, which showed a higher average weight than the mice from the control group G4 for the fourth week post-immunization (p < 0.05, two-way ANOVA test).

All challenged mice from immunized groups G1, G2, G3 and control group G4 showed high levels of IgG against *T. gondii* at day 42 after the challenge (Figure 4B). Mice from groups G2 and G3 showed higher levels of IgG than the control group G4 (p < 0.05, one-way ANOVA test followed by Tukey's multiple comparisons test). All groups of mice from G1, G2 and G3 also showed significantly higher IgG1 levels than the control group G4, but there was absence of significant difference neither in increased IgG2a levels of the immunized groups *versus* control group, nor IgG1/IgG2a ratios among the challenged groups.

Parasite DNA was detected in at least one target tissue in all challenged mice as a confirmation of *T. gondii* infection. Parasite DNA was not detected by RT-PCR in target organs from sentinel group G5.

The average parasite burden by milligram of tissue detected in target organs of the challenged groups are shown in **Table 10.** The average burden detected on mice of the immunized groups G1, G2 and G3 were significantly lower than those of the control group G4 (p < 0.05, Kruskal-Walli's test followed of a Dunn's multiple comparisons test). The percentage of reduction of average burdens was higher than 80% in all target organs immunized with the TgAE of the TgPigSp1 isolate. The reduction in parasite burdens was ≥ 90% in the quadriceps and loin muscles of all immunized groups G1, G2 and G3 **(Table 10).**

**Table 10: Average parasite burden per mg of tissue in target organs.**

| **Average parasite burdens/mg tissue ± SEM*** | **G1 (TgAE TgShSp3)** | **G2 (TgAE TgPigSp1)** | **G3 (TgAE TgShSp3+TgPigSp1)** | **G4 (Quil-A^{®})** |
|---|---|---|---|---|
| Brain | 33.67 ± 7 | 25.28 ± 6.9 | 35.70 ± 10.7 | 161.6 ± 38.4 |
| Tongue | 28.36 ± 18.9 | 2.83 ± 1.2 | 4.178 ± 2.9 | 62.44 ± 17.6 |
| Loin | 5.721 ± 5.0 | 9.281 ± 5.916 | 4.167 ± 3.3 | 92.53 ± 27.7 |
| Quadriceps | 5.910 ± 3.7 | 2.180 ± 1.3 | 1.312 ± 0.6 | 72.54 ± 36.1 |

| | | | | |
|---|---|---|---|---|
| * SEM: standard error of the mean. All average parasite burdens/mg of tissue in all target organs (brain, tongue, loin and quadriceps) in the vaccinated mice groups G1, G2 and G3 were significantly lower than the control group G4 (p < 0.05, Kruskal-Wallis test followed of a Dunn's multiple comparisons test). | | | | |

### 5. Safety, immunogenicity and efficacy of TgAE vaccine against chronic T. gondii infection in porcine

In this example, the safety, immunogenicity, and efficacy of the TgAE formulated as a vaccine was evaluated in a *T. gondii* chronic infection model in piglets based on oral inoculation of 1000 sporulated oocysts of the TgShSp1 isolate (Largo de la Torre et al., 2022 Front Immunol. 13:1021556).

TgAE vaccine was formulated with the saponin Quil-A^{®} as adjuvant. For this, the volume of TgAE of the TgPigSp1 containing the amount of required protein quantified by Bradford per dose (20 µg or 40 µg) was diluted in PBS and mixed with 300 µg of Quil-A^{®} in a final volume of 1 ml per doses.

Twenty-seven female Landrace-Large White crossbreed piglets 21-28 days old acquired from a high sanitary status farm (Agropardal de Almendros S.L., Cuenca, Spain) were included in the vaccine assay. The absence of specific antibodies against *T. gondii* in the recruited piglets was confirmed by ELISA as described below (Largo de la Torre et al., 2022 Front Immunol. 13:1021556). Animals were handled according to the UE regulations in force (Community Directive 2010/63/EU). Piglets were randomly allocated into three groups of 8 animals each (G1, G2 and G3) and one sentinel group of three individuals (G4); each group was independently housed in conditioned boxes with controlled room temperature and humidity, bedding, and water and food *ad libitum* throughout the experiment. After an adaptation period of a week, piglets were intramuscularly inoculated twice: first inoculation and booster, in a 14-day interval with the 40 µg TgAE vaccine (group G1), with the 20 µg TgAE vaccine (group G2) or PBS (groups G3 and G4). Safety of vaccines was evaluated after each vaccination registering adverse effects, systemic and local reactions associated with inoculation for 14 days after each inoculation. Rectal temperature was also recorded daily for 14 days after inoculation and booster. For evaluation of immunogenicity, blood samples were collected before immunization at day 0, and at days 3, 7, 16, 24 and 28 after first inoculation by cranial cava venepuncture using vacuum devices with and without heparin anticoagulant (BD Vacutainer^{®} Plus Plastic Serum, Franklin Lakes, NJ, USA). After extraction, blood samples collected without anticoagulant were allowed to clot and were centrifuged (1200×g, 10 minutes, 4°C) to obtain serum samples for humoral immune response analyses. Blood samples collected with anticoagulant were immediately used for a peripheral blood immunostimulation assay and subsequent study of the cellular immune responses by measuring of cytokine interferon-gamma production after specific stimulation with whole-inactivated *T. gondii* antigen (see below). At 14 days after booster -at 28 days after first inoculation-, piglets of the immunized groups G1, G2 and G3 were orally dosed with 10³ oocysts of TgShSp1 in 1 mL of PBS by direct deposition on the piglets' oral cavity using a syringe. Sentinel group G4 was orally inoculated with 1 mL PBS. After the challenge, clinical signs were monitored daily throughout the entire experimental period focusing on detection of those associated with *T. gondii* oocyst infection: diarrhoea, anorexia, apathy, and behavioural changes. Rectal temperatures were recorded daily for 14 days post-challenge and then weekly until the end of the experiment at 42 days-post challenge. Any temperature above 40°C was considered as fever. Blood samples were collected as described above the day 3, 7, 14, 28 and 35 post-challenge for the study of humoral and cellular immune response. At the end of the experiment - day 42 post-challenge -, the animals were sedated by intramuscular administration in the neck and then immediately euthanized by an intravenous overdose of pentobarbital (Dolethal, Vetoquinol). During necropsy, approximately 50 g of tissue samples were collected from brain, heart, and skeletal muscles, *longissimus dorsi* -loin- and semimembranosus, for parasite detection by mouse bioassay and/or quantification of parasite DNA by RT-qPCR. For this, heart and muscle tissue samples were processed for obtaining homogenates by acid-pepsin artificial digestion. Brain tissues were homogenized in PBS without acid-pepsin digestion, filtered through sterile gauze and then centrifuged at 1350×g for 15 minutes at 4°C (Largo de la Torre et al., 2022 Front Immunol. 13:1021556).

*T*. gondii-specific IgG, IgG1 and IgG2 response was evaluated in piglets using an in-house ELISA with whole lyophilized TgME49 tachyzoites as antigen. Briefly, 96-well microtiter plates Maxisorp^{®} (Thermo Scientific) were coated with 10⁵ inactivated tachyzoites in 50 mM sodium carbonate buffer (pH 9.6) and blocked in 5% powdered skim milk in 0.05% PBS-Tween. Sera samples were diluted 1:100 in blocking solution and after washing, were incubated with protein G + HRP at 1:6000 (Sigma Aldrich) for IgG response. For IgG1 and IgG2 responses plates were incubated with anti-porcine mouse IgG1 and IgG2 monoclonal antibodies (Bio-Rad) at 1:2500 (IgG1) or 1:6000 (IgG2) dilutions, followed by anti-mouse IgG - peroxidase antibody at 1:10000 -1:5000 (Sigma Aldrich). Humoral responses were determined using a relative index percent (RIPC). Samples were considered positive (i.e., animals had seroconverted) when statistically significant differences were observed compared to the negative control.

For analysis of interferon-gamma production, 500 µL heparinized blood samples were mixed with 500 µL RPMI 1640 medium (Gibco) supplemented with 10% foetal bovine serum (Thermo Fisher Scientific) and 1% antibiotic/antimycotic solution (Lonza) and blood cells were cultured in 24-well plates in the presence of 10⁶ lyophilized *T. gondii* tachyzoites as antigen, concanavalin A at final concentration of 5 µg/mL as a positive control (Sigma-Aldrich), or PBS as a negative control. The plates were incubated in a 5% CO_{2/}37°C/100 % humidity atmosphere for 48 h and centrifuged at 1000×g for 10 min at 4 °C. Interferon-gamma was assessed in cell-free culture supernatants using a commercial ELISA sandwich kit (ELISA Flex: Porcine IFN-γ (HRP), Mabtech) following the manufacturer's recommendations.

For parasite detection in tissue homogenates by mouse bioassay, 500 µL of brain sediment and acid-pepsin-treated homogenates from the heart, tongue, *longissimus dorsi* -loin-, and semimembranosus were inoculated subcutaneously in three mice per tissue. Animals were examined daily for clinical signs compatible with toxoplasmosis and were humanely euthanized according to severity of clinical signs as described in the Example 4. All remaining mice were euthanized at 30 days post-challenge, and during necropsy, brain was collected for detection of *T. gondii* DNA by RT-PCR as above. Parasite detection in the brain of one mouse out the three mice inoculated with each tissue homogenate determine positive presence of *T. gondii* in the target tissue. Additionally, genomic DNA was extracted from 50 to 80 mg of each tissue homogenate (≈ 50 µL) and *T. gondii* parasite DNA was quantified by 529 bp repetitive element (RE) RT-PCR as described in the Example 4.

After first immunization and booster, no adverse effects were detected associated with the immunization. Sporadic diarrhoea was observed in a limited number of piglets of all groups, including the sentinel G4, and it was associated with changes in the feed after weaning. No local reactions were recorded. Fever was detected sporadically in no more than two animals of all groups for 1 day after first immunization and booster. No there was significant variation in the mean body temperature among the piglet groups and increase of rectal temperature was less than 1 °C in all groups prior to challenge.

Kinetics of the IgG immune response after first immunization and booster is shown in **Figure 5A****.** Piglets of the immunized groups G1 and G2 seroconverted from day 7 after booster and IgG levels were maintained in the day 14 after booster when piglets were challenged (p < 0.05, two-way ANOVA test). Seroconversion was not detected in the control groups G3 and G4 previously to challenge. Similar kinetics was observed for IgG1 and IgG2 levels. The *T*. gondii-specific interferon-gamma production was also increased at day 14 after booster previously to challenge in the piglets of the immunized group G1 in comparison with the control group G3 (p < 0.05, two-way ANOVA test) **(****Figure 5B****).**

After the challenge, no severe clinical signs were observed in piglets of all groups. Piglets of the control group G3 showed apathy and anorexia according to reduction of food consumption between 6- and 8-days post-challenge. These mild clinical signs were associated with the presence of fever (>40°C) in all animals of the challenged control group G3. Thus, a significant increase in median rectal temperatures was detected from six to eight days post-challenge in the piglets of the control group G3 *versus* piglets of the sentinel group G4 (p < 0.05; two-way ANOVA) **(****Figure 6****).** During the same time was detected sporadically fever only in two and one piglet of the immunized groups G1 and G2, respectively, for one day.

The levels of IgG detected on the vaccinated groups G1 and G2 were maintained until day 14 after challenge when they significantly increased until the end of the experiment. Piglets of the challenged control group G3 seroconverted at day 21 post-challenge and then were significantly increased until the end of the experiment. Nevertheless, the IgG levels reached by immunized groups G1 and G2 were significantly higher than IgG levels of the challenged control group G3 throughout the experiment (p < 0.05, two-way ANOVA test) **(****Figure 7A****).** IgG response was not detected in the sentinel group G4. Similar results were obtained with IgG1 and IgG2 responses with a high increase of antibody levels from day 14 post-challenge in the groups G1 and G2, seroconversion at day 21 post-challenge in the group G3, and highest IgG1 and IgG2 levels in the vaccinated groups G1 and G2 until the end of experiment. Production of interferon-gamma in the peripheral blood stimulation assays was also maintained in immunized groups G1 and G2 until day 8 post-challenge when they were significantly increased until the end of experiment **(****Figure 7B****).** Production of interferon-gamma was not detected in the challenged control group G3 until the same day 8 post-challenge when they were detected **(****Figure 7B****).** Production was also maintained high in the control group G3 until the end of experiment. No significant differences in the production of interferon-gamma were found among the challenged groups G1, G2 and G3.

Frequencies of *T. gondii* parasite detection in target tissues determined by mouse bioassay are shown in **Table 11.** Parasite infection was not detected in target tissues of the piglets of the sentinel group G4. *T. gondii* infection was detected in four -out of eight- piglets of the group G1 in the brain, heart, heart and loin, and semimembranosus, respectively, in three - out of eight- piglets of the group G2, in the loin, brain, and brain and semimembranosus, respectively, and all eight piglets of the control group G3 in brain or heart and other target tissues. A drastic reduction on frequencies of *T. gondii* detection in target tissues of immunized groups G1 and G2 was also observed by RT-PCR: parasite was detected in the loin of one piglet of the group G1, in the brain and the heart of two piglets of the group G2, and in the brain, heart or loin of most of piglets -seven out of eight- of the control group G3 **(Table 11).** *T. gondii* was not detected by RT-PCR on semimembranosus. According to parasite detection results, at least, the 50% of piglets of the vaccinated groups G1 and G2 were free of *T. gondii* infection. The analysis of mean parasite burdens determined by RT-PCR in target tissues showed a reduction > 90% in brain, heart and loin of piglets of the immunized groups G1 and G2 when compared with the challenged control group G3 (p < 0.05, Kruskal-Wallis test followed of a Dunn's multiple comparisons test) in most of target tissues **(Table 12).**

**Table 11. Frequencies of parasite detection in target tissues of piglets from groups G1, G2 and G3 by mouse bioassay and RT-PCR.**

| **Group** | **Piglet** | **Parasite detection (Bioassay: +/n)*** | **Parasite detection (RT-PCR: +/n)**** |
|---|---|---|---|
| G1 (40 µg) | 1 | 0/12 | 0/4 |
| | 2 | 1/12 | 0/4 |
| | 3 | 0/12 | 0/4 |
| | 4 | 0/12 | 0/4 |
| | 5 | 1/12 | 0/4 |
| | 6 | 0/12 | 0/4 |
| | 7 | 2/12 | 1/4 |
| | 8 | 1/12 | 0/4 |
| G2 (20 µg) | 9 | 0/12 | 0/4 |
| | 10 | 0/12 | 0/4 |
| | 11 | 1/12 | 0/4 |
| | 12 | 1/12 | 0/4 |
| | 13 | 2/12 | 2/4 |
| | 14 | 0/12 | 0/4 |
| | 15 | 0/12 | 1/4 |
| | 16 | 0/12 | 0/4 |
| G3 (PBS) | 17 | 1/12 | 1/4 |
| | 18 | 8/12 | 2/4 |
| | 19 | 9/12 | 3/4 |
| | 20 | 3/12 | 0/4 |
| | 21 | 3/12 | 2/4 |
| | 22 | 5/12 | 2/4 |
| | 23 | 10/12 | 2/4 |
| | 24 | 3/12 | 1/4 |

| | | | |
|---|---|---|---|
| * +: number of *T. gondii* positive mice/number of mice inoculated with samples of brain, heart, loin and semimembranosus homogenates. ** +: number of *T. gondii* positive tissue homogenate/number of samples of brain, heart, loin and semimembranosus homogenates. | | | |

Association between immunogenicity induced by vaccination and efficacy of vaccines based on reduction of parasite burdens in target organs was studied by Spearman correlation analysis. Reduction on parasite burdens detected in the brain or the heart of piglets from the groups G1 and G2 was inversely correlated with IgG responses at day 7 and 15 after booster (Spearman coefficient -r-: < -0.72 - (-0.77), p<0.0001 for parasite burdens in brain and r:-0.47 - (-0.45), p<0.05 for parasite burdens in heart) and interferon-gamma production at day 7 and 14 after booster (r : -0.50 - (-0.52); p<0.01 for parasite burdens in brain).

**Table 12. Mean parasite burden per mg of tissue in target organs.**

| **Mean parasite burdens/mg tissue ± SEM*** | **G1** | **G2** | **G3** |
|---|---|---|---|
| | **(40 µg TgAE)** | **(20 µg TgAE)** | **(PBS)** |
| Brain | 0.00** | 0.01± 0.01** | 2.10 ± 1.78 |
| Heart | 0.00** | 0.09± 0.06 | 0.86± 0.46 |
| Loin | 0.03± 0.03** | 0.00 | 1.56± 1.50 |

| | | | |
|---|---|---|---|
| * SEM: standard error of the mean. **Mean parasite burdens/mg of tissue significantly lower than mean parasite burdens in the sentinel control group G4 (p < 0.05, Kruskal-Wallis test followed of a Dunn's multiple comparisons test). | | | |

### 6. Safety, immunogenicity and efficacy of TgAE vaccine against reproductive losses caused by T. gondii in ovine

In this example, the inventors evaluated the safety, immunogenicity, and efficacy of the TgAE vaccine against the reproductive losses caused by *T. gondii* infection in ovine. For this, we used a toxoplasmosis pregnant sheep model based on oral inoculation of 10 oocysts of the isolate TgShSp1 at day 90 of pregnancy with an expected foetal loss of 80% (Sánchez-Sánchez et al., 2019, Front Cell Infect Microbiol. 8:436).

TgAE vaccine was formulated mixing the volume of TgAE containing 40 µg of protein quantified by Bradford per doses diluted in PBS buffer and a volume containing 300 µg saponin Quil-A^{®} in a final volume of 2 mL.

Fourteen pure Rasa Aragonesa breed pregnant ewes aged 24 months were selected for vaccine *in vivo* assay from a commercial flock. All animals were tested seronegative for *T. gondii, N. caninum,* as well as other abortifacients present in the area (*Coxiella burnetii, Chlamydophila abortus* and Schmallenberg disease virus and border disease virus (BDV)) by ELISA. Animals were handled according to the UE regulations in force (Community Directive 2010/63/EU). They were estrus-synchronized and mated with pure-bred Rasa Aragonesa tups for 2 days, after which the rams were separated from the ewes. Pregnancy and foetal viability were confirmed by ultrasound scanning (US) on day 40 post-mating - day 40 of pregnancy- and pregnant ewes were randomly distributed into three experimental groups: three ewes in the group G1, eight ewes in the group G2 and other three ewes in the sentinel group G3. Each group was independently housed in conditioned boxes with controlled room temperature and humidity, bedding, and water and food *ad libitum* throughout the experiment. After an adaptation period of a week, ewes were subcutaneously inoculated twice: first inoculation and booster, in a 22-day interval with the 40 µg TgAE vaccine (group G1) or PBS (groups G2 and G3) at days 55 and 77 of pregnancy. Safety of vaccines was evaluated after each vaccination registering adverse effects, systemic and local reactions associated with inoculation for 14 days after first inoculation and booster. Rectal temperatures were also daily recorded and temperatures above 40°C were considered fever. Clinical consequences on pregnancy were also evaluated weekly by ultrasound scanning and foetal viability was assessed by monitoring of foetal heartbeat and movements. Blood samples were collected before first immunization at day 0, before booster at day 22, at day 30 after first inoculation and at day 36 before challenge by jugular venipuncture in 10 ml tubes type Vacuntainer (Terumo Europe) without anti-coagulant for evaluating immunogenicity. Ewes of the immunized groups G1 and G2 were orally dosed at 36 days after first immunization on day 90 of pregnancy with a dose of 10 oocysts of TgShSp1. The three-remaining sheep of the sentinel group G3 received PBS. Pregnant ewes were observed daily throughout the experiment. Rectal temperatures were recorded daily from day 0 until 14 days post-challenge and then weekly to evaluate morbidity. Foetal viability was assessed by US monitoring weekly until parturition of lambs at 145-150 days of pregnancy. Blood samples were collected at 3-, 5-, 7-, and 10-days post-challenge and then weekly for studying humoral immune response. Precolostral serum was also collected from lambs immediately after delivery from dams to evaluate expose of foetuses to *T. gondii* infection. Thoracic and abdominal fluids were also collected from stillborn lambs from which precolostral sera could not be obtained. When all foetal death occurred for pregnancy, or after parturition, dams and lambs were euthanized, first sedated with xylazine (Rompun, Bayer) and then by an intravenous overdose of embutramide and mebezonium iodide (T61, Intervet). During necropsy, six randomly selected placentomes or cotyledons from aborted dams and dams that gave birth, respectively, were recovered from each placenta, for parasite detection by RT-PCR. Samples from foetal tissues, including brain and lungs were also evaluated by RT-PCR for detection and quantification of parasite DNA in these target tissues by 529 bp repetitive element (RE) RT-PCR as described in the Example 4.

*T*. gondii-specific IgG antibody levels in sheep and lambs were measured using an in-house indirect ELISA. Briefly, 96-well microtiter plates Maxisorp^{®} (Thermo Scientific) were coated with 10⁵ inactivated tachyzoites in 100 mM sodium carbonate buffer (pH 9.6) and blocked in 5% powdered skim milk in 0.05% PBS-Tween. Sera samples were diluted 1:100 in blocking solution and after washing, were incubated with anti-goat/sheep monoclonal- IgG at 1:8000 (Sigma Aldrich) for IgG response. Humoral responses were determined using a relative index percent (RIPC). Samples were considered positive (i.e., animals had seroconverted) when statistically significant differences were observed compared to the negative control.

No adverse effects or local reactions attributed to the immunization were observed in pregnant sheep. No consequences on pregnancy were detected and foetuses remained live until the challenge. Rectal temperatures were significantly increased in the immunized group G1 at 1 day after first immunization and booster, but immediately returned to normal levels at day 2 post-inoculation. Low-grade fever (temperature ≤ 40.2 ºC) was observed in two out of three ewes of the immunized group G1.

All sheep of the vaccinated group G1 showed high level of IgG response at 8 days after booster -at 30 days after first immunization-, that were maintained until the challenge. No IgG response was observed in pregnant sheep of the control groups G2 and G3 **(****Figure 8A****).** The IgG levels were maintained in ewes of the immunized group G1 until day 21 post-challenge when they significantly increased and maintained until the end of the experiment. All animals of the control group G2 seroconverted at day 21 post-challenge and IgG levels were gradually increased until the end of the experiment, but IgG levels in the immunized group G1 were significantly higher than those in the challenged control group G2 until the end of the experiment (p < 0.05, two-way ANOVA test) **(****Figure 8B****).**

Mean rectal temperature was significantly increased in the challenged control group G2 from day five to 10 after challenge **(****Figure 9****).** Most of ewes (≥ 7 out the 8) showed fever from day six to eight after challenge. Mean rectal temperature was significantly increased two days after in the immunized group G1, from day seven to 10. All three ewes showed mild fever at day seven (temperature < 41 °C), but the number of ewes with hyperthermia decreased following days to one ewe with low-grade fever at day 10 post-challenge. Mean temperature increase was also detected in the immunized G1 group when parturition.

Consequences on pregnancy are summarized in the **Table 13.** Foetal losses were limited to a stillborn lamb in multiple pregnancies of the immunized group G1 and the sentinel group G3, corresponding to a 16.6% and a 20% of foetal mortality, respectively. Foetal mortality recorded in the challenged control group G2 reached the 64.7%, with one foetal abortion at day 13 post-challenge, delivery of four mummified foetuses from two ewes, and seven stillborn lambs **(Table 13).** Thus, foetal mortality was significantly diminished in the immunized group G1 *versus* the control group G3 (p<0.05; Fisher's exact test).

**Table 13: Result of pregnancy: foetal losses and number of healthy lambs born in groups G1, G2 and G3.**

| **Group** | **Ewe** | **Number of foetuses** | **Foetal losses** | | **Healthy** |
|---|---|---|---|---|---|
| | | | **Abortion-mummified** | **Stillborn** | |
| **G1** | 1 | 2 | | 1 | 1 |
| **(40 µg TgAE-challenged)** | 2 | 2 | | | 2 |
| | 3 | 2 | | | 2 |
| **G2** | 4 | 1 | | | 1 |
| **(PBS-challenged)** | 5 | 2 | | | 2 |
| | 6 | 1 | | | 1 |
| | 7 | 2 | 2 | | |
| | 8 | 4 | 2 | 1 | 1 |
| | 9 | 1 | | 1 | |
| | 10 | 2 | | 2 | |
| | 11 | 3 | | 3 | |
| **G3 (PBS)** | 12 | 1 | | | 1 |
| | 13 | 3 | | 1 | 2 |
| | 14 | 1 | | | 1 |

*T*. gondii-specific IgG response was detected in thoracic fluids of all stillborn of control group G3. IgG responses in precolostral sera of healthy lambs was detected on all healthy lambs of the immunized group G1 and two of the five healthy lambs delivered in the challenged control group G2 from ewes 4 and 8 **(Table 13).** *T. gondii* infection was detected in placentomes/cotyledons of all ewes and lungs of all stillborn and healthy lambs of the immunized group G1 and challenged control group G2, confirming *T. gondii* congenital infection in all animals of the challenged groups. Mean parasite burdens in cotyledons, and foetal lung and brain in the immunized group G1 were lower than mean parasite burdens in the challenged control group G2, and it was significantly lower in the brain **(Table 14).**

**Table 14. Mean parasite burden per mg of tissue in target organs.**

| **Mean parasite burdens/mg tissue ± SEM*** | **G1** | **G2** |
|---|---|---|
| | **(40 µg TgAE)** | **(PBS)** |
| **Cotyledon** | 317 ± 128.4 | 442.5± 142 |
| **Foetal lung** | 52.5 ± 13.81 | 74.98± 24.6 |
| **Foetal brain** | 0.24± 0.14** | 1.95± 1.32 |

| | | |
|---|---|---|
| * SEM: standard error of the mean. **Mean parasite burdens/mg of tissue significantly lower than average parasite burdens in the control group G5 (p < 0.05, U-Mann Whitney test). | | |

### ITEMS OF THE PRESENT INVENTION

1. A protein composition comprising at least one native *T. gondii* tachyzoite-specific protein and at least one native *T. gondii* bradyzoite and/or cyst specific protein.
2. The protein composition according to item 1, wherein the composition further comprises at least one native bradyzoite and/or cyst specific protein selected from:

| **Accession¹** | **Description²** | **SEQ ID NO.³** |
|---|---|---|
| TGME49_207160-t26_1-p1 | SAG-related sequence SRS49D | 52 |
| TGME49_208730-t26_1-p1 | microneme protein, putative | 54 |
| TGME49_253330-t26_1-p1 | Rhoptry kinase family protein, truncated (incomplete catalytic triad) | 72 |
| TGME49_259020-t26_1-p1 | bradyzoite antigen BAG1 | 74 |
| TGME49_270240-t26_1-p1 | MAG1 | 80 |
| TGME49_320190-t26_1-p1 | SAG-related sequence SRS16B | 93 |
| TGME49_280570-t26_1-p1 | SAG-related sequence SRS35A | 83 |
| TGME49_264660-t26_1-p1 | SAG-related sequence SRS44 | 96 |
| TGME49_258870-t26_1-p1 | hypothetical protein | 95 |

| | | |
|---|---|---|
| ¹Accession number for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ²Protein description in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ³ Example of predicted sequence for the corresponding protein on the ToxoDB database (ToxoDB-51 TgondiiME49 AnnotatedProteins.fasta, Last modified: 2021-03-06 09:21; Size: 9.2M | | |

3. The protein composition according to item 1 to 2, wherein the composition further comprises at least one native tachyzoite-specific protein selected from:

| **Accession¹** | **Description²** | **SEQ ID NO.³** |
|---|---|---|
| TGME49_233460-t26_1-p1 | SAG-related sequence SRS29B | 17 |
| TGME49_233480-t26_1-p1 | SAG-related sequence | 18 |
| TGME49_271050-t26_1-p1 | SAG-related sequence SRS34A | 31 |
| TGME49_285870-t26_1-p1 | SAG-related sequence SRS20A | 35 |
| TGME49_233450-t26_1-p1 | SAG-related sequence SRS29A | 94 |
| TGME49_213280-t26_1-p1 | SAG-related sequence SRS25 | 6 |
| TGME49_308840-t26_1-p1 | SAG-related sequence SRS51 | 46 |

| | | |
|---|---|---|
| ¹Accession number for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ²Protein description in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ³ Example of predicted sequence for the corresponding protein on the ToxoDB database (ToxoDB-51 TgondiiME49 AnnotatedProteins.fasta, Last modified: 2021-03-06 09:21; Size: 9.2M | | |

4. The protein composition according to any one of items 1 to 2, wherein the composition further comprises at least one, preferably all, native protein(s) specific of the chronic phase infection selected from:

| **Accession number¹** | **Description²** | **SEQ ID NO.³** |
|---|---|---|
| TGME49_201840-t26_1-p1 | aspartyl protease ASP1 | 50 |
| TGME49_203290-t26_1-p1 | hypothetical protein | 51 |
| TGME49_207160-t26_1-p1 | SAG-related sequence SRS49D | 52 |
| TGME49 207710-t26_1-p1 | phosphatidylinositol synthase, putative | 53 |
| TGME49_208730-t26_1-p1 | microneme protein, putative | 54 |
| TGME49_209985-t26_1-p1 | cAMP-dependent protein kinase | 55 |
| TGME49_210810-t26_1-p1 | hypothetical protein | 56 |
| TGME49_214410-t26_1-p1 | hypothetical protein | 57 |
| TGME49_215910-t26_1-p1 | hypothetical protein | 58 |
| TGME49_224170-t26_1-p1 | SAG-related sequence SRS60A | 59 |
| TGME49_225540-t26_1-p1 | hypothetical protein | 60 |
| TGME49_225790-t26_1-p1 | PDI family protein | 61 |
| TGME49_225940-t26_1-p1 | hypothetical protein | 62 |
| TGME49_226420-t26_1-p1 | peptidase family M3 protein | 63 |
| TGME49_227020-t26_1-p1 | histone deacetylase SIR2 | 64 |
| TGME49_227380-t26_1-p1 | hypothetical protein | 65 |
| TGME49_234380-t26_1-p1 | hypothetical protein | 66 |
| TGME49_234530-t26_1-p1 | hypothetical protein | 67 |
| TGME49_236070-t26_1-p1 | pyrroline-5-carboxylate reductase | 68 |
| TGME49_236860-t26_1-p1 | haloacid dehalogenase family hydrolase domain-containing protein | 69 |
| TGME49_248990-t26_1-p1 | hypothetical protein | 70 |
| TGME49_251540-t26_1-p1 | dense granule protein GRA9 | 71 |
| TGME49_253330-t26_1-p1 | Rhoptry kinase family protein, truncated (incomplete catalytic triad) | 72 |
| TGME49_255900-t26_1-p1 | Bax inhibitor-1, putative | 73 |
| TGME49_259020-t26_1-p1 | bradyzoite antigen BAG1 | 74 |
| TGME49_260190-t26_1-p1 | microneme protein MIC13 | 75 |
| TGME49_261710-t26_1-p1 | ankyrin repeat-containing protein | 76 |
| TGME49_263270-t26_1-p1 | glycerophosphodiester phosphodiesterase family protein | 77 |
| TGME49_268790-t26_1-p1 | hypothetical protein | 78 |
| TGME49_268860-t26_1-p1 | enolase 1 | 79 |
| TGME49_270240-t26_1-p1 | MAG1 | 80 |
| TGME49_273320-t26_1-p1 | hypothetical protein | 81 |
| TGME49_278080-t26_1-p1 | *Toxoplasma gondii* family A protein | 82 |
| TGME49_280570-t26_1-p1 | SAG-related sequence SRS35A | 83 |
| TGME49_290300-t26_1-p1 | hypothetical protein | 84 |
| TGME49_290970-t26_1-p1 | 8-amino-7-oxononanoate synthase | 85 |
| TGME49_291040-t26_1-p1 | lactate dehydrogenase LDH2 | 86 |
| TGME49_293690-t26_1-p1 | profilin PRF | 87 |
| TGME49_309930-t26_1-p1 | melibiase subfamily protein | 88 |
| TGME49_309990-t26_1-p1 | hypothetical protein | 89 |
| TGME49_310670-t26_1-p1 | glycogen phosphorylase 1, putative | 90 |
| TGME49_312600-t26_1-p1 | heat shock protein HSP21 | 91 |
| TGME49_313050-t26_1-p1 | oxidoreductase, short chain dehydrogenase/reductase family protein | 92 |
| TGME49_320190-t26_1-p1 | SAG-related sequence SRS16B | 93 |

| | | |
|---|---|---|
| ¹Accession number for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ²Protein description in ToxoDB database (ToxoDB-51_TgondiiME49_AnnotatedProteins.asta, Last modified: 2021-03-06 09:21; Size: 9.2M; Description: 8322 Proteins, translated CDS (AA) fasta format) ³ Example of predicted sequence for the corresponding protein on the ToxoDB database (ToxoDB-51 TgondiiME49 AnnotatedProteins.fasta, Last modified: 2021-03-06 09:21; Size: 9.2M | | |

and at least one, preferably all, native protein(s) specific of the acute phase selected from:

| **Accession¹** | **Description²** | **SEQ ID NO.³** |
|---|---|---|
| TGME49_201780-t26_1-p1 | microneme protein MIC2 | 1 |
| TGME49_204050-t26_1-p1 | subtilisin SUB1 | 2 |
| TGME49_205470-t26_1-p1 | translation elongation factor 2 family protein, putative | 3 |
| TGME49_210370-t26_1-p1 | hypothetical protein | 4 |
| TGME49_211290-t26_1-p1 | rhoptry protein ROP15 | 5 |
| TGME49_213280-t26_1-p1 | SAG-related sequence SRS25 | 6 |
| TGME49_214940-t26_1-p1 | MIC2-associated protein M2AP | 7 |
| TGME49_218410-t26_1-p1 | ribosomal protein RPPO | 8 |
| TGME49_219540-t26_1-p1 | cytosolic tRNA-Ala synthetase | 9 |
| TGME49_221480-t26_1-p1 | hypothetical protein | 10 |
| TGME49_221620-t26_1-p1 | beta-tubulin, putative | 11 |
| TGME49_222170-t26_1-p1 | dense-granule antigen | 12 |
| TGME49 224460-t26_1-p1 | aminopeptidase n, putative | 13 |
| TGME49_226570-t26_1-p1 | hypothetical protein | 14 |
| TGME49_230180-t26_1-p1 | hypothetical protein | 15 |
| TGME49_232350-t26_1-p1 | lactate dehydrogenase LDH1 | 16 |
| TGME49_233460-t26_1-p1 | SAG-related sequence SRS29B | 17 |
| TGME49_233480-t26_1-p1 | SAG-related sequence SRS29C | 18 |
| TGME49_243570-t26_1-p1 | ribosomal protein RPS26 | 19 |
| TGME49_243730-t26_1-p1 | rhoptry protein ROP9 | 20 |
| TGME49_246540-t26_1-p1 | cytochrome c1, heme protein | 21 |
| TGME49_249180-t26_1-p1 | bifunctional dihydrofolate reductase-thymidylate synthase | 22 |
| TGME49_253430-t26_1-p1 | asparagine synthetase, putative | 23 |
| TGME49_254720-t26_1-p1 | dense granule protein GRA8 | 24 |
| TGME49_262050-t26_1-p1 | rhoptry kinase family protein ROP39 | 25 |
| TGME49_262400-t26_1-p1 | lipase | 26 |
| TGME49_262730-t26_1-p1 | rhoptry protein ROP16 | 27 |
| TGME49_263520-t26_1-p1 | microtubule associated protein SPM1 | 28 |
| TGME49_266970-t26_1-p1 | hypothetical protein | 29 |
| TGME49_268850-t26_1-p1 | enolase 2 | 30 |
| TGME49_271050-t26_1-p1 | SAG-related sequence SRS34A | 31 |
| TGME49_275810-t26_1-p1 | ribosomal protein RPS10 | 32 |
| TGME49_275860-t26_1-p1 | hypothetical protein | 33 |
| TGME49_277080-t26_1-p1 | microneme protein MIC5 | 34 |
| TGME49_285870-t26_1-p1 | SAG-related sequence SRS20A | 35 |
| TGME49_286770-t26_1-p1 | hypothetical protein | 36 |
| TGME49_288720-t26_1-p1 | ribosomal protein RPL10 | 37 |
| TGME49_289630-t26_1-p1 | microneme protein MIC16 | 38 |
| TGME49_290200-t26_1-p1 | NAD/NADP octopine/nopaline dehydrogenase, alpha-helical domain-containing protein | 39 |
| TGME49_291890-t26_1-p1 | microneme protein MIC1 | 40 |
| TGME49_291960-t26_1-p1 | rhoptry kinase family protein ROP40 (incomplete catalytic triad) | 41 |
| TGME49_292110-t26_1-p1 | formate/nitrite transporter protein | 42 |
| TGME49_293430-t26_1-p1 | hypothetical protein | 43 |
| TGME49_300260-t26_1-p1 | threonyl-tRNA synthetase family protein | 44 |
| TGME49_305050-t26_1-p1 | calmodulin, putative | 45 |
| TGME49_308840-t26_1-p1 | SAG-related sequence SRS51 | 46 |
| TGME49_309590-t26_1-p1 | rhoptry protein ROP1 | 47 |
| TGME49_310780-t26_1-p1 | dense granule protein GRA4 | 48 |
| TGME49_318430-t26_1-p1 | malate dehydrogenase MDH | 49 |

| | | |
|---|---|---|
| ¹Accession number for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ²Protein description in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ³ Example of predicted sequence for the corresponding protein on the ToxoDB database (ToxoDB-51 TgondiiME49 AnnotatedProteins.fasta, Last modified: 2021-03-06 09:21; Size: 9.2M | | |

5. A method for producing a protein composition comprising the following steps:
a. Providing *Toxoplasma gondii* cells in a hypertonic solution;
b. Centrifuging said solution obtained in step (a) under conditions suitable for separating the soluble fraction (supernatant) and insoluble fraction (precipitate);
c. Recovering the precipitate from step (b); and
d. Mixing said precipitate with a non-ionic surfactant.
6. The method according to item 5, wherein the *Toxoplasma gondii* cells are *Toxoplasma gondii* tachyzoites and/or bradyzoites.
7. The method according to items 5 to 6, wherein the *Toxoplasma gondii* cells:
(i) maintain/retain the ability of forming cysts; and/or
(ii) maintain/retain the ability of spontaneous conversion from tachyzoite stage to bradyzoite stage under conventional conditions of growth *in vitro,* and/or
(iii) maintain/retain the ability to produce proteins associated to the chronic phase, comprising cyst- and bradyzoite-specific proteins, and proteins associated to the acute phase, comprising tachyzoite-specific proteins.
8. The method according to items 5 to 7, wherein the *Toxoplasma gondii* cells belong to the PCR-RFLP genotypes ToxoDB #2 and/or #3.
9. The method according to items 5 to 8, wherein the *Toxoplasma gondii* cells belong to the isolates TgShSp3 and/or TgPigSpl, deposited under the Budapest Treaty, with accession number CCAP 2074/1 and CCAP 2074/2, respectively.
10. The method according to any one of items 5 to 9, wherein the hypertonic solution comprises sucrose, and/or sorbitol, and/or mannitol, preferably 10-80% sucrose (w/v in PBS), more preferably 15-40% sucrose (w/v in PBS), even more preferably 20% sucrose (w/v in PBS).
11. The method according to any one of items 5 to 10, wherein the centrifugation takes place at 8000-15000xg, during 40-90 min and at a temperature of 1-10°C, preferably at 8000-15000xg, during 60 min at about 4 °C, more preferably at 10000xg, during 60 min at about 4°C.
12. The method according to any one of items 5 to 11, wherein the non-ionic surfactant is selected from the group consisting of: Polysorbate 80, Triton X-I 14, Triton X-100 and Tween 20 and NP-40, preferably the non-ionic surfactant is NP-40.
13. The method according to any one of items 5 to 12, wherein the method further comprises the step of homogenising the mixture obtained after step (d).
14. A pharmaceutical composition comprising the protein composition according to any one of items 1 to 4, wherein preferably, the pharmaceutical composition further comprises one or more excipients and/or one or more pharmaceutically acceptable carriers or diluents, preferably selected from the list consisting of water, culture fluid, a solution of physiological salt concentration and/or stabilisers such as SPGA, carbohydrates (e.g., sorbitol, mannitol, starch, sucrose, glucose, dextran), proteins such as albumin or casein, protein containing agents such as bovine serum or skimmed milk and buffers (e.g., phosphate buffer), and wherein preferably, said pharmaceutical composition further comprises immunomodulant-immunostimulant substances such as cytokines.
15. A vaccine comprising the pharmaceutical composition according to item 14.
16. The vaccine according to item 15, wherein the vaccine comprises an adjuvant, wherein the adjuvant is preferably a saponin adjuvant, preferably Quil-A^{®}.
17. The pharmaceutical composition according to item 14 and/or the vaccine according to any one of items 15 to 16, wherein said pharmaceutical composition comprises an amount of from about 0.0001% to about 0.5% w/v of the protein composition as defined in any one of items 1 to 4, or obtained by the method of any one of items 5 to 13, preferably an amount of from about 0.0005% to about 0.2% w/v, more preferably an amount of from about 0.001% to about 0.02% w/v, even more preferably an amount from about 0.002% to about 0.004% w/v of the protein composition as defined in any one of items 1 to 4, or obtained by the method of any one of items 5 to 13.
18. The protein composition according to any one of items 1 to 4 or obtained by the method of any one of items 5 to 13, the pharmaceutical composition according to item 14 and/or the vaccine according to any one of items 15 to 17 for use as a medicament.
19. The protein composition according to any one of items 1 to 4 or obtained by the method of any one of items 5 to 13, the pharmaceutical composition according to item 14 and/or the vaccine according to any one of items 15 to 17 for use in a method of therapeutic treatment (after infection or after the clinical manifestation of the disease caused by the infection) and/or prophylactic treatment (before infection or before the clinical manifestation of the disease caused by the infection) of infections caused by *Toxoplasma gondii.*
20. The protein composition according to any one of items 1 to 4 or obtained by the method of any one of items 5 to 13, the pharmaceutical composition according to item 14 and/or the vaccine according to any one of items 15 to 17, for use according to any one of items 18 to 19, wherein the protein composition and/or the pharmaceutical composition and/or the vaccine is administered to a mammal, preferably to a mammal selected from the group consisting of domestic pig (*Sus scrofa* and *Sus scrofa domestica*)*,* sheep (*Ovis aries*), goat (*Capra aegagrus hircus*) non-human primates, humans (*Homo sapiens*)*,* cats (*Felis catus*) and marsupials.
21. The protein composition according to any one of items 1 to 4 or obtained by the method of any one of items 5 to 13, the pharmaceutical composition according to item 14 and/or the vaccine according to any one of items 15 to 17, for use according to any one of items 18 to 19, wherein the protein composition and/or the pharmaceutical composition and/or the vaccine is administered to a mammal in an amount of 0.001-10 µg of the protein composition as defined in any one of items 1 to 8 *per* kg of the individual (mammal) to which the pharmaceutical composition and/or the vaccine is administrated, preferably an amount of 0.01-2 µg/kg, preferably at least two times, with at least 14-21 days between each of the administrations, and/or wherein the protein composition and/or the pharmaceutical composition and/or the vaccine is administered intranasally, intradermally, subcutaneously, by aerosol, intramuscularly, wing web and eye-drop administration, preferably subcutaneously and/or intramuscularly and/or intradermally, more preferably subcutaneously and/or intramuscularly.

## Claims

1. A protein composition comprising at least one native *T. gondii* bradyzoite and/or cyst specific protein selected from:
| **Accession¹** | **Description²** | **SEQ ID NO.³** |
|---|---|---|
| TGME49_207160-t26_1-p1 | SAG-related sequence SRS49D | 52 |
| TGME49_208730-t26_1-p1 | microneme protein, putative | 54 |
| TGME49_253330-t26_1-p1 | Rhoptry kinase family protein, truncated (incomplete catalytic triad) | 72 |
| TGME49_259020-t26_1-p1 | bradyzoite antigen BAG1 | 74 |
| TGME49_270240-t26_1-p1 | MAG1 | 80 |
| TGME49_320190-t26_1-p1 | SAG-related sequence SRS16B | 93 |
| TGME49_280570-t26_1-p1 | SAG-related sequence SRS35A | 83 |
| TGME49_264660-t26_1-p1 | SAG-related sequence SRS44 | 96 |
| TGME49_258870-t26_1-p1 | hypothetical protein | 95 |
| | | |
|---|---|---|
| ¹Accession number for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ²Protein description in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ³ Example of predicted sequence for the corresponding protein on the ToxoDB database (ToxoDB-51 TgondiiME49 AnnotatedProteins.fasta, Last modified: 2021-03-06 09:21; Size: 9.2M | | |
and at least one native tachyzoite-specific protein selected from:
| **Accession¹** | **Description²** | **SEQ ID NO.³** |
|---|---|---|
| TGME49_233460-t26_1-p1 | SAG-related sequence SRS29B | 17 |
| TGME49_233480-t26_1-p1 | SAG-related sequence | 18 |
| TGME49_271050-t26_1-p1 | SAG-related sequence SRS34A | 31 |
| TGME49_285870-t26_1-p1 | SAG-related sequence SRS20A | 35 |
| TGME49_233450-t26_1-p1 | SAG-related sequence SRS29A | 94 |
| TGME49_213280-t26_1-p1 | SAG-related sequence SRS25 | 6 |
| TGME49_308840-t26_1-p1 | SAG-related sequence SRS51 | 46 |
| | | |
|---|---|---|
| ¹Accession number for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ²Protein description in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ³ Example of predicted sequence for the corresponding protein on the ToxoDB database (ToxoDB-51 TgondiiME49 AnnotatedProteins.fasta, Last modified: 2021-03-06 09:21; Size: 9.2M | | |

2. The protein composition according to claim 1, wherein the composition comprises the following proteins:
| **Accession¹** | **Description²** | **SEQ ID NO.³** |
|---|---|---|
| TGME49_207160-t26_1-p1 | SAG-related sequence SRS49D | 52 |
| TGME49_208730-t26_1-p1 | microneme protein, putative | 54 |
| TGME49_253330-t26_1-p1 | Rhoptry kinase family protein, truncated (incomplete catalytic triad) | 72 |
| TGME49_259020-t26_1-p1 | bradyzoite antigen BAG1 | 74 |
| TGME49_270240-t26_1-p1 | MAG1 | 80 |
| TGME49_320190-t26_1-p1 | SAG-related sequence SRS16B | 93 |
| TGME49_280570-t26_1-p1 | SAG-related sequence SRS35A | 83 |
| TGME49_264660-t26_1-p1 | SAG-related sequence SRS44 | 96 |
| TGME49_258870-t26_1-p1 | hypothetical protein | 95 |
| | | |
|---|---|---|
| ¹Accession number for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ²Protein description in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ³ Example of predicted sequence for the corresponding protein on the ToxoDB database (ToxoDB-51 TgondiiME49 AnnotatedProteins.fasta, Last modified: 2021-03-06 09:21; Size: 9.2M | | |
and
| **Accession¹** | **Description²** | **SEQ ID NO.³** |
|---|---|---|
| TGME49_233460-t26_1-p1 | SAG-related sequence SRS29B | 17 |
| TGME49_233480-t26_1-p1 | SAG-related sequence | 18 |
| TGME49_271050-t26_1-p1 | SAG-related sequence SRS34A | 31 |
| TGME49_285870-t26_1-p1 | SAG-related sequence SRS20A | 35 |
| TGME49_233450-t26_1-p1 | SAG-related sequence SRS29A | 94 |
| TGME49_213280-t26_1-p1 | SAG-related sequence SRS25 | 6 |
| TGME49_308840-t26_1-p1 | SAG-related sequence SRS51 | 46 |
| | | |
|---|---|---|
| ¹Accession number for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ²Protein description in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ³ Example of predicted sequence for the corresponding protein on the ToxoDB database (ToxoDB-51 TgondiiME49 AnnotatedProteins.fasta, Last modified: 2021-03-06 09:21; Size: 9.2M | | |

3. The protein composition according to claims 1 or 2, wherein the composition further comprises at least one, preferably all, native protein(s) specific of *T. gondii* chronic phase infection selected from:
| **Accession number¹** | **Description²** | **SEQ ID NO.³** |
|---|---|---|
| TGME49_201840-t26_1-p1 | aspartyl protease ASP1 | 50 |
| TGME49_203290-t26_1-p1 | hypothetical protein | 51 |
| TGME49_207160-t26_1-p1 | SAG-related sequence SRS49D | 52 |
| TGME49 207710-t26_1-p1 | phosphatidylinositol synthase, putative | 53 |
| TGME49_208730-t26_1-p1 | microneme protein, putative | 54 |
| TGME49_209985-t26_1-p1 | cAMP-dependent protein kinase | 55 |
| TGME49_210810-t26_1-p1 | hypothetical protein | 56 |
| TGME49_214410-t26_1-p1 | hypothetical protein | 57 |
| TGME49_215910-t26_1-p1 | hypothetical protein | 58 |
| TGME49_224170-t26_1-p1 | SAG-related sequence SRS60A | 59 |
| TGME49_225540-t26_1-p1 | hypothetical protein | 60 |
| TGME49_225790-t26_1-p1 | PDI family protein | 61 |
| TGME49_225940-t26_1-p1 | hypothetical protein | 62 |
| TGME49_226420-t26_1-p1 | peptidase family M3 protein | 63 |
| TGME49_227020-t26_1-p1 | histone deacetylase SIR2 | 64 |
| TGME49_227380-t26_1-p1 | hypothetical protein | 65 |
| TGME49_234380-t26_1-p1 | hypothetical protein | 66 |
| TGME49_234530-t26_1-p1 | hypothetical protein | 67 |
| TGME49_236070-t26_1-p1 | pyrroline-5-carboxylate reductase | 68 |
| TGME49_236860-t26_1-p1 | haloacid dehalogenase family hydrolase domain-containing protein | 69 |
| TGME49_248990-t26_1-p1 | hypothetical protein | 70 |
| TGME49_251540-t26_1-p1 | dense granule protein GRA9 | 71 |
| TGME49_253330-t26_1-p1 | Rhoptry kinase family protein, truncated (incomplete catalytic triad) | 72 |
| TGME49_255900-t26_1-p1 | Bax inhibitor-1, putative | 73 |
| TGME49_259020-t26_1-p1 | bradyzoite antigen BAG1 | 74 |
| TGME49_260190-t26_1-p1 | microneme protein MIC13 | 75 |
| TGME49_261710-t26_1-p1 | ankyrin repeat-containing protein | 76 |
| TGME49_263270-t26_1-p1 | glycerophosphodiester phosphodiesterase family protein | 77 |
| TGME49_268790-t26_1-p1 | hypothetical protein | 78 |
| TGME49_268860-t26_1-p1 | enolase 1 | 79 |
| TGME49_270240-t26_1-p1 | MAG1 | 80 |
| TGME49_273320-t26_1-p1 | hypothetical protein | 81 |
| TGME49_278080-t26_1-p1 | Toxoplasma gondii family A protein | 82 |
| TGME49_280570-t26_1-p1 | SAG-related sequence SRS35A | 83 |
| TGME49_290300-t26_1-p1 | hypothetical protein | 84 |
| TGME49_290970-t26_1-p1 | 8-amino-7-oxononanoate synthase | 85 |
| TGME49_291040-t26_1-p1 | lactate dehydrogenase LDH2 | 86 |
| TGME49_293690-t26_1-p1 | profilin PRF | 87 |
| TGME49_309930-t26_1-p1 | melibiase subfamily protein | 88 |
| TGME49_309990-t26_1-p1 | hypothetical protein | 89 |
| TGME49_310670-t26_1-p1 | glycogen phosphorylase 1, putative | 90 |
| TGME49_312600-t26_1-p1 | heat shock protein HSP21 | 91 |
| TGME49_313050-t26_1-p1 | oxidoreductase, short chain | 92 |
| | dehydrogenase/reductase family protein | |
| TGME49_320190-t26_1-p1 | SAG-related sequence SRS16B | 93 |
| | | |
|---|---|---|
| ¹Accession number for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ²Protein description in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ³ Example of predicted sequence for the corresponding protein on the ToxoDB database (ToxoDB-51 TgondiiME49 AnnotatedProteins.fasta, Last modified: 2021-03-06 09:21; Size: 9.2M | | |

4. The protein composition according to any one of claims 1 to 3, wherein the composition further comprises at least one, preferably all, native protein(s) specific of *T. gondii* acute phase infection selected from:
| **Accession¹** | **Description²** | **SEQ ID NO.³** |
|---|---|---|
| TGME49_201780-t26_1-p1 | microneme protein MIC2 | 1 |
| TGME49_204050-t26_1-p1 | subtilisin SUB1 | 2 |
| TGME49_205470-t26_1-p1 | translation elongation factor 2 family protein, putative | 3 |
| TGME49_210370-t26_1-p1 | hypothetical protein | 4 |
| TGME49_211290-t26_1-p1 | rhoptry protein ROP15 | 5 |
| TGME49_213280-t26_1-p1 | SAG-related sequence SRS25 | 6 |
| TGME49_214940-t26_1-p1 | MIC2-associated protein M2AP | 7 |
| TGME49_218410-t26_1-p1 | ribosomal protein RPP0 | 8 |
| TGME49_219540-t26_1-p1 | cytosolic tRNA-Ala synthetase | 9 |
| TGME49_221480-t26_1-p1 | hypothetical protein | 10 |
| TGME49_221620-t26_1-p1 | beta-tubulin, putative | 11 |
| TGME49_222170-t26_1-p1 | dense-granule antigen | 12 |
| TGME49 224460-t26_1-p1 | aminopeptidase n, putative | 13 |
| TGME49_226570-t26_1-p1 | hypothetical protein | 14 |
| TGME49_230180-t26_1-p1 | hypothetical protein | 15 |
| TGME49_232350-t26_1-p1 | lactate dehydrogenase LDH1 | 16 |
| TGME49_233460-t26_1-p1 | SAG-related sequence SRS29B | 17 |
| TGME49_233480-t26_1-p1 | SAG-related sequence SRS29C | 18 |
| TGME49_243570-t26_1-p1 | ribosomal protein RPS26 | 19 |
| TGME49_243730-t26_1-p1 | rhoptry protein ROP9 | 20 |
| TGME49_246540-t26_1-p1 | cytochrome c1, heme protein | 21 |
| TGME49_249180-t26_1-p1 | bifunctional dihydrofolate reductase-thymidylate synthase | 22 |
| TGME49_253430-t26_1-p1 | asparagine synthetase, putative | 23 |
| TGME49_254720-t26_1-p1 | dense granule protein GRA8 | 24 |
| TGME49_262050-t26_1-p1 | rhoptry kinase family protein ROP39 | 25 |
| TGME49_262400-t26_1-p1 | lipase | 26 |
| TGME49_262730-t26_1-p1 | rhoptry protein ROP16 | 27 |
| TGME49_263520-t26_1-p1 | microtubule associated protein SPM1 | 28 |
| TGME49_266970-t26_1-p1 | hypothetical protein | 29 |
| TGME49_268850-t26_1-p1 | enolase 2 | 30 |
| TGME49_271050-t26_1-p1 | SAG-related sequence SRS34A | 31 |
| TGME49_275810-t26_1-p1 | ribosomal protein RPS10 | 32 |
| TGME49_275860-t26_1-p1 | hypothetical protein | 33 |
| TGME49_277080-t26_1-p1 | microneme protein MIC5 | 34 |
| TGME49_285870-t26_1-p1 | SAG-related sequence SRS20A | 35 |
| TGME49_286770-t26_1-p1 | hypothetical protein | 36 |
| TGME49_288720-t26_1-p1 | ribosomal protein RPL10 | 37 |
| TGME49_289630-t26_1-p1 | microneme protein MIC16 | 38 |
| TGME49_290200-t26_1-p1 | NAD/NADP octopine/nopaline dehydrogenase, alpha-helical domain-containing protein | 39 |
| TGME49_291890-t26_1-p1 | microneme protein MIC1 | 40 |
| TGME49_291960-t26_1-p1 | rhoptry kinase family protein ROP40 (incomplete catalytic triad) | 41 |
| TGME49_292110-t26_1-p1 | formate/nitrite transporter protein | 42 |
| TGME49_293430-t26_1-p1 | hypothetical protein | 43 |
| TGME49_300260-t26_1-p1 | threonyl-tRNA synthetase family protein | 44 |
| TGME49_305050-t26_1-p1 | calmodulin, putative | 45 |
| TGME49_308840-t26_1-p1 | SAG-related sequence SRS51 | 46 |
| TGME49_309590-t26_1-p1 | rhoptry protein ROP1 | 47 |
| TGME49_310780-t26_1-p1 | dense granule protein GRA4 | 48 |
| TGME49_318430-t26_1-p1 | malate dehydrogenase MDH | 49 |
| | | |
|---|---|---|
| ¹Accession number for the identified protein in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ²Protein description in ToxoDB database (ToxoDB-51_TgondiiME49_AnnotatedProteins.fasta, Last modified: 2021-03-06 09:21; Size: 9.2M; Description: 8322 Proteins, translated CDS (AA) fasta format) ³ Example of predicted sequence for the corresponding protein on the ToxoDB database (ToxoDB-51 TgondiiME49 AnnotatedProteins.fasta, Last modified: 2021-03-06 09:21; Size: 9.2M | | |

5. A method for producing a protein composition comprising the following steps:
a. Providing *Toxoplasma gondii* cells in a hypertonic solution;
b. Centrifuging said solution obtained in step (a) under conditions suitable for separating the soluble fraction (supernatant) and insoluble fraction (precipitate);
c. Recovering the precipitate from step (b); and
d. Mixing said precipitate with a non-ionic surfactant.

6. The method according to claim 5, wherein the *Toxoplasma gondii* cells comprise *Toxoplasma gondii* tachyzoites and bradyzoites.

7. The method according to any one of claims 5 to 6, wherein the *Toxoplasma gondii* cells:
(i) maintain/retain the ability of forming cysts; and/or
(ii) maintain/retain the ability of spontaneous conversion from tachyzoite stage to bradyzoite stage under conventional conditions of growth *in vitro,* and/or
(iii) maintain/retain the ability to produce proteins associated to the chronic phase, comprising cyst- and bradyzoite-specific proteins, and proteins associated to the acute phase, comprising tachyzoite-specific proteins, preferably wherein the *Toxoplasma gondii* cells belong to the PCR-RFLP genotypes ToxoDB #2 and/or #3.

8. The method according to any one of claims 5 to 7, wherein the *Toxoplasma gondii* cells belong to the isolates TgShSp3 and/or TgPigSp1, deposited under the Budapest Treaty with accession number CCAP 2074/1 and CCAP 2074/2, respectively.

9. A pharmaceutical composition comprising the protein composition according to any one of claims 1 to 4, or obtainable by the method as defined in any one of claims 5 to 8, wherein the pharmaceutical composition preferably further comprises one or more excipients and/or one or more pharmaceutically acceptable carriers or diluents.

10. A vaccine comprising the pharmaceutical composition according to claim 9.

11. The vaccine according to claim 10, wherein the vaccine comprises an adjuvant, wherein the adjuvant is preferably a saponin adjuvant, preferably Quil-A^{®}.

12. The protein composition according to any one of claims 1 to 4, or obtainable by the method as defined in any one of claims 5 to 8, the pharmaceutical composition according to claim 9 and/or the vaccine according to any one of claims 10 to 11 for use as a medicament.

13. The protein composition according to any one of claims 1 to 4, or obtainable by the method as defined in any one of claims 5 to 8, the pharmaceutical composition according to claim 9 and/or the vaccine according to any one of claims 10 to 11 for use in a method of therapeutic and/or prophylactic treatment of infections caused by *Toxoplasma gondii.*

14. The protein composition according to any one of claims 1 to 4, or obtainable by the method as defined in any one of claims 5 to 8, the pharmaceutical composition according to claim 9 and/or the vaccine according to any one of claims 10 to 11 for use according to claim 13 wherein the protein composition and/or the pharmaceutical composition and/or the vaccine is administered to a mammal.
